# EUROPEAN PATENT APPLICATION

(11) **EP 3 594 345 A1**
(43) Date of publication of application: **15.01.2020**
(21) Application number: 18764304.4
(22) Date of filing: 07.03.2018
(51) Int. Cl.: C12N 15/113, A61K 31/713, A61K 48/00, A61P 7/02, A61P 9/10, A61P 13/12

(54) **NUCLEIC ACID CAPABLE OF INHIBITING EXPRESSION OF MASP2**

(30) Priority: 09.03.2017 JP 2017045226
(71) Applicant: Kyowa Kirin Co., Ltd., Chiyoda-ku Tokyo 1000004 (JP)
(72) Inventor: MASUDA, Kazuhiro, Tokyo 1000004 (JP); YAMADA, Yoji, Tokyo 1000004 (JP); IWAI, Hiroto, Tokyo 1000004 (JP); ISODA, Yuya, Tokyo 1000004 (JP); PIAO, Wen, Tokyo 1000004 (JP)
(74) Representative: D Young & Co LLP
(86) International application number: PCT/JP2018/008788
(87) International publication number: WO 2018/164186

(57) **Abstract**

The present invention provides a nucleic acid having activity to suppress expression of MASP2, a pharmaceutical composition comprising the nucleic acid, and a prophylactic or therapeutic drug containing the nucleic acid for autoimmune diseases such as APS, SLE and the like and thrombosis in hemodialysis.

## Description

### Technical Field

The present invention relates to a nucleic acid for use in suppressing expression of MASP2 or a pharmaceutical composition comprising the nucleic acid.

### Background Art

Mannan-binding lectin serine protease 2 (MASP2) is a protein composed of 686 amino acids, and is constituted of CUB domain-EGF domain-CUB domain-CCP1-CCP2-serine protease domain from the N-terminal side. MASP2 is one of the complement proteins produced in the liver and is involved in the activation of lectin pathway among the complement pathways (non-patent document 1).

As for the association with diseases, MASP2 is considered to be an important disease-related protein in autoimmune diseases involving complements such as IgA nephropathy, membranous nephropathy, Lupus nephropathy, C3 nephropathy, thrombotic thrombocytopenic purpura, thrombotic microangiopathy and atypical hemolytic uremic syndrome (aHUS) (non-patent documents 2, 3). MASP2 has been reported to be one of the causes of tissue injury during ischemia-reperfusion injury, and it is strongly suggested from studies using animal models and clinical studies that kidney disorder during ischemia and tissue injury during cerebral infarction can be reduced by inhibiting MASP2 (non-patent documents 4, 5). It is expected that the above-mentioned diseases can be prevented or treated by specifically inhibiting MASP2; however, no drug has been reported to date that specifically inhibits expression of MASP2.

As a method of suppressing expression itself of genes, a method utilizing, for example, RNA interference (hereinafter to be referred to as RNAi) and the like are known. To be specific, it was found that expression of a target gene is specifically suppressed by introducing a double-stranded RNA having the same sequence as the target gene and the RNA is named as short interfering RNA (siRNA) (patent document 1). As a method of suppressing expression of gene besides RNA interference, moreover, an antisense method is known (patent document 2). However, a siRNA sequence that has been shown to effectively suppress human MASP2 gene is not known.

### [Document List]

### [Patent documents]

patent document 1: WO 2001/75164
patent document 2: WO 98/56905

### [non-patent document]

non-patent document 1: Immunobiology, 205, 455-466 (2002)
non-patent document 2: Nat Rev Nephrol., 12, 383-401 (2016)
non-patent document 3: Nephrol Dial Transplant., 13, 1984-1990 (1998)
non-patent document 4: FASEB J., 28, 3996-4003 (2014)
non-patent document 5: J Neuroinflammation., 13, 213 (2016)

### [SUMMARY OF THE INVENTION]

### [Problems to be Solved by the Invention]

The present invention aims to provide a nucleic acid capable of suppressing expression of MASP2. The present invention also aims to provide a pharmaceutical composition for the prophylaxis or treatment of diseases associated with MASP2 expression.

### [Means of Solving the Problems]

The present invention relates to the following (1) to (16) .
(1) A double-stranded nucleic acid that decreases expression of MASP2 gene, which consists of a sense strand and an antisense strand, and comprises a double-stranded region of at least 11 base pairs, wherein an oligonucleotide strand having a strand length of at least 17 nucleotides and 30 nucleotides at most in the aforementioned antisense strand is complementary to a target MASP2 mRNA sequence selected from the group described in Table 1-1 to Table 1-22.
(2) The double-stranded nucleic acid of (1), wherein the aforementioned double-stranded region is composed of 11 - 27 base pairs, and the 2nd nucleotide from the 5'-terminus of the aforementioned antisense strand complementary to the target MASP2 mRNA sequence selected from the group described in Tables 1-1 to Table 1-22 is complement to the 2nd ribonucleotide from the 3'-terminus of the target MASP2 mRNA sequence.
(3) The double-stranded nucleic acid of (1) or (2), wherein the 3'-terminus of the aforementioned sense strand and the 5'-terminus of the aforementioned antisense strand form a blunt end.
(4) The double-stranded nucleic acid of (3), wherein the aforementioned sense strand is 21 nucleotides in length and the aforementioned antisense strand is 23 nucleotides in length.
(5) The double-stranded nucleic acid of (1) or (2), wherein the aforementioned sense strand is 21 nucleotides in length and the aforementioned antisense strand is 21 nucleotides in length.
(6) The double-stranded nucleic acid of (5), wherein the 3'-terminal of the aforementioned sense strand and the 3'-terminal of the aforementioned antisense strand have an overhang.
(7) The double-stranded nucleic acid of any one of (1) to (6), wherein the aforementioned antisense strand comprises a sequence selected from the groups described in "antisense strand" in Table 1-1 to Table 1-22 and Table 2.
(8) The double-stranded nucleic acid of any one of (1) to (6), wherein the aforementioned sense strand comprises a sequence selected from the groups described in "sense strand" in Table 1-1 to Table 1-22 and Table 2.
(9) The double-stranded nucleic acid of any one of (1) to (6), comprising a sequence of 1 pair of sense strand/antisense strand selected from the group consisting of the sense strands/antisense strands described in Table 1-1 to Table 1-22 and Table 2.
(10) The double-stranded nucleic acid of any one of the aforementioned (1) to (9), comprising a 2'-modified nucleotide.
(11) The double-stranded nucleic acid of aforementioned (10), wherein 50 - 100% of the nucleotides in the double strand region are 2'-modified nucleotides.
(12) The double-stranded nucleic acid of any one of (1) to (11), comprising a ligand.
(13) A single strand nucleic acid consisting only of an antisense strand in the double-stranded nucleic acid of any one of (1) to (12).
(14) A pharmaceutical composition comprising the nucleic acid of any one of (1) to (13).
(15) A method of treating a disorder mediated by abnormal complement lectin pathway, comprising a step of administering a therapeutically effective amount of the nucleic acid of any one of (1) to (13) or the pharmaceutical composition of (14) to a human in need of such treatment.
(16) The method of (15), wherein the aforementioned disorder is IgA nephropathy, membranous nephropathy, lupus nephropathy, C3 nephropathy, thrombotic thrombocytopenic purpura, thrombotic microangiopathy, atypical hemolytic uremic syndrome (aHUS), kidney disorder during ischemia or tissue injury during cerebral infarction.

### [Effect of the Invention]

Expression of MASP2 can be suppressed by administering the nucleic acid of the present invention or pharmaceutical composition comprising the nucleic acid. Particularly, it is useful for the treatment or prophylaxis of a disease associated with the expression of MASP2.

### [Description of Embodiments]

As a MASP2 gene (gene encoding MASP2) targeted by the nucleic acid of the present invention, a gene producing a full-length mRNA of MASP2 corresponding to MASP2 cDNA (SEQ ID NO: 2827) registered as Genbank Accession No. NM_006610.3 can be mentioned.

In addition, mRNA of MASP2 gene of biological species other than human can also be a target gene of the nucleic acid of the present invention. For example, Genbank Accession No. NM_001003893.2 can be recited as a cDNA base sequence corresponding to the full-length mRNA of mouse MASP2 gene. Genbank Accession No. NM_172043.1 can be recited as a cDNA base sequence corresponding to the full-length mRNA of rat MASP2 gene. Genbank Accession No. XM-005544812.2 can be recited as a cDNA base sequence corresponding to the full-length mRNA of cynomolgus monkey CFB gene. Genbank Accession No. XM-001118827.3 and the like can be recited as a cDNA base sequence corresponding to the full-length mRNA of rhesus monkey CFB gene.

### 1. Nucleic acid of the present invention

In the present invention, a nucleic acid comprising a nucleotide sequence complementary to MASP2 mRNA is referred to as an antisense strand nucleic acid, and a nucleic acid comprising a nucleotide sequence complementary to a nucleotide sequence of an antisense strand nucleic acid is also referred to as a sense strand nucleic acid. In the present specification, unless otherwise specified, "the nucleic acid of the present invention" is used to encompass antisense strand nucleic acid, sense strand nucleic acid, and double-stranded nucleic acid pairing a sense strand and an antisense strand nucleic acid.

The nucleic acid of the present invention may be any molecule as long as it is a molecule wherein nucleotide or molecule having equivalent function as that of the nucleotide are polymerized. Examples of thereof include RNA which is a polymer of ribonucleotide, DNA which is a polymer of deoxyribonucleotide, chimeric nucleic acid composed of RNA and DNA, and nucleotide polymer wherein at least one nucleotide of these nucleic acids is substituted by a molecule having equivalent function as that of nucleotide. In addition, a derivative containing at least one molecule having equivalent function as that of the nucleotide in these nucleic acids is also encompassed in the nucleic acid of the present invention. Uracil (U) can be unambiguously read as thymine (T).

Examples of the molecule having equivalent function as that of the nucleotide include nucleotide derivatives and the like. The nucleotide derivative may be any molecule as long as it is a molecule obtained by modifying nucleotide. For example, a molecule obtained by modifying ribonucleotide or deoxyribonucleotide and the like to improve or stabilize nuclease resistance, enhance affinity for complementary strand nucleic acid, enhance cell permeability or visualize same, as compared to RNA or DNA, are preferably used.

Examples of the molecule obtained by modifying a nucleotide include sugar moiety-modified nucleotide, phosphodiester bond-modified nucleotide, base-modified nucleotide, a nucleotide wherein at least one of a sugar moiety, a phosphodiester bond and a base is modified and the like.

While the sugar moiety-modified nucleotide may be any as long as the chemical structure of sugar of nucleotide is partly or entirely modified or substituted by any substituent, or substituted by any atom, 2'-modified nucleotide is preferably used.

Examples of the 2'-modified nucleotide include a nucleotide wherein 2'-OH group of ribose is substituted by a substituent selected from H, OR, R, R'OR, SH, SR, NH₂, NHR, NR₂, N₃, CN, F, Cl, Br and I (R is alkyl or aryl, preferably alkyl having 1 - 6 carbon atoms, R' is alkylene, preferably alkylene having 1 - 6 carbon atoms), preferably a nucleotide wherein 2'-OH group is substituted by H, F or methoxy group, more preferably a nucleotide wherein 2'-OH group is substituted by F or methoxy group. In addition, a nucleotide wherein 2'-OH group is substituted by a substituent selected from the group consisting of 2-(methoxy)ethoxy group, 3-aminopropoxy group, 2-[(N,N-dimethylamino)oxy]ethoxy group, 3-(N,N-dimethylamino)propoxy group, 2-[2-(N,N-dimethylamino)ethoxy]ethoxy group, 2-(methylamino)-2-oxoethoxy group, 2-(N-methylcarbamoyl)ethoxy group and 2-cyanoetoxy group, and the like can also be mentioned.

The 2'-modified nucleotide is preferably contained at 50 - 100%, more preferably 70 - 100%, further preferably 90 - 100%, relative to the nucleotide in a double-stranded nucleic acid region.

As the double-stranded nucleic acid to which 2'-modified nucleotides are applied, for example, a double-stranded nucleic acid composed of a pair of sense strand/antisense strand sequences selected from the group consisting of the sense strands/antisense strands described in Table 2 can be mentioned. In Table 2, N(M) shows 2'-O-methyl-RNA and N(F) shows 2'-F-RNA. Here, N shows A, C, G or U.

As the sugar moiety modified nucleotide, a crosslinking structure type artificial nucleic acid having two cyclic structures by introducing a crosslinking structure into the sugar moiety (Bridged Nucleic Acid) (BNA) can be mentioned. Specific examples thereof include locked artificial nucleic acid wherein the 2'-position oxygen atom and the 4'-position carbon atom are crosslinked via methylene (Locked Nucleic Acid) (LNA), ethylene crosslinking structure type artificial nucleic acid (Ethylene bridged nucleic acid) (ENA) [Nucleic Acid Research, 32, e175(2004)], Constrained Ethyl (cEt) [The Journal of Organic Chemistry 75, 1569 (2010)], Amido-Bridged Nucleic Acid (AmNA)[Chem Bio Chem 13, 2513 (2012)] and 2'-O,4'-C-Spirocyclopropylene bridged nucleic acid (scpBNA)[Chem. Commun., 51, 9737 (2015)] and the like, and further, peptide nucleic acid (PNA) [Acc. Chem. Res., 32, 624 (1999)], oxy-peptide nucleic acid (OPNA) [J. Am. Chem. Soc., 123, 4653 (2001)], peptide ribonucleic acid (PRNA) [J. Am. Chem. Soc., 122, 6900 (2000)] and the like.

The phosphodiester bond-modified nucleotide may be any as long as the chemical structure of the phosphodiester bond is partly or entirely modified or substituted by any substituent, or substituted by any atom. Examples thereof include a nucleotide wherein phosphodiester bond is substituted by phosphorothioate bond, a nucleotide wherein phosphodiester bond is substituted by phosphorodithioate bond, a nucleotide wherein phosphodiester bond is substituted by alkylphosphonate bond, a nucleotide wherein phosphodiester bond is substituted by phosphoramidate bond and the like.

The base-modified nucleotide may be any as long as the chemical structure of the base of the nucleotide is partly or entirely modified or substituted by any substituent, or substituted by any atom. Examples thereof include one wherein oxygen atom in the base is substituted by sulfur atom, one wherein hydrogen atom is substituted by alkyl group having 1-6 carbon atoms, halogen and the like, one wherein methyl group is substituted by hydrogen, hydroxymethyl, alkyl group having 2 - 6 carbon atoms and the like, and one wherein amino group is substituted by alkyl group having 1 - 6 carbon atoms, alkanoyl group having 1 - 6 carbon atoms, oxo group, hydroxy group, and the like. Use of 5-methylcytosine (5-mC) as a base-modified nucleotide instead of cytosine (C) is also one of the preferable forms of the present invention.

As the nucleotide derivative, one obtained by adding directly or via linker other chemical substance such as ligand, for example, lipids such as cholesterol, fatty acid, tocopherol, retinoid and the like, saccharides such as N-acetylgalactosamine (GalNAc), galactose (Gal), mannose (Man) and the like, fragment antibodies such as full antibody, Fab, scFv, VHH and the like, proteins such as low-density lipoprotein (LDL), human serum albumin and the like, peptides such as RGD, NGR, R9, CPP and the like, small molecules such as phenazine, phenanthridine, anthraquinone, folic acid and the like, synthesized polymers such as synthetic polyamino acid and the like, nucleic acid aptamers, dyes such as acridine, fluorescein, rhodamine, coumarin and the like, fluorophores such as Cy3 series, Alexa series, Black Hole Quencher and the like, or the like to a nucleotide or a nucleotide derivative wherein at least one of sugar moiety, phosphodiester bond and base is modified can also be mentioned. Specifically, polyamine-added nucleotide derivative, cholesterol-added nucleotide derivative, steroid-added nucleotide derivative, GalNAc-added nucleotide derivative, bile acid-added nucleotide derivative, fatty acid-added nucleotide derivative, vitamin-added nucleotide derivative, Cy5-added nucleotide derivative, Cy3-added nucleotide derivative, 6-FAM-added nucleotide derivative and biotin-added nucleotide derivative and the like can be mentioned, and GalNAc-added nucleotide derivative can be preferably mentioned. It is possible to provide a modification on the 5'-terminus, the 3'-terminus or/and an inner portion of the sequence by reacting, during elongation reaction on a solid phase, a modifier capable of reaction on the solid phase. A nucleotide derivative can also be obtained by synthesizing and purifying, in advance, a nucleic acid introduced with a functional group such as amino group, mercapto group, azido group, triple bond and the like, and reacting same with a modifier.

The nucleotide derivative may form a crosslinking structure, such as alkylene structure, peptide structure, nucleotide structure, ether structure, ester structure, a structure of a combination of at least one of these and the like, with other nucleotide or nucleotide derivative in the nucleic acid.

The nucleic acid of the present invention also encompasses a nucleic acid wherein the atoms in a molecule are partly or entirely substituted by an atom (isotope) having a different mass number.

In the present specification, "complement" means a relationship forming a base pairing between two bases, and refers to a double helix structure as a whole double-stranded region via a loose hydrogen bond, for example, the relationship between adenine and thymine or uracil, and the relationship between guanine and cytosine.

In the present specification, "complementary" means not only complete complementarity between two nucleotide sequences, but also includes 0 - 30%, 0 - 20% or 0 - 10% of mismatch bases between the nucleotide sequences. For example, an antisense strand complementary to MASP2 mRNA may contain substitution of one or more bases in a nucleotide sequence completely complementary to a partial nucleotide sequence of the mRNA. To be specific, an antisense strand may contain 1 - 8, preferably 1 - 6, 1 - 4, 1 - 3, particularly 2 or one mismatch base in a target sequence of the target gene. For example, when the antisense strand has 21 bases in length, it may contain 6, 5, 4, 3, 2 or one mismatch base in a target sequence of the target gene, and the position of the mismatch may be the 5'-terminus or 3'-terminus of the sequence.

In addition, "complementary" encompasses a nucleotide sequence wherein one of the sequences is completely complementary to the other nucleotide sequence, and one or more bases are added and/or deleted. For example, MASP2 mRNA and the antisense strand nucleic acid of the present invention may contain 1 or 2 bulge bases in an antisense strand and/or target MASP2 mRNA region due to the addition and/or deletion of base in the antisense strand.

The nucleic acid of the present invention may be constituted of any nucleotide or a derivative thereof as long as it is a nucleic acid containing a nucleotide sequence complementary to a part of the nucleotide sequence of MASP2 mRNA and/or a nucleic acid containing a nucleotide sequence complementary to the nucleotide sequence of the nucleic acid. The double-stranded nucleic acid of the present invention may have any length as long as a nucleic acid containing a nucleotide sequence complementary to the target MASP2 mRNA sequence and a nucleic acid containing a nucleotide sequence complementary to the nucleotide sequence of the nucleic acid can form a double strand. The length of the sequence capable of forming a double strand is generally 11 - 35 bases, preferably 15 - 30 bases, more preferably 17 - 25 bases, further preferably 17 - 23 bases, particularly preferably 19 - 23 bases. It is also preferable that the sequence consists of 21 - 23 bases.

As the antisense strand nucleic acid of the present invention, a nucleic acid containing a nucleotide sequence complementary to the target MASP2 mRNA sequence is used, wherein 1 - 3 bases, preferably 1 - 2 bases, more preferably 1 base, in the nucleic acid may be deleted, substituted or added.

As a nucleic acid that suppresses expression of MASP2, a single strand nucleic acid containing a nucleotide sequence complementary to the target MASP2 mRNA sequence and capable of suppressing the expression of MASP2, or a double-stranded nucleic acid consisting of a nucleic acid containing a nucleotide sequence complementary to the target MASP2 mRNA sequence and a nucleic acid containing a nucleotide sequence complementary to the nucleotide sequence of the nucleic acid, and capable of suppressing the expression of MASP2 is preferably used.

In the present invention, a double-stranded nucleic acid refers to a nucleic acid wherein two nucleotide strands are paired to form a double-stranded region. The double-stranded region refers to a portion in which a nucleotide or a derivative thereof constituting a double-stranded nucleic acid constitutes a base pair to form a double strand. The double-stranded region generally contains 11 - 27 base pairs, preferably 15 - 25 base pairs, more preferably 15 - 23 base pairs, further preferably 17 - 21 base pairs, particularly preferably 17 - 19 base pairs.

A single strand nucleic acid constituting a double-stranded nucleic acid generally consists of 11 - 30 bases, preferably 15 - 29 bases, more preferably 15 - 27 bases, further preferably 15 - 25 bases, particularly preferably 17 - 23 bases, most preferably 19 - 23 bases. It is also preferable that it consists of 21 - 23 bases.

When the double-stranded nucleic acid of the present invention has an additional nucleotide or nucleotide derivative that does not form a double strand on the 3'-side or 5'-side next to a double-stranded region, it is called an overhang. When an overhang is present, a nucleotide constituting the overhang may be ribonucleotide, deoxyribonucleotide or a derivative thereof.

As a double-stranded nucleic acid having an overhang, one having an overhang of 1 - 6 bases, generally 1 - 3 bases, preferably one having an overhang of 2 bases, for example, overhang composed of dTdT or UU, on the 3'-terminus or 5'-terminus of at least one of the strands is used. The overhang may be present in an antisense strand alone, a sense strand alone, or both an antisense strand and a sense strand. In the present invention, a double-stranded nucleic acid having overhang in both an antisense strand and a sense strand is preferably used. In the antisense strand, an oligonucleotide strand consisting of at least 17 nucleotides and 30 nucleotides at most and comprising a double-stranded region and a subsequent overhang is sufficiently complementary to a target MASP2 mRNA sequence selected from the group described in Table 1 -1 to Table 1 -22 and Table 2. As the double-stranded nucleic acid of the present invention, moreover, a nucleic acid molecule generating the above-mentioned double-stranded nucleic acid by the action of a ribonuclease such as Dicer and the like (WO2005/089287), a double-stranded nucleic acid forming a blunt end without having an overhang on the 3'-terminus or 5'-terminus, a double-stranded nucleic acid with an overhang of a sense strand or antisense strand alone (US2012/0040459) and the like can also be used.

As the double-stranded nucleic acid of the present invention, a nucleic acid consisting of the same sequence as a nucleotide sequence of the target gene or a nucleotide sequence of a complementary strand thereof may be used, or a double-stranded nucleic acid consisting of a nucleic acid wherein 1 - 4 bases on the 5'-terminus or 3'-terminus of at least one of the strands of the nucleic acid is deleted, and a nucleic acid containing a nucleotide sequence complementary to a nucleotide sequence of the nucleic acid may be used.

The double-stranded nucleic acid of the present invention may be a double-stranded RNA (dsRNA) wherein RNAs form a double strand, a double-stranded DNA (dsDNA) wherein DNAs form a double strand, or a hybrid nucleic acid wherein RNA and DNA form a double strand. Alternatively, one or both of the strands of the double strand may be a chimeric nucleic acid of DNA and RNA. Preferred is a double-stranded RNA (dsRNA).

The 2nd nucleotide from the 5'-terminus of the antisense strand of the present invention is preferably complement to the 2nd ribonucleotide from the 3'-terminus of the target MASP2 mRNA sequence, the 2 - 7th nucleotides from the 5'-terminus of the antisense strand is more preferably completely complement to the 2 - 7th ribonucleotides from the 3'-terminus of the target MASP2 mRNA sequence, and the 2 - 11th nucleotides from the 5'-terminus of the antisense strand is further preferably completely complement to the 2 - 11th ribonucleotides from the 3'-terminus of the target MASP2 mRNA sequence. In addition, the 11th nucleotide from the 5'-terminus of the antisense strand of the nucleic acid of the present invention is preferably complement to the 11th ribonucleotide from the 3'-terminus of the target MASP2 mRNA sequence, the 9 - 13th nucleotides from the 5'-terminus of the antisense strand is more preferably completely complement to the 9 - 13th ribonucleotides from the 3'-terminus of the target MASP2 mRNA sequence, and the 7 - 15th nucleotides from the 5'-terminus of the antisense strand is further preferably completely complement to the 7 - 15th ribonucleotides from the 3'-terminus of the target MASP2 mRNA sequence.

A method of producing the nucleic acid of the present invention is not particularly limited, and a method using a known chemical synthesis, or an enzymatic transcription method and the like can be mentioned. As a method using a known chemical synthesis, a phosphoramidite method, a phosphorothioate method, a phosphotriester method, a CEM method [Nucleic Acid Research, 35, 3287 (2007)] and the like can be mentioned and, for example, it can be synthesized by ABI3900 High Throughput nucleic acid synthesizer (manufactured by Applied Biosystems). After completion of the synthesis, desorption from a solid phase, removal of a protecting group, purification of the object product and the like are performed. It is desirable to obtain a nucleic acid having purity of not less than 90%, preferably not less than 95%, by purification. In the case of a double-stranded nucleic acid, synthesized and purified sense strand and antisense strand are mixed at a suitable ratio, for example, 0.1 - 10 equivalents, preferably 0.5 - 2 equivalents, more preferably 0.9 - 1.1 equivalents, further preferably an equivalent molar quantity, of sense strand per 1 equivalent of antisense strand, and may be used after annealing, or directly used without a step of annealing the mixture. Annealing may be performed under any conditions as long as a double-stranded nucleic acid can be formed. It is generally performed by mixing almost equivalent molar quantities of sense strand and antisense strand, heating same at about 94°C for about 5 min and slowly cooling to room temperature. As an enzymatic transcription method for producing the nucleic acid of the present invention, a method using a plasmid or DNA having the object nucleotide sequence as a template, and including transcription using phage RNA polymerase, for example, T7, T3, or SP6RNA polymerase, can be mentioned.

The nucleic acid of the present invention can be introduced into a cell by using a carrier for transfection, preferably a cationic carrier such as cationic liposome and the like. Also, it can be directly introduced into a cell by a calcium phosphate method, an electroporation method, a microinjection method and the like.

In the nucleic acid of the present invention, the 5'-terminus, the 3'-terminus or/and an inner portion of sequence may be modified by one or more ligands and fluorophores, and a nucleic acid modified by a ligand or fluorophore is also called a conjugate nucleic acid. It is possible to provide a modification on the 5'-terminus, the 3'-terminus or/and an inner portion of sequence by reacting, during elongation reaction on a solid phase, a modifier capable of reaction on the solid phase. A conjugate nucleic acid can also be obtained by synthesizing and purifying, in advance, a nucleic acid introduced with a functional group such as amino group, mercapto group, azido group, triple bond and the like, and reacting same with a modifier. While the ligand may be a molecule having affinity for a biological molecule, for example, lipids such as cholesterol, fatty acid, tocopherol, retinoid and the like, saccharides such as N-acetylgalactosamine (GalNAc), galactose (Gal), mannose (Man) and the like, antibodies such as full antibody, Fab, VHH and the like, proteins such as low-density lipoprotein (LDL), human serum albumin and the like, peptides such as RGD, NGR, R9, CPP and the like, small molecules such as folic acid and the like, synthesis polymers such as synthetic polyamino acid and the like, nucleic acid aptamers and the like can be mentioned, and these can also be used in combination. Examples of the fluorophore include Cy3 series, Alexa series, Black Hole Quencher and the like.

A vector capable of expressing the nucleic acid of the present invention after introduction into a cell may be used instead of the nucleic acid of the present invention. To be specific, an expression vector is constructed by inserting a sequence encoding the nucleic acid of the present invention into the downstream of a promoter in the expression vector, and introduced into a cell, whereby the nucleic acid and the like can be expressed. Examples of the expression vector include pCDNA6.2-GW/miR (manufactured by Invitrogen), pSilencer 4.1-CMV (manufactured by Ambion), pSINsi-hH1 DNA (manufactured by Takara Bio Inc.), pSINsi-hU6 DNA (manufactured by Takara Bio Inc.), pENTR/U6 (manufactured by Invitrogen) and the like.

It is also possible to use a recombinant viral vector produced by inserting a sequence encoding the nucleic acid of the present invention into the downstream of a promoter in the expression vector and introducing the vector into a packaging cell. Examples of the viral vector include retroviral vector, lentiviral vector, adenoviral vector, adeno-associated viral vector and the like.

### 2. Nucleic acid having activity to suppress expression of MASP2

The antisense strand and sense strand of the present invention can be designed based on, for example, a nucleotide sequence (SEQ ID NO: 2827) of cDNA (sense strand) of the full length mRNA of human MASP2 registered as Genbank Accession No.NM_006610.3. In addition, antisense and sense strands that suppress expression of MASP2 mRNA in multiple species can also be designed by comparing the base sequence of full length mRNA cDNA of human MASP2 and the base sequence of full length mRNA cDNA of other species.

As a nucleic acid having an activity to suppress expression of MASP2, a double-stranded nucleic acid having an activity to suppress expression of MASP2, which consists of the antisense strand nucleic acid of the present invention containing a nucleotide sequence complementary to MASP2 mRNA, and the sense strand nucleic acid of the present invention containing a nucleotide sequence complementary to the nucleotide sequence of the nucleic acid, can be mentioned. A single strand nucleic acid constituting the double-stranded nucleic acid generally consists of 11 - 30 bases, preferably 15 - 29 bases, more preferably 15 - 27 bases, further preferably 15 - 25 bases, particularly preferably 17 - 23 bases, most preferably 19 - 21 bases. It may also consist of 19 - 23 bases, preferably 21 - 23 bases. The double-stranded nucleic acid has a double-stranded region generally consisting of 15 - 27 base pairs, preferably 15 - 25 base pairs, more preferably 15 - 23 base pairs, further preferably 15 - 21 base pairs.

The expression of MASP2 can be suppressed by introducing these double-stranded nucleic acids into a cell. For example, the double-stranded nucleic acid of the present invention introduced into a cell at a concentration of several pM - several nM can suppress expression of MASP2 mRNA when cultured for not less than 24 hrs, for example, for 48 hrs.

The expression suppressive activity on MASP2 mRNA by the double-stranded nucleic acid of the present invention can be evaluated by transfecting the nucleic acid and the like to a human cell line and the like by using a cationic liposome and the like, culturing same for a given period, and quantifying the expression level of MASP2 mRNA in the human cell line.

As a nucleic acid having an activity to suppress expression of MASP2 besides the above-mentioned double-stranded nucleic acid, a single strand nucleic acid containing a nucleotide sequence complementary to a part of the nucleotide sequence of MASP2 mRNA, and suppress expression of the MASP2 can be mentioned. While a single strand nucleic acid constituting the nucleic acid generally consists of 8 - 30 bases, it preferably consists of 12 - 30 bases, more preferably 12 - 20 bases. It may also consist of 19 - 23 bases, preferably 21 - 23 bases.

These single strand nucleic acids introduced into the cell can also suppress expression of MASP2. For example, the single strand nucleic acid of the present invention introduced into a cell at a concentration of several pM - several nM can suppress expression of MASP2 mRNA when cultured for not less than 24 hrs, for example, for 48 hrs.

The expression suppressive activity on MASP2 mRNA by the single strand nucleic acid of the present invention can be evaluated by transfecting the nucleic acid and the like to a human cell line and the like by using a cationic liposome and the like, culturing same for a given period, and quantifying the expression level of MASP2 mRNA in the human cell line.

### 3. Pharmaceutical composition of the present invention

The present invention also relates to a pharmaceutical composition comprising a nucleic acid such as the above-mentioned double-stranded nucleic acid, single strand nucleic acid and the like as an active ingredient. The pharmaceutical composition of the present invention can be used as a therapeutic or prophylactic agent for autoimmune diseases such as anti-phospholipid antibody syndrome (APS) and systemic lupus erythematosus (SLE), and thrombosis in hemodialysis.

The pharmaceutical composition of the present invention can be preferably used for the treatment or prophylaxis of diseases relating to the expression of MASP2, particularly, disorders mediated by abnormal complement lectin pathway. In the present specification, the disorders mediated by abnormal complement lectin pathway refer to the above-mentioned autoimmune diseases (e.g., APS, SLE), thrombosis in hemodialysis, IgA nephropathy, membranous nephropathy, Lupus nephropathy, C3 nephropathy, thrombotic thrombocytopenic purpura, thrombotic microangiopathy, atypical hemolytic uremic syndrome (aHUS), kidney disorder during ischemia, tissue injury during cerebral infarction, and the like. Therefore, the pharmaceutical composition of the present invention can be used as an agent for treating or preventing IgA nephropathy, membranous nephropathy, lupus nephropathy, C3 nephropathy, thrombotic thrombocytopenic purpura, thrombotic microangiopathy, atypical hemolytic uremic syndrome (aHUS), kidney disorder during ischemia, tissue injury during cerebral infarction and the like.

The pharmaceutical composition can further comprise a carrier effective for intracellular transfer of the nucleic acid. Examples of the carrier effective for intracellular transfer of the nucleic acid include cationic carriers. Examples of the cationic carrier include a cationic liposome, a cationic polymer and the like. As a carrier effective for intracellular transfer of the nucleic acid, a carrier utilizing a virus envelope may also be used. As a cationic polymer, JetSI (Qbiogene Inc.), Jet-PEI (polyethyleneimine; Qbiogene Inc.) and the like are preferably used. As a carrier utilizing a virus envelope, GenomeOne (registered trade mark) (HVJ-E liposome; ISHIHARA SANGYO KAISHA, LTD.) and the like are preferably used.

A composition comprising the nucleic acid of the present invention and the above-mentioned carrier can be prepared by a method known to those of ordinary skill in the art. For example, it can be prepared by mixing a carrier dispersion liquid and a nucleic acid solution at suitable concentrations. When a cationic carrier is used, generally, it can be prepared easily by mixing in an aqueous solution by a conventional method, since a nucleic acid has a negative electric charge in aqueous solutions. Examples of the aqueous solvent used for the preparation of the composition include electrolytic solutions such as water for injection, distilled water for injection, saline and the like, sugar solutions such as glucose solution, maltose solution and the like, and the like. The conditions such as pH and temperature and the like for preparation of the composition can be appropriately selected by those of ordinary skill in the art. Where necessary, the composition can also be formed as a uniform composition by a dispersion treatment using an ultrasonic dispersion apparatus, a high-pressure emulsion apparatus and the like. Since the method and conditions optimal for the preparation of a composition comprising a carrier and a nucleic acid depend on the carrier to be used, those of ordinary skill in the art can select an optimal method for the carrier to be used irrespective of the above-mentioned methods.

As the pharmaceutical composition of the present invention, a composition constituted of a composite particle comprising a nucleic acid and a lead particle as constituent components and a lipid membrane covering the composite particle can also be used preferably. Examples of the lead particle include a lipid assembly, a liposome, an emulsion particle, a polymer, a metal colloid, a fine particle preparation and the like, and a cationic liposome is preferably used. The lead particle in the present invention may contain a complex of a combination of not less than two from a lipid assembly, a liposome, an emulsion particle, a polymer, a metal colloid, a fine particle preparation and the like as a constituent component, or a complex of a combination of a lipid assembly, a liposome, an emulsion particle, a polymer, a metal colloid, a fine particle preparation and the like and other compound (e.g., sugar, lipid, inorganic compound etc.) as a constituent component.

Examples of the lipid membrane covering the composite particle include those comprising non-cationic lipid, lipid inhibiting aggregation of particles and cationic lipid and the like as a constituent component.

The composition can be prepared according to, for example, the method described in WO 2006/080118 and the like.

A suitable mixing ratio of the nucleic acid and the carrier comprised in the pharmaceutical composition of the present invention is 1 - 200 parts by weight of a carrier per 1 part by weight of nucleic acid. It is preferably 2.5 - 100 parts by weight, further preferably 7 - 25 parts by weight, of a carrier per 1 part by weight of a nucleic acid.

An average particle size of the pharmaceutical composition of the present invention is preferably about 10 nm - 300 nm, more preferably about 30 nm - 200 nm, further preferably about 50 nm - 150 nm.

The pharmaceutical composition of the present invention may also comprise a pharmaceutically acceptable carrier, a diluent and the like besides the above-mentioned carrier. A pharmaceutically acceptable carrier, a diluent and the like are essentially chemically-inactive and harmless compositions, and do not at all influence the biological activity of the pharmaceutical composition of the present invention. Examples of the carrier and diluent include, but are not limited to, a salt solution, a sugar solution, a glycerol solution, ethanol and the like.

The pharmaceutical composition of the present invention comprises the complex in an amount effective for the treatment or prevention of diseases and is provided in a form permitting appropriate administration to patients. The formulation of the pharmaceutical composition of the present invention may be, for example, a liquid such as injection, eye drop, inhalation and the like, for example, an external preparation such as ointment, lotion and the like, and the like.

In the case of a liquid, the concentration range of the active ingredient in the pharmaceutical composition of the present invention is generally 0.001 - 25%(w/v), preferably 0.1 - 10%(w/v), more preferably 0.5 - 5%(w/v). The pharmaceutical composition of the present invention may comprise an adequate amount of any pharmaceutically acceptable additive, for example, an emulsion adjuvant, a stabilizer, an isotonicifier, a pH adjuster and the like. Any pharmaceutically acceptable additive can be added in a suitable step before or after dispersion of the complex.

The pH of the liquid is generally adjusted to about 5.0 - about 8.5, preferably about 6.0 - about 8.0, and preferably subjected to a sterilization treatment such as sterilization by filtration and the like, by using a membrane filter and the like.

The pharmaceutical composition of the present invention can also be prepared as a freeze-dried preparation. A freeze-dried preparation can be prepared by a dispersion treatment of a nucleic acid and a carrier, followed by a freeze-drying treatment. A freeze-drying treatment can be performed by a conventional method. For example, a given amount of a complex solution after the above-mentioned dispersion treatment is dispensed in a vial container under sterile conditions, predried for about 2 hrs under the condition of about -40°C to -20°C, primarily predried at about 0 - 10°C under reduced pressure, then secondarily dried at about 15 - 25°C under reduced pressure to perform freeze-drying. Then, for example, the inside of the vial is substituted with a nitrogen gas and a cap is provided, whereby a freeze-dried preparation of the pharmaceutical composition of the present invention can be obtained.

The freeze-dried preparation can be used by redissolving by the addition of any suitable solution. Examples of the solution include electrolytic solutions such as water for injection, saline and the like, glucose solution, other general infusions and the like. While the liquid volume of this solution varies depending on the use and the like and is not particularly limited, it is preferably a 0.5- to 2-fold amount of the liquid volume before freeze-drying, or not more than 500 ml.

The pharmaceutical composition of the present invention can be administered to animals including human by, for example, intravenous administration, intraarterial administration, oral administration, tissue administration, transdermal administration, transmucosal administration or rectal administration, and is preferably administered by an appropriate method according to the symptom of the patient. Particularly, intravenous administration, transdermal administration, and transmucosal administration are preferably used. In addition, topical administration such as topical administration to a cancer site and the like can also be employed. Examples of the dosage form suitable for these administration methods include various injections, oral preparations, drip infusions, absorbents, eye drops, ointments, lotions, suppositories and the like.

While the dose of the pharmaceutical composition of the present invention is desirably determined in consideration of nucleic acid, dosage form, condition of patient such as age, body weight and the like, administration route, nature and severity of the disease and the like, it is generally 0.1 mg - 10 g/day, preferably 1 mg - 500 mg/day, for an adult in the mass of the nucleic acid. In some cases, a dose below these levels may be sufficient, or a dose above these levels may be conversely required. The pharmaceutical composition can be administered one to several times per day, or can be administered at one to several day intervals.

### 4. Treatment method

The present invention further provides a method for treating diseases related to disorders mediated by abnormal complement lectin pathway, including a step of administering a therapeutically effective amount of the nucleic acid of the present invention or the pharmaceutical composition of the present invention to a human in need of such treatment (treatment method of the present invention).

The treatment method of the present invention is preferably a method of treating diseases such as IgA nephropathy, membranous nephropathy, lupus nephropathy, C3 nephropathy, thrombotic thrombocytopenic purpura, thrombotic microangiopathy, atypical hemolytic uremic syndrome (aHUS), kidney disorder during ischemia, tissue injury during cerebral infarction and the like, and is characterized by administering a therapeutically effective amount of the nucleic acid of the present invention or the pharmaceutical composition of the present invention to a human in need of such treatment. Other steps and conditions are not limited in any manner.

In the treatment method of the present invention, for example, the administration method, dose, preparation method and the like of the aforementioned pharmaceutical composition of the present invention can be used.

The present invention is explained in the following by referring to Examples, which are not to be construed as limitative.

### [Example 1]

### Measurement of knockdown activity of MASP2 mRNA in human cell

HepG2/C3A cells (obtained from ATCC, ATCC number: CRL-10741), which is a cell line derived from human liver cancer, were seeded to a 96-well culture plate at 10,000 cells/80 µL/well. As the medium, MEM medium (Nacalai Tesque manufactured by, catalog No.:21442-25) containing 10% fetal bovine serum (FBS), 1 mM sodium pyruvate (manufactured by Nacalai Tesque, catalog No.: 06977-34), 1% MEM Non-Essential Amino Acids (manufactured by Thermo Fisher Scientific, catalog No.: 11140-050) was used. As the double stranded nucleic acids, those described in Table 1-1 to Table 1-22 and synthesized by Sigma-Aldrich were used. Specifically, they are constituted of double-stranded nucleic acids obtained by annealing sense strands consisting of the ribonucleotide shown in SEQ ID NO: 1 - 942 and antisense strands consisting of the ribonucleotide shown in SEQ ID NO: 943 - 1884 (sense strand shown in SEQ ID NO: n (n=1 - 942) and antisense strand shown in SEQ ID NO: [n+942] form a pair). A double-stranded nucleic acid described in Table 1-1 to Table 1-22 and an RNAiMax transfection reagent (manufactured by Thermo Fisher Scientific, catalog No.: 13778-150) were diluted with Opti-MEM medium (manufactured by Thermo Fisher Scientific, catalog No. 31985-070), and 20 µL of each siRNA/RNAiMax mixture was added to 96-well culture plate to the final concentration of double-stranded nucleic acid of 10 nM, and the mixture was cultured under the conditions 37°C, 5% CO₂ for 24 hrs. Thereafter, the cells were washed with PBS (phosphate buffered saline), and cDNA was synthesized from each plate by using SuperPrep (registered trade mark) Cell Lysis & RT Kit for qPCR kit (manufactured by Toyobo, catalog No.: SCQ-101) and according to the method described in the manual attached to the product. The cDNA (4 µL) was added to MicroAmp Optical 384-well plate (manufactured by Applied Biosystems, catalog No. 4309849), and 10 µL of TaqMan (registered trade mark) Gene Expression Master Mix (manufactured by Applied Biosystems, catalog No. 4369016), 4 µL of UltraPure Distilled Water (manufactured by Thermo Fisher Scientific, catalog No.: 10977-015), 1 µL of human MASP2 probe (manufactured by Applied Biosystems, Hs00198244_m1), and 1 µL of human GAPDH probe (manufactured by Applied Biosystems, Hs02786624_g1) were further added. The real-time PCR of human MASP2 gene and human GAPDH (D-glyceraldehyde-3-phosphate dehydrogenase) gene was performed by using the QuantStudio (registered trade mark) 12K Flex real-time PCR system. GAPDH is a constitutively expressed gene and was measured as the internal control, and the MASP2 expression level was normalized. The MASP2 mRNA relative expression level when each double stranded nucleic acid was introduced was calculated relative to the MASP2 mRNA amount when HepG2/C3A cells were treated with a transfection reagent alone without addition of siRNA as 1.0. This experiment was performed 2 or 3 times and the mean of the MASP2 mRNA relative expression level is shown in Table 1-1 to Table 1-22. In the column of note in Table 1-1 to Table 1-22, * shows mean of two experiments and other shows mean of three experiments.

**[Table 1-1]**

| double stranded nucleic acid number | SEQ ID NO: | sense strand sequence (5'--->3') | SEQ ID NO: | antisense strand sequence (5'--->3') | SEQ ID NO: | target MASP2 mRNA sequence | relative expression level | note |
|---|---|---|---|---|---|---|---|---|
| MA0001 | SEQ ID NO: 1 | CAGGCCAGGCCAGCUGGACGG | SEQ ID NO: 943 | GUCCAGCUGGCCUGGCCUGGU | SEQ ID NO: 1885 | CAGGCCAGGCCAGCUGGAC | 0.5565 | * |
| MA0002 | SEQ ID NO: 2 | GCCAGGCCAGCUGGACGGGCA | SEQ ID NO: 944 | CCCGUCCAGCUGGCCUGGCCU | SEQ ID NO: 1886 | GCCAGGCCAGCUGGACGGG | 0.5660 | * |
| MA0003 | SEQ ID NO: 3 | CAGGCCAGCUGGACGGGCACA | SEQ ID NO: 945 | UGCCCGUCCAGCUGGCCUGGC | SEQ ID NO: 1887 | CAGGCCAGCUGGACGGGCA | 0.2343 | |
| MA0004 | SEQ ID NO: 4 | AGGCCAGCUGGACGGGCACAC | SEQ ID NO: 946 | GUGCCCGUCCAGCUGGCCUGG | SEQ ID NO: 1888 | AGGCCAGCUGGACGGGCAC | 0.5005 | * |
| MA0005 | SEQ ID NO: 5 | GGCCAGCUGGACGGGCACACC | SEQ ID NO: 947 | UGUGCCCGUCCAGCUGGCCUG | SEQ ID NO: 1889 | GGCCAGCUGGACGGGCACA | 0.5564 | * |
| MA0006 | SEQ ID NO: 6 | CAGCUGGACGGGCACACCAUG | SEQ ID NO: 948 | UGGUGUGCCCGUCCAGCUGGC | SEQ ID NO: 1890 | CAGCUGGACGGGCACACCA | 0.5744 | * |
| MA0007 | SEQ ID NO: 7 | AGCUGGACGGGCACACCAUGA | SEQ ID NO: 949 | AUGGUGUGCCCGUCCAGCUGG | SEQ ID NO: 1891 | AGCUGGACGGGCACACCAU | 0.2778 | |
| MA0008 | SEQ ID NO: 8 | CUGGACGGGCACACCAUGAGG | SEQ ID NO: 950 | UCAUGGUGUGCCCGUCCAGCU | SEQ ID NO: 1892 | CUGGACGGGCACACCAUGA | 0.4999 | * |
| MA0009 | SEQ ID NO: 9 | GGACGGGCACACCAUGAGGCU | SEQ ID NO: 951 | CCUCAUGGUGUGCCCGUCCAG | SEQ ID NO: 1893 | GGACGGGCACACCAUGAGG | 0.4423 | |
| MA0010 | SEQ ID NO: 10 | ACGGGCACACCAUGAGGCUGC | SEQ ID NO: 952 | AGCCUCAUGGUGUGCCCGUCC | SEQ ID NO: 1894 | ACGGGCACACCAUGAGGCU | 0.5181 | * |
| MA0011 | SEQ ID NO: 11 | CGGGCACACCAUGAGGCUGCU | SEQ ID NO: 953 | CAGCCUCAUGGUGUGCCCGUC | SEQ ID NO: 1895 | CGGGCACACCAUGAGGCUG | 0.4459 | |
| MA0012 | SEQ ID NO: 12 | GGGCACACCAUGAGGCUGCUG | SEQ ID NO: 954 | GCAGCCUCAUGGUGUGCCCGU | SEQ ID NO: 1896 | GGGCACACCAUGAGGCUGC | 0.4654 | |
| MA0013 | SEQ ID NO: 13 | GGCACACCAUGAGGCUGCUGA | SEQ ID NO: 955 | AGCAGCCUCAUGGUGUGCCCG | SEQ ID NO: 1897 | GGCACACCAUGAGGCUGCU | 0.5938 | |
| MA0014 | SEQ ID NO: 14 | CCAUGAGGCUGCUGACCCUCC | SEQ ID NO: 956 | AGGGUCAGCAGCCUCAUGGUG | SEQ ID NO: 1898 | CCAUGAGGCUGCUGACCCU | 0.5938 | |
| MA0015 | SEQ ID NO: 15 | CAUGAGGCUGCUGACCCUCCU | SEQ ID NO: 957 | GAGGGUCAGCAGCCUCAUGGU | SEQ ID NO: 1899 | CAUGAGGCUGCUGACCCUC | 0.5229 | * |
| MA0016 | SEQ ID NO: 16 | GAGGCUGCUGACCCUCCUGGG | SEQ ID NO: 958 | CAGGAGGGUCAGCAGCCUCAU | SEQ ID NO: 1900 | GAGGCUGCUGACCCUCCUG | 0.4945 | |
| MA0017 | SEQ ID NO: 17 | GGCUGCUGACCCUCCUGGGCC | SEQ ID NO: 959 | CCCAGGAGGGUCAGCAGCCUC | SEQ ID NO: 1901 | GGCUGCUGACCCUCCUGGG | 0.3354 | |
| MA0018 | SEQ ID NO: 18 | GCUGCUGACCCUCCUGGGCCU | SEQ ID NO: 960 | GCCCAGGAGGGUCAGCAGCCU | SEQ ID NO: 1902 | GCUGCUGACCCUCCUGGGC | 0.4020 | |
| MA0019 | SEQ ID NO: 19 | CUGCUGACCCUCCUGGGCCUU | SEQ ID NO: 961 | GGCCCAGGAGGGUCAGCAGCC | SEQ ID NO: 1903 | CUGCUGACCCUCCUGGGCC | 0.5934 | * |
| MA0020 | SEQ ID NO: 20 | UGCUGACCCUCCUGGGCCUUC | SEQ ID NO: 962 | AGGCCCAGGAGGGUCAGCAGC | SEQ ID NO: 1904 | UGCUGACCCUCCUGGGCCU | 0.1989 | |
| MA0021 | SEQ ID NO: 21 | CUGACCCUCCUGGGCCUUCUG | SEQ ID NO: 963 | GAAGGCCCAGGAGGGUCAGCA | SEQ ID NO: 1905 | CUGACCCUCCUGGGCCUUC | 0.3474 | |
| MA0022 | SEQ ID NO: 22 | UGACCCUCCUGGGCCUUCUGU | SEQ ID NO: 964 | AGAAGGCCCAGGAGGGUCAGC | SEQ ID NO: 1906 | UGACCCUCCUGGGCCUUCU | 0.2850 | |
| MA0023 | SEQ ID NO: 23 | CCCUCCUGGGCCUUCUGUGUG | SEQ ID NO: 965 | CACAGAAGGCCCAGGAGGGUC | SEQ ID NO: 1907 | CCCUCCUGGGCCUUCUGUG | 0.5761 | |
| MA0024 | SEQ ID NO: 24 | CCUCCUGGGCCUUCUGUGUGG | SEQ ID NO: 966 | ACACAGAAGGCCCAGGAGGGU | SEQ ID NO: 1908 | CCUCCUGGGCCUUCUGUGU | 0.5151 | * |
| MA0025 | SEQ ID NO: 25 | CUGGGCCUUCUGUGUGGCUCG | SEQ ID NO: 967 | AGCCACACAGAAGGCCCAGGA | SEQ ID NO: 1909 | CUGGGCCUUCUGUGUGGCU | 0.5209 | * |
| MA0026 | SEQ ID NO: 26 | GGGCCUUCUGUGUGGCUCGGU | SEQ ID NO: 968 | CGAGCCACACAGAAGGCCCAG | SEQ ID NO: 1910 | GGGCCUUCUGUGUGGCUCG | 0.3202 | |
| MA0027 | SEQ ID NO: 27 | GGCCUUCUGUGUGGCUCGGUG | SEQ ID NO: 969 | CCGAGCCACACAGAAGGCCCA | SEQ ID NO: 1911 | GGCCUUCUGUGUGGCUCGG | 0.5230 | * |
| MA0028 | SEQ ID NO: 28 | CCUUCUGUGUGGCUCGGUGGC | SEQ ID NO: 970 | CACCGAGCCACACAGAAGGCC | SEQ ID NO: 1912 | CCUUCUGUGUGGCUCGGUG | 0.3391 | |
| MA0029 | SEQ ID NO: 29 | CUUCUGUGUGGCUCGGUGGCC | SEQ ID NO: 971 | CCACCGAGCCACACAGAAGGC | SEQ ID NO: 1913 | CUUCUGUGUGGCUCGGUGG | 0.5886 | * |
| MA0030 | SEQ ID NO: 30 | CUGUGUGGCUCGGUGGCCACC | SEQ ID NO: 972 | UGGCCACCGAGCCACACAGAA | SEQ ID NO: 1914 | CUGUGUGGCUCGGUGGCCA | 0.3355 | |
| MA0031 | SEQ ID NO: 31 | GUGUGGCUCGGUGGCCACCCC | SEQ ID NO: 973 | GGUGGCCACCGAGCCACACAG | SEQ ID NO: 1915 | GUGUGGCUCGGUGGCCACC | 0.5075 | * |
| MA0032 | SEQ ID NO: 32 | GCUCGGUGGCCACCCCCUUGG | SEQ ID NO: 974 | AAGGGGGUGGCCACCGAGCCA | SEQ ID NO: 1916 | GCUCGGUGGCCACCCCCUU | 0.4531 | * |
| MA0033 | SEQ ID NO: 33 | CUCGGUGGCCACCCCCUUGGG | SEQ ID NO: 975 | CAAGGGGGUGGCCACCGAGCC | SEQ ID NO: 1917 | CUCGGUGGCCACCCCCUUG | 0.5918 | |
| MA0034 | SEQ ID NO: 34 | CCACCCCCUUGGGCCCGAAGU | SEQ ID NO: 976 | UUCGGGCCCAAGGGGGUGGCC | SEQ ID NO: 1918 | CCACCCCCUUGGGCCCGAA | 0.5778 | * |
| MA0035 | SEQ ID NO: 35 | CCCCCUUGGGCCCGAAGUGGC | SEQ ID NO: 977 | CACUUCGGGCCCAAGGGGGUG | SEQ ID NO: 1919 | CCCCCUUGGGCCCGAAGUG | 0.3954 | |
| MA0036 | SEQ ID NO: 36 | GGCCCGAAGUGGCCUGAACCU | SEQ ID NO: 978 | GUUCAGGCCACUUCGGGCCCA | SEQ ID NO: 1920 | GGCCCGAAGUGGCCUGAAC | 0.2172 | |
| MA0037 | SEQ ID NO: 37 | GCCCGAAGUGGCCUGAACCUG | SEQ ID NO: 979 | GGUUCAGGCCACUUCGGGCCC | SEQ ID NO: 1921 | GCCCGAAGUGGCCUGAACC | 0.4503 | |
| MA0038 | SEQ ID NO: 38 | CCCGAAGUGGCCUGAACCUGU | SEQ ID NO: 980 | AGGUUCAGGCCACUUCGGGCC | SEQ ID NO: 1922 | CCCGAAGUGGCCUGAACCU | 0.4177 | |
| MA0039 | SEQ ID NO: 39 | CCGAAGUGGCCUGAACCUGUG | SEQ ID NO: 981 | CAGGUUCAGGCCACUUCGGGC | SEQ ID NO: 1923 | CCGAAGUGGCCUGAACCUG | 0.4746 | |
| MA0040 | SEQ ID NO: 40 | CGAAGUGGCCUGAACCUGUGU | SEQ ID NO: 982 | ACAGGUUCAGGCCACUUCGGG | SEQ ID NO: 1924 | CGAAGUGGCCUGAACCUGU | 0.3387 | |
| MA0041 | SEQ ID NO: 41 | AAGUGGCCUGAACCUGUGUUC | SEQ ID NO: 983 | ACACAGGUUCAGGCCACUUCG | SEQ ID NO: 1925 | AAGUGGCCUGAACCUGUGU | 0.5072 | |
| MA0042 | SEQ ID NO: 42 | AGUGGCCUGAACCUGUGUUCG | SEQ ID NO: 984 | AACACAGGUUCAGGCCACUUC | SEQ ID NO: 1926 | AGUGGCCUGAACCUGUGUU | 0.3258 | |
| MA0043 | SEQ ID NO: 43 | GUGGCCUGAACCUGUGUUCGG | SEQ ID NO: 985 | GAACACAGGUUCAGGCCACUU | SEQ ID NO: 1927 | GUGGCCUGAACCUGUGUUC | 0.2609 | |
| MA0044 | SEQ ID NO: 44 | UGGCCUGAACCUGUGUUCGGG | SEQ ID NO: 986 | CGAACACAGGUUCAGGCCACU | SEQ ID NO: 1928 | UGGCCUGAACCUGUGUUCG | 0.5049 | * |

**[Table 1-2]**

| double stranded nucleic acid number | SEQ ID NO: | sense strand sequence (5'--->3') | SEQ ID NO: | antisense strand sequence (5'--->3') | SEQ ID NO: | target MASP2 mRNA sequence | relative expression level | note |
|---|---|---|---|---|---|---|---|---|
| MA0045 | SEQ ID NO: 45 | GGCCUGAACCUGUGUUCGGGC | SEQ ID NO: 987 | CCGAACACAGGUUCAGGCCAC | SEQ ID NO: 1929 | GGCCUGAACCUGUGUUCGG | 0.3877 | |
| MA0045 | SEQ ID NO: 45 | GGCCUGAACCUGUGUUCGGGC | SEQ ID NO: 987 | CCGAACACAGGUUCAGGCCAC | SEQ ID NO: 1929 | GGCCUGAACCUGUGUUCGG | 0.3877 | |
| MA0046 | SEQ ID NO: 46 | GCCUGAACCUGUGUUCGGGCG | SEQ ID NO: 988 | CCCGAACACAGGUUCAGGCCA | SEQ ID NO: 1930 | GCCUGAACCUGUGUUCGGG | 0.3492 | |
| MA0047 | SEQ ID NO: 47 | CCUGAACCUGUGUUCGGGCGC | SEQ ID NO: 989 | GCCCGAACACAGGUUCAGGCC | SEQ ID NO: 1931 | CCUGAACCUGUGUUCGGGC | 0.5680 | * |
| MA0048 | SEQ ID NO: 48 | GAACCUGUGUUCGGGCGCCUG | SEQ ID NO: 990 | GGCGCCCGAACACAGGUUCAG | SEQ ID NO: 1932 | GAACCUGUGUUCGGGCGCC | 0.3936 | |
| MA0049 | SEQ ID NO: 49 | ACCUGUGUUCGGGCGCCUGGC | SEQ ID NO: 991 | CAGGCGCCCGAACACAGGUUC | SEQ ID NO: 1933 | ACCUGUGUUCGGGCGCCUG | 0.4534 | |
| MA0050 | SEQ ID NO: 50 | GUGUUCGGGCGCCUGGCAUCC | SEQ ID NO: 992 | AUGCCAGGCGCCCGAACACAG | SEQ ID NO: 1934 | GUGUUCGGGCGCCUGGCAU | 0.5830 | * |
| MA0051 | SEQ ID NO: 51 | CCUGGCAUCCCCCGGCUUUCC | SEQ ID NO: 993 | AAAGCCGGGGGAUGCCAGGCG | SEQ ID NO: 1935 | CCUGGCAUCCCCCGGCUUU | 0.4024 | |
| MA0052 | SEQ ID NO: 52 | CUGGCAUCCCCCGGCUUUCCA | SEQ ID NO: 994 | GAAAGCCGGGGGAUGCCAGGC | SEQ ID NO: 1936 | CUGGCAUCCCCCGGCUUUC | 0.4241 | |
| MA0053 | SEQ ID NO: 53 | CCGGCUUUCCAGGGGAGUAUG | SEQ ID NO: 995 | UACUCCCCUGGAAAGCCGGGG | SEQ ID NO: 1937 | CCGGCUUUCCAGGGGAGUA | 0.2817 | |
| MA0054 | SEQ ID NO: 54 | CGGCUUUCCAGGGGAGUAUGC | SEQ ID NO: 996 | AUACUCCCCUGGAAAGCCGGG | SEQ ID NO: 1938 | CGGCUUUCCAGGGGAGUAU | 0.3568 | |
| MA0055 | SEQ ID NO: 55 | GGCUUUCCAGGGGAGUAUGCC | SEQ ID NO: 997 | CAUACUCCCCUGGAAAGCCGG | SEQ ID NO: 1939 | GGCUUUCCAGGGGAGUAUG | 0.4706 | |
| MA0056 | SEQ ID NO: 56 | UUUCCAGGGGAGUAUGCCAAU | SEQ ID NO: 998 | UGGCAUACUCCCCUGGAAAGC | SEQ ID NO: 1940 | UUUCCAGGGGAGUAUGCCA | 0.5581 | |
| MA0057 | SEQ ID NO: 57 | CAGGGGAGUAUGCCAAUGACC | SEQ ID NO: 999 | UCAUUGGCAUACUCCCCUGGA | SEQ ID NO: 1941 | CAGGGGAGUAUGCCAAUGA | 0.4027 | |
| MA0058 | SEQ ID NO: 58 | AGGGGAGUAUGCCAAUGACCA | SEQ ID NO: 1000 | GUCAUUGGCAUACUCCCCUGG | SEQ ID NO: 1942 | AGGGGAGUAUGCCAAUGAC | 0.5934 | * |
| MA0059 | SEQ ID NO: 59 | GGGGAGUAUGCCAAUGACCAG | SEQ ID NO: 1001 | GGUCAUUGGCAUACUCCCCUG | SEQ ID NO: 1943 | GGGGAGUAUGCCAAUGACC | 0.5499 | |
| MA0060 | SEQ ID NO: 60 | GGGAGUAUGCCAAUGACCAGG | SEQ ID NO: 1002 | UGGUCAUUGGCAUACUCCCCU | SEQ ID NO: 1944 | GGGAGUAUGCCAAUGACCA | 0.4827 | |
| MA0061 | SEQ ID NO: 61 | GGAGUAUGCCAAUGACCAGGA | SEQ ID NO: 1003 | CUGGUCAUUGGCAUACUCCCC | SEQ ID NO: 1945 | GGAGUAUGCCAAUGACCAG | 0.5511 | * |
| MA00 62 | SEQ ID NO: 62 | GAGUAUGCCAAUGACCAGGAG | SEQ ID NO: 1004 | CCUGGUCAUUGGCAUACUCCC | SEQ ID NO: 1946 | GAGUAUGCCAAUGACCAGG | 0.4938 | |
| MA0063 | SEQ ID NO: 63 | GCCAAUGACCAGGAGCGGCGC | SEQ ID NO: 1005 | GCCGCUCCUGGUCAUUGGCAU | SEQ ID NO: 1947 | GCCAAUGACCAGGAGCGGC | 0.5419 | |
| MA0064 | SEQ ID NO: 64 | CCAAUGACCAGGAGCGGCGCU | SEQ ID NO: 1006 | CGCCGCUCCUGGUCAUUGGCA | SEQ ID NO: 1948 | CCAAUGACCAGGAGCGGCG | 0.4878 | |
| MA0065 | SEQ ID NO: 65 | CAAUGACCAGGAGCGGCGCUG | SEQ ID NO: 1007 | GCGCCGCUCCUGGUCAUUGGC | SEQ ID NO: 1949 | CAAUGACCAGGAGCGGCGC | 0.5854 | * |
| MA0066 | SEQ ID NO: 66 | AUGACCAGGAGCGGCGCUGGA | SEQ ID NO: 1008 | CAGCGCCGCUCCUGGUCAUUG | SEQ ID NO: 1950 | AUGACCAGGAGCGGCGCUG | 0.5111 | * |
| MA0067 | SEQ ID NO: 67 | ACCAGGAGCGGCGCUGGACCC | SEQ ID NO: 1009 | GUCCAGCGCCGCUCCUGGUCA | SEQ ID NO: 1951 | ACCAGGAGCGGCGCUGGAC | 0.5592 | * |
| MA0068 | SEQ ID NO: 68 | GGAGCGGCGCUGGACCCUGAC | SEQ ID NO: 1010 | CAGGGUCCAGCGCCGCUCCUG | SEQ ID NO: 1952 | GGAGCGGCGCUGGACCCUG | 0.5431 | * |
| MA0069 | SEQ ID NO: 69 | GAGCGGCGCUGGACCCUGACU | SEQ ID NO: 1011 | UCAGGGUCCAGCGCCGCUCCU | SEQ ID NO: 1953 | GAGCGGCGCUGGACCCUGA | 0.3272 | |
| MA0070 | SEQ ID NO: 70 | AGCGGCGCUGGACCCUGACUG | SEQ ID NO: 1012 | GUCAGGGUCCAGCGCCGCUCC | SEQ ID NO: 1954 | AGCGGCGCUGGACCCUGAC | 0.4880 | * |
| MA0071 | SEQ ID NO: 71 | GCGGCGCUGGACCCUGACUGC | SEQ ID NO: 1013 | AGUCAGGGUCCAGCGCCGCUC | SEQ ID NO: 1955 | GCGGCGCUGGACCCUGACU | 0.3677 | |
| MA0072 | SEQ ID NO: 72 | CGGCGCUGGACCCUGACUGCA | SEQ ID NO: 1014 | CAGUCAGGGUCCAGCGCCGCU | SEQ ID NO: 1956 | CGGCGCUGGACCCUGACUG | 0.4002 | |
| MA0073 | SEQ ID NO: 73 | GGCGCUGGACCCUGACUGCAC | SEQ ID NO: 1015 | GCAGUCAGGGUCCAGCGCCGC | SEQ ID NO: 1957 | GGCGCUGGACCCUGACUGC | 0.5808 | |
| MA0074 | SEQ ID NO: 74 | GCGCUGGACCCUGACUGCACC | SEQ ID NO: 1016 | UGCAGUCAGGGUCCAGCGCCG | SEQ ID NO: 1958 | GCGCUGGACCCUGACUGCA | 0.2831 | |
| MA0075 | SEQ ID NO: 75 | CGCUGGACCCUGACUGCACCC | SEQ ID NO: 1017 | GUGCAGUCAGGGUCCAGCGCC | SEQ ID NO: 1959 | CGCUGGACCCUGACUGCAC | 0.4658 | * |
| MA0076 | SEQ ID NO: 76 | GCUGGACCCUGACUGCACCCC | SEQ ID NO: 1018 | GGUGCAGUCAGGGUCCAGCGC | SEQ ID NO: 1960 | GCUGGACCCUGACUGCACC | 0.3838 | |
| MA0077 | SEQ ID NO: 77 | GGACCCUGACUGCACCCCCCG | SEQ ID NO: 1019 | GGGGGUGCAGUCAGGGUCCAG | SEQ ID NO: 1961 | GGACCCUGACUGCACCCCC | 0.4356 | |
| MA0078 | SEQ ID NO: 78 | GACCCUGACUGCACCCCCCGG | SEQ ID NO: 1020 | GGGGGGUGCAGUCAGGGUCCA | SEQ ID NO: 1962 | GACCCUGACUGCACCCCCC | 0.3597 | |
| MA0079 | SEQ ID NO: 79 | GACUGCACCCCCCGGCUACCG | SEQ ID NO: 1021 | GUAGCCGGGGGGUGCAGUCAG | SEQ ID NO: 1963 | GACUGCACCCCCCGGCUAC | 0.5523 | |
| MA0080 | SEQ ID NO: 80 | CCGGCUACCGCCUGCGCCUCU | SEQ ID NO: 1022 | AGGCGCAGGCGGUAGCCGGGG | SEQ ID NO: 1964 | CCGGCUACCGCCUGCGCCU | 0.5702 | * |
| MA0081 | SEQ ID NO: 81 | GGCUACCGCCUGCGCCUCUAC | SEQ ID NO: 1023 | AGAGGCGCAGGCGGUAGCCGG | SEQ ID NO: 1965 | GGCUACCGCCUGCGCCUCU | 0.4539 | |
| MA0082 | SEQ ID NO: 82 | GCUACCGCCUGCGCCUCUACU | SEQ ID NO: 1024 | UAGAGGCGCAGGCGGUAGCCG | SEQ ID NO: 1966 | GCUACCGCCUGCGCCUCUA | 0.1727 | |
| MA0083 | SEQ ID NO: 83 | CUACCGCCUGCGCCUCUACUU | SEQ ID NO: 1025 | GUAGAGGCGCAGGCGGUAGCC | SEQ ID NO: 1967 | CUACCGCCUGCGCCUCUAC | 0.5262 | * |
| MA0084 | SEQ ID NO: 84 | ACCGCCUGCGCCUCUACUUCA | SEQ ID NO: 1026 | AAGUAGAGGCGCAGGCGGUAG | SEQ ID NO: 1968 | ACCGCCUGCGCC UCUACUU | 0.3332 | |
| MA0085 | SEQ ID NO: 85 | CCGCCUGCGCCUCUACUUCAC | SEQ ID NO: 1027 | GAAGUAGAGGCGCAGGCGGUA | SEQ ID NO: 1969 | CCGCCUGCGCCUCUACUUC | 0.4093 | |
| MA0086 | SEQ ID NO: 86 | CGCCUGCGCCUCUACUUCACC | SEQ ID NO: 1028 | UGAAGUAGAGGCGCAGGCGGU | SEQ ID NO: 1970 | CGCCUGCGCCUCUACUUCA | 0.2987 | |
| MA0087 | SEQ ID NO: 87 | GCCUGCGCCUCUACUUCACCC | SEQ ID NO: 1029 | GUGAAGUAGAGGCGCAGGCGG | SEQ ID NO: 1971 | GCCUGCGCCUCUACUUCAC | 0.2198 | |
| MA0088 | SEQ ID NO: 88 | CCUGCGCCUCUACUUCACCCA | SEQ ID NO: 1030 | GGUGAAGUAGAGGCGCAGGCG | SEQ ID NO: 1972 | CCUGCGCCUCUACUUCACC | 0.4851 | |

**[Table 1-3]**

| double stranded nucleic acid number | SEQ ID NO: | sense strand sequence (5' --->3') | SEQ ID NO: | antisense strand sequence (5' --->3') | SEQ ID NO: | target MASP2 mRNA sequence | relative expression level | note |
|---|---|---|---|---|---|---|---|---|
| MA0089 | SEQ ID NO: 89 | CUGCGCCUCUACUUCACCCAC | SEQ ID NO: 1031 | GGGUGAAGUAGAGGCGCAGGC | SEQ ID NO: 1973 | CUGCGCCUCUACUUCACCC | 0.4230 | |
| MA0090 | SEQ ID NO: 90 | UGCGCCUCUACUUCACCCACU | SEQ ID NO: 1032 | UGGGUGAAGUAGAGGCGCAGG | SEQ ID NO: 1974 | UGCGCCUCUACUUCACCCA | 0.4596 | |
| MA0091 | SEQ ID NO: 91 | CGCCUCUACUUCACCCACUUC | SEQ ID NO: 1033 | AGUGGGUGAAGUAGAGGCGCA | SEQ ID NO: 1975 | CGCCUCUACUUCACCCACU | 0.3119 | |
| MA0092 | SEQ ID NO: 92 | GCCUCUACUUCACCCACUUCG | SEQ ID NO: 1034 | AAGUGGGUGAAGUAGAGGCGC | SEQ ID NO: 1976 | GCCUCUACUUCACCCACUU | 0.4140 | |
| MA0093 | SEQ ID NO: 93 | CCUCUACUUCACCCACUUCGA | SEQ ID NO: 1035 | GAAGUGGGUGAAGUAGAGGCG | SEQ ID NO: 1977 | CCUCUACUUCACCCACUUC | 0.4361 | |
| MA0094 | SEQ ID NO: 94 | CUCUACUUCACCCACUUCGAC | SEQ ID NO: 1036 | CGAAGUGGGUGAAGUAGAGGC | SEQ ID NO: 1978 | CUCUACUUCACCCACUUCG | 0.5968 | * |
| MA0095 | SEQ ID NO: 95 | UCUACUUCACCCACUUCGACC | SEQ ID NO: 1037 | UCGAAGUGGGUGAAGUAGAGG | SEQ ID NO: 1979 | UCUACUUCACCCACUUCGA | 0.2870 | |
| MA0096 | SEQ ID NO: 96 | CUACUUCACCCACUUCGACCU | SEQ ID NO: 1038 | GUCGAAGUGGGUGAAGUAGAG | SEQ ID NO: 1980 | CUACUUCACCCACUUCGAC | 0.3223 | |
| MA0097 | SEQ ID NO: 97 | ACUUCACCCACUUCGACCUGG | SEQ ID NO: 1039 | AGGUCGAAGUGGGUGAAGUAG | SEQ ID NO: 1981 | ACUUCACCCACUUCGACCU | 0.3548 | |
| MA0098 | SEQ ID NO: 98 | CUUCACCCACUUCGACCUGGA | SEQ ID NO: 1040 | CAGGUCGAAGUGGGUGAAGUA | SEQ ID NO: 1982 | CUUCACCCACUUCGACCUG | 0.5948 | * |
| MA0099 | SEQ ID NO: 99 | UCACCCACUUCGACCUGGAGC | SEQ ID NO: 1041 | UCCAGGUCGAAGUGGGUGAAG | SEQ ID NO: 1983 | UCACCCACUUCGACCUGGA | 0.2592 | |
| MA0100 | SEQ ID NO: 100 | CCCACUUCGACCUGGAGCUCU | SEQ ID NO: 1042 | AGCUCCAGGUCGAAGUGGGUG | SEQ ID NO: 1984 | CCCACUUCGACCUGGAGCU | 0.3671 | |
| MA0101 | SEQ ID NO: 101 | CACUUCGACCUGGAGCUCUCC | SEQ ID NO: 1043 | AGAGCUCCAGGUCGAAGUGGG | SEQ ID NO: 1985 | CACUUCGACCUGGAGCUCU | 0.3351 | |
| MA0102 | SEQ ID NO: 102 | CGACCUGGAGCUCUCCCACCU | SEQ ID NO: 1044 | GUGGGAGAGCUCCAGGUCGAA | SEQ ID NO: 1986 | CGACCUGGAGCUCUCCCAC | 0.5289 | |
| MA0103 | SEQ ID NO: 103 | CCUGGAGCUCUCCCACCUCUG | SEQ ID NO: 1045 | GAGGUGGGAGAGCUCCAGGUC | SEQ ID NO: 1987 | CCUGGAGCUCUCCCACCUC | 0.2921 | |
| MA0104 | SEQ ID NO: 104 | GGAGCUCUCCCACCUCUGCGA | SEQ ID NO: 1046 | GCAGAGGUGGGAGAGCUCCAG | SEQ ID NO: 1988 | GGAGCUCUCCCACCUCUGC | 0.3509 | |
| MA0105 | SEQ ID NO: 105 | GAGCUCUCCCACCUCUGCGAG | SEQ ID NO: 1047 | CGCAGAGGUGGGAGAGCUCCA | SEQ ID NO: 1989 | GAGCUCUCCCACCUCUGCG | 0.5587 | * |
| MA0106 | SEQ ID NO: 106 | AGCUCUCCCACCUCUGCGAGU | SEQ ID NO: 1048 | UCGCAGAGGUGGGAGAGCUCC | SEQ ID NO: 1990 | AGCUCUCCCACCUCUGCGA | 0.4287 | |
| MA0107 | SEQ ID NO: 107 | UCUCCCACCUCUGCGAGUACG | SEQ ID NO: 1049 | UACUCGCAGAGGUGGGAGAGC | SEQ ID NO: 1991 | UCUCCCACCUCUGCGAGUA | 0.4939 | * |
| MA0108 | SEQ ID NO: 108 | CUCCCACCUCUGCGAGUACGA | SEQ ID NO: 1050 | GUACUCGCAGAGGUGGGAGAG | SEQ ID NO: 1992 | CUCCCACCUCUGCGAGUAC | 0.4280 | |
| MA0109 | SEQ ID NO: 109 | CCCACCUCUGCGAGUACGACU | SEQ ID NO: 1051 | UCGUACUCGCAGAGGUGGGAG | SEQ ID NO: 1993 | CCCACCUCUGCGAGUACGA | 0.2889 | |
| MA0110 | SEQ ID NO: 110 | CCACCUCUGCGAGUACGACUU | SEQ ID NO: 1052 | GUCGUACUCGCAGAGGUGGGA | SEQ ID NO: 1994 | CCACCUCUGCGAGUACGAC | 0.3150 | |
| MA0111 | SEQ ID NO: 111 | ACCUCUGCGAGUACGACUUCG | SEQ ID NO: 1053 | AAGUCGUACUCGCAGAGGUGG | SEQ ID NO: 1995 | ACCUCUGCGAGUACGACUU | 0.2940 | |
| MA0112 | SEQ ID NO: 112 | CCUCUGCGAGUACGACUUCGU | SEQ ID NO: 1054 | GAAGUCGUACUCGCAGAGGUG | SEQ ID NO: 1996 | CCUCUGCGAGUACGACUUC | 0.4900 | * |
| MA0113 | SEQ ID NO: 113 | UCUGCGAGUACGACUUCGUCA | SEQ ID NO: 1055 | ACGAAGUCGUACUCGCAGAGG | SEQ ID NO: 1997 | UCUGCGAGUACGACUUCGU | 0.4205 | |
| MA0114 | SEQ ID NO: 114 | CUGCGAGUACGACUUCGUCAA | SEQ ID NO: 1056 | GACGAAGUCGUACUCGCAGAG | SEQ ID NO: 1998 | CUGCGAGUACGACUUCGUC | 0.5010 | |
| MA0115 | SEQ ID NO: 115 | GCGAGUACGACUUCGUCAAGC | SEQ ID NO: 1057 | UUGACGAAGUCGUACUCGCAG | SEQ ID NO: 1999 | GCGAGUACGACUUCGUCAA | 0.4090 | |
| MA0116 | SEQ ID NO: 116 | CGAGUACGACUUCGUCAAGCU | SEQ ID NO: 1058 | CUUGACGAAGUCGUACUCGCA | SEQ ID NO: 2000 | CGAGUACGACUUCGUCAAG | 0.5685 | |
| MA0117 | SEQ ID NO: 117 | GAGUACGACUUCGUCAAGCUG | SEQ ID NO: 1059 | GCUUGACGAAGUCGUACUCGC | SEQ ID NO: 2001 | GAGUACGACUUCGUCAAGC | 0.3707 | |
| MA0118 | SEQ ID NO: 118 | AGUACGACUUCGUCAAGCUGA | SEQ ID NO: 1060 | AGCUUGACGAAGUCGUACUCG | SEQ ID NO: 2002 | AGUACGACUUCGUCAAGCU | 0.2847 | |
| MA0119 | SEQ ID NO: 119 | GUACGACUUCGUCAAGCUGAG | SEQ ID NO: 1061 | CAGCUUGACGAAGUCGUACUC | SEQ ID NO: 2003 | GUACGACUUCGUCAAGCUG | 0.4394 | * |
| MA0120 | SEQ ID NO: 120 | ACGACUUCGUCAAGCUGAGCU | SEQ ID NO: 1062 | CUCAGCUUGACGAAGUCGUAC | SEQ ID NO: 2004 | ACGACUUCGUCAAGCUGAG | 0.3891 | |
| MA0121 | SEQ ID NO: 121 | CGACUUCGUCAAGCUGAGCUC | SEQ ID NO: 1063 | GCUCAGCUUGACGAAGUCGUA | SEQ ID NO: 2005 | CGACUUCGUCAAGCUGAGC | 0.5477 | |
| MA0122 | SEQ ID NO: 122 | GACUUCGUCAAGCUGAGCUCG | SEQ ID NO: 1064 | AGCUCAGCUUGACGAAGUCGU | SEQ ID NO: 2006 | GACUUCGUCAAGCUGAGCU | 0.5264 | * |
| MA0123 | SEQ ID NO: 123 | CGUCAAGCUGAGCUCGGGGGC | SEQ ID NO: 1065 | CCCCGAGCUCAGCUUGACGAA | SEQ ID NO: 2007 | CGUCAAGCUGAGCUCGGGG | 0.3898 | |
| MA0124 | SEQ ID NO: 124 | CCAAGGUGCUGGCCACGCUGU | SEQ ID NO: 1066 | AGCGUGGCCAGCACCUUGGCC | SEQ ID NO: 2008 | CCAAGGUGCUGGCCACGCU | 0.5599 | |
| MA0125 | SEQ ID NO: 125 | UGGCCACGCUGUGCGGGCAGG | SEQ ID NO: 1067 | UGCCCGCACAGCGUGGCCAGC | SEQ ID NO: 2009 | UGGCCACGCUGUGCGGGCA | 0.4506 | |
| MA0126 | SEQ ID NO: 126 | CCACGCUGUGCGGGCAGGAGA | SEQ ID NO: 1068 | UCCUGCCCGCACAGCGUGGCC | SEQ ID NO: 2010 | CCACGCUGUGCGGGCAGGA | 0.5102 | * |
| MA0127 | SEQ ID NO: 127 | GUGCGGGCAGGAGAGCACAGA | SEQ ID NO: 1069 | UGUGCUCUCCUGCCCGCACAG | SEQ ID NO: 2011 | GUGCGGGCAGGAGAGCACA | 0.3473 | |
| MA0128 | SEQ ID NO: 128 | GGCAGGAGAGCACAGACACGG | SEQ ID NO: 1070 | GUGUCUGUGCUCUCCUGCCCG | SEQ ID NO: 2012 | GGCAGGAGAGCACAGACAC | 0.3693 | |
| MA0129 | SEQ ID NO: 129 | GCAGGAGAGCACAGACACGGA | SEQ ID NO: 1071 | CGUGUCUGUGCUCUCCUGCCC | SEQ ID NO: 2013 | GCAGGAGAGCACAGACACG | 0.2405 | |
| MA0130 | SEQ ID NO: 130 | AGGAGAGCACAGACACGGAGC | SEQ ID NO: 1072 | UCCGUGUCUGUGCUCUCCUGC | SEQ ID NO: 2014 | AGGAGAGCACAGACACGGA | 0.2569 | |
| MA0131 | SEQ ID NO: 131 | GGAGAGCACAGACACGGAGCG | SEQ ID NO: 1073 | CUCCGUGUCUGUGCUCUCCUG | SEQ ID NO: 2015 | GGAGAGCACAGACACGGAG | 0.4257 | |
| MA0132 | SEQ ID NO: 132 | GAGAGCACAGACACGGAGCGG | SEQ ID NO: 1074 | GCUCCGUGUCUGUGCUCUCCU | SEQ ID NO: 2016 | GAGAGCACAGACACGGAGC | 0.4422 | |

**[Table 1-4]**

| double stranded nucleic acid number | SEQ ID NO: | sense strand sequence (5'--->3') | SEQ ID NO: | antisense strand sequence (5' --->3') | SEQ ID NO: | target MASP2 mRNA sequence | relative expression level | note |
|---|---|---|---|---|---|---|---|---|
| MA0133 | SEQ ID NO: 133 | AGAGCACAGACACGGAGCGGG | SEQ ID NO: 1075 | CGCUCCGUGUCUGUGCUCUCC | SEQ ID NO: 2017 | AGAGCACAGACACGGAGCG | 0.3852 | |
| MA0134 | SEQ ID NO: 134 | CGGGCCCCUGGCAAGGACACU | SEQ ID NO: 1076 | UGUCCUUGCCAGGGGCCCGCU | SEQ ID NO: 2018 | CGGGCCCCUGGCAAGGACA | 0.4629 | * |
| MA0135 | SEQ ID NO: 135 | GCCCCUGGCAAGGACACUUUC | SEQ ID NO: 1077 | AAGUGUCCUUGCCAGGGGCCC | SEQ ID NO: 2019 | GCCCCUGGCAAGGACACUU | 0.2544 | |
| MA0136 | SEQ ID NO: 136 | CUGGCAAGGACACUUUCUACU | SEQ ID NO: 1078 | UAGAAAGUGUCCUUGCCAGGG | SEQ ID NO: 2020 | CUGGCAAGGACACUUUCUA | 0.2309 | |
| MA0137 | SEQ ID NO: 137 | GGCAAGGACACUUUCUACUCG | SEQ ID NO: 1079 | AGUAGAAAGUGUCCUUGCCAG | SEQ ID NO: 2021 | GGCAAGGACACUUUCUACU | 0.4818 | * |
| MA0138 | SEQ ID NO: 138 | GCAAGGACACUUUCUACUCGC | SEQ ID NO: 1080 | GAGUAGAAAGUGUCCUUGCCA | SEQ ID NO: 2022 | GCAAGGACACUUUCUACUC | 0.2564 | |
| MA0139 | SEQ ID NO: 139 | AGGACACUUUCUACUCGCUGG | SEQ ID NO: 1081 | AGCGAGUAGAAAGUGUCCUUG | SEQ ID NO: 2023 | AGGACACUUUCUACUCGCU | 0.4851 | * |
| MA0140 | SEQ ID NO: 140 | GGACACUUUCUACUCGCUGGG | SEQ ID NO: 1082 | CAGCGAGUAGAAAGUGUCCUU | SEQ ID NO: 2024 | GGACACUUUCUACUCGCUG | 0.3642 | |
| MA0141 | SEQ ID NO: 141 | GACACUUUCUACUCGCUGGGC | SEQ ID NO: 1083 | CCAGCGAGUAGAAAGUGUCCU | SEQ ID NO: 2025 | GACACUUUCUACUCGCUGG | 0.2720 | |
| MA0142 | SEQ ID NO: 142 | ACACUUUCUACUCGCUGGGCU | SEQ ID NO: 1084 | CCCAGCGAGUAGAAAGUGUCC | SEQ ID NO: 2026 | ACACUUUCUACUCGCUGGG | 0.5051 | * |
| MA0143 | SEQ ID NO: 143 | ACUUUCUACUCGCUGGGCUCC | SEQ ID NO: 1085 | AGCCCAGCGAGUAGAAAGUGU | SEQ ID NO: 2027 | ACUUUCUACUCGCUGGGCU | 0.1889 | |
| MA0144 | SEQ ID NO: 144 | CUUUCUACUCGCUGGGCUCCA | SEQ ID NO: 1086 | GAGCCCAGCGAGUAGAAAGUG | SEQ ID NO: 2028 | CUUUCUACUCGCUGGGCUC | 0.4350 | |
| MA0145 | SEQ ID NO: 145 | CUCGCUGGGCUCCAGCCUGGA | SEQ ID NO: 1087 | CAGGCUGGAGCCCAGCGAGUA | SEQ ID NO: 2029 | CUCGCUGGGCUCCAGCCUG | 0.3224 | |
| MA0146 | SEQ ID NO: 146 | CGCUGGGCUCCAGCCUGGACA | SEQ ID NO: 1088 | UCCAGGCUGGAGCCCAGCGAG | SEQ ID NO: 2030 | CGCUGGGCUCCAGCCUGGA | 0.4621 | * |
| MA0147 | SEQ ID NO: 147 | CUGGGCUCCAGCCUGGACAUU | SEQ ID NO: 1089 | UGUCCAGGCUGGAGCCCAGCG | SEQ ID NO: 2031 | CUGGGCUCCAGCCUGGACA | 0.2722 | |
| MA0148 | SEQ ID NO: 148 | UGGGCUCCAGCCUGGACAUUA | SEQ ID NO: 1090 | AUGUCCAGGCUGGAGCCCAGC | SEQ ID NO: 2032 | UGGGCUCCAGCCUGGACAU | 0.3401 | |
| MA0149 | SEQ ID NO: 149 | GGGCUCCAGCCUGGACAUUAC | SEQ ID NO: 1091 | AAUGUCCAGGCUGGAGCCCAG | SEQ ID NO: 2033 | GGGCUCCAGCCUGGACAUU | 0.3403 | |
| MA0150 | SEQ ID NO: 150 | GGCUCCAGCCUGGACAUUACC | SEQ ID NO: 1092 | UAAUGUCCAGGCUGGAGCCCA | SEQ ID NO: 2034 | GGCUCCAGCCUGGACAUUA | 0.2812 | |
| MA0151 | SEQ ID NO: 151 | GCUCCAGCCUGGACAUUACCU | SEQ ID NO: 1093 | GUAAUGUCCAGGCUGGAGCCC | SEQ ID NO: 2035 | GCUCCAGCCUGGACAUUAC | 0.2949 | |
| MA0152 | SEQ ID NO: 152 | CUCCAGCCUGGACAUUACCUU | SEQ ID NO: 1094 | GGUAAUGUCCAGGCUGGAGCC | SEQ ID NO: 2036 | CUCCAGCCUGGACAUUACC | 0.3877 | |
| MA0153 | SEQ ID NO: 153 | CCAGCCUGGACAUUACCUUCC | SEQ ID NO: 1095 | AAGGUAAUGUCCAGGCUGGAG | SEQ ID NO: 2037 | CCAGCCUGGACAUUACCUU | 0.2816 | |
| MA0154 | SEQ ID NO: 154 | CAGCCUGGACAUUACCUUCCG | SEQ ID NO: 1096 | GAAGGUAAUGUCCAGGCUGGA | SEQ ID NO: 2038 | CAGCCUGGACAUUACCUUC | 0.4591 | |
| MA0155 | SEQ ID NO: 155 | GCCUGGACAUUACCUUCCGCU | SEQ ID NO: 1097 | CGGAAGGUAAUGUCCAGGCUG | SEQ ID NO: 2039 | GCCUGGACAUUACCUUCCG | 0.4473 | |
| MA0156 | SEQ ID NO: 156 | CCUGGACAUUACCUUCCGCUC | SEQ ID NO: 1098 | GCGGAAGGUAAUGUCCAGGCU | SEQ ID NO: 2040 | CCUGGACAUUACCUUCCGC | 0.4523 | * |
| MA0157 | SEQ ID NO: 157 | CUGGACAUUACCUUCCGCUCC | SEQ ID NO: 1099 | AGCGGAAGGUAAUGUCCAGGC | SEQ ID NO: 2041 | CUGGACAUUACCUUCCGCU | 0.4886 | |
| MA0158 | SEQ ID NO: 158 | GGACAUUACCUUCCGCUCCGA | SEQ ID NO: 1100 | GGAGCGGAAGGUAAUGUCCAG | SEQ ID NO: 2042 | GGACAUUACCUUCCGCUCC | 0.4470 | |
| MA0159 | SEQ ID NO: 159 | GACAUUACCUUCCGCUCCGAC | SEQ ID NO: 1101 | CGGAGCGGAAGGUAAUGUCCA | SEQ ID NO: 2043 | GACAUUACCUUCCGCUCCG | 0.3543 | |
| MA0160 | SEQ ID NO: 160 | ACAUUACCUUCCGCUCCGACU | SEQ ID NO: 1102 | UCGGAGCGGAAGGUAAUGUCC | SEQ ID NO: 2044 | ACAUUACCUUCCGCUCCGA | 0.4627 | * |
| MA0161 | SEQ ID NO: 161 | CAUUACCUUCCGCUCCGACUA | SEQ ID NO: 1103 | GUCGGAGCGGAAGGUAAUGUC | SEQ ID NO: 2045 | CAUUACCUUCCGCUCCGAC | 0.4652 | |
| MA0162 | SEQ ID NO: 162 | UUACCUUCCGCUCCGACUACU | SEQ ID NO: 1104 | UAGUCGGAGCGGAAGGUAAUG | SEQ ID NO: 2046 | UUACCUUCCGCUCCGACUA | 0.4862 | |
| MA0163 | SEQ ID NO: 163 | ACCUUCCGCUCCGACUACUCC | SEQ ID NO: 1105 | AGUAGUCGGAGCGGAAGGUAA | SEQ ID NO: 2047 | ACCUUCCGCUCCGACUACU | 0.5657 | * |
| MA0164 | SEQ ID NO: 164 | CCUUCCGCUCCGACUACUCCA | SEQ ID NO: 1106 | GAGUAGUCGGAGCGGAAGGUA | SEQ ID NO: 2048 | CCUUCCGCUCCGACUACUC | 0.4293 | |
| MA0165 | SEQ ID NO: 165 | CUUCCGCUCCGACUACUCCAA | SEQ ID NO: 1107 | GGAGUAGUCGGAGCGGAAGGU | SEQ ID NO: 2049 | CUUCCGCUCCGACUACUCC | 0.4224 | |
| MA0166 | SEQ ID NO: 166 | UCCGACUACUCCAACGAGAAG | SEQ ID NO: 1108 | UCUCGUUGGAGUAGUCGGAGC | SEQ ID NO: 2050 | UCCGACUACUCCAACGAGA | 0.5686 | * |
| MA0167 | SEQ ID NO: 167 | CCGACUACUCCAACGAGAAGC | SEQ ID NO: 1109 | UUCUCGUUGGAGUAGUCGGAG | SEQ ID NO: 2051 | CCGACUACUCCAACGAGAA | 0.3156 | |
| MA0168 | SEQ ID NO: 168 | CGACUACUCCAACGAGAAGCC | SEQ ID NO: 1110 | CUUCUCGUUGGAGUAGUCGGA | SEQ ID NO: 2052 | CGACUACUCCAACGAGAAG | 0.2102 | |
| MA0169 | SEQ ID NO: 169 | ACUCCAACGAGAAGCCGUUCA | SEQ ID NO: 1111 | AACGGCUUCUCGUUGGAGUAG | SEQ ID NO: 2053 | ACUCCAACGAGAAGCCGUU | 0.3899 | |
| MA0170 | SEQ ID NO: 170 | CUCCAACGAGAAGCCGUUCAC | SEQ ID NO: 1112 | GAACGGCUUCUCGUUGGAGUA | SEQ ID NO: 2054 | CUCCAACGAGAAGCCGUUC | 0.4075 | |
| MA0171 | SEQ ID NO: 171 | ACGAGAAGCCGUUCACGGGGU | SEQ ID NO: 1113 | CCCGUGAACGGCUUCUCGUUG | SEQ ID NO: 2055 | ACGAGAAGCCGUUCACGGG | 0.5797 | * |
| MA0172 | SEQ ID NO: 172 | CGAGAAGCCGUUCACGGGGUU | SEQ ID NO: 1114 | CCCCGUGAACGGCUUCUCGUU | SEQ ID NO: 2056 | CGAGAAGCCGUUCACGGGG | 0.5345 | * |
| MA0173 | SEQ ID NO: 173 | GAGAAGCCGUUCACGGGGUUC | SEQ ID NO: 1115 | ACCCCGUGAACGGCUUCUCGU | SEQ ID NO: 2057 | GAGAAGCCGUUCACGGGGU | 0.3813 | |
| MA0174 | SEQ ID NO: 174 | GAAGCCGUUCACGGGGUUCGA | SEQ ID NO: 1116 | GAACCCCGUGAACGGCUUCUC | SEQ ID NO : 2058 | GAAGCCGUUCACGGGGUUC | 0.5874 | * |
| MA0175 | SEQ ID NO: 175 | AGCCGUUCACGGGGUUCGAGG | SEQ ID NO: 1117 | UCGAACCCCGUGAACGGCUUC | SEQ ID NO: 2059 | AGCCGUUCACGGGGUUCGA | 0.3347 | |
| MA0176 | SEQ ID NO: 176 | CCGUUCACGGGGUUCGAGGCC | SEQ ID NO: 1118 | CCUCGAACCCCGUGAACGGCU | SEQ ID NO: 2060 | CCGUUCACGGGGUUCGAGG | 0.3233 | |

**[Table 1-5]**

| double stranded nucleic acid number | SEQ ID NO: | sense strand sequence (5'--->3') | SEQ ID NO: | antisense strand sequence (5'--->3') | SEQ ID NO: | target MASP2 mRNA sequence | relative expression level | note |
|---|---|---|---|---|---|---|---|---|
| MA0177 | SEQ ID NO: 177 | CGUUCACGGGGUUCGAGGCCU | SEQ ID NO: 1119 | GCCUCGAACCCCGUGAACGGC | SEQ ID NO: 2061 | CGUUCACGGGGUUCGAGGC | 0.4308 | |
| MA0178 | SEQ ID NO: 178 | UCACGGGGUUCGAGGCCUUCU | SEQ ID NO: 1120 | AAGGCCUCGAACCCCGUGAAC | SEQ ID NO: 2062 | UCACGGGGUUCGAGGCCUU | 0.2955 | |
| MA0179 | SEQ ID NO: 179 | GGGGUUCGAGGCCUUCUAUGC | SEQ ID NO: 1121 | AUAGAAGGCCUCGAACCCCGU | SEQ ID NO: 2063 | GGGGUUCGAGGCCUUCUAU | 0.4207 | |
| MA0180 | SEQ ID NO: 180 | GGGUUCGAGGCCUUCUAUGCA | SEQ ID NO: 1122 | CAUAGAAGGCCUCGAACCCCG | SEQ ID NO: 2064 | GGGUUCGAGGCCUUCUAUG | 0.4314 | |
| MA0181 | NO: 181 | GGUUCGAGGCCUUCUAUGCAG | SEQ ID NO: 1123 | GCAUAGAAGGCCUCGAACCCC | SEQ ID NO: 2065 | GGUUCGAGGCCUUCUAUGC | 0.4212 | |
| MA0182 | SEQ ID NO: 182 | GUUCGAGGCCUUCUAUGCAGC | SEQ ID NO: 1124 | UGCAUAGAAGGCCUCGAACCC | SEQ ID NO: 2066 | GUUCGAGGCCUUCUAUGCA | 0.3042 | |
| MA0183 | SEQ ID NO: 183 | GGCCUUCUAUGCAGCCGAGGA | SEQ ID NO: 1125 | CUCGGCUGCAUAGAAGGCCUC | SEQ ID NO: 2067 | GGCCUUCUAUGCAGCCGAG | 0.4830 | * |
| MA0184 | SEQ ID NO: 184 | UCUAUGCAGCCGAGGACAUUG | SEQ ID NO: 1126 | AUGUCCUCGGCUGCAUAGAAG | SEQ ID NO: 2068 | UCUAUGCAGCCGAGGACAU | 0.4556 | |
| MA0185 | SEQ ID NO: 185 | CUAUGCAGCCGAGGACAUUGA | SEQ ID NO: 1127 | AAUGUCCUCGGCUGCAUAGAA | SEQ ID NO: 2069 | CUAUGCAGCCGAGGACAUU | 0.5866 | |
| MA0186 | SEQ ID NO: 186 | UAUGCAGCCGAGGACAUUGAC | SEQ ID NO: 1128 | CAAUGUCCUCGGCUGCAUAGA | SEQ ID NO: 2070 | UAUGCAGCCGAGGACAUUG | 0.4871 | |
| MA0187 | SEQ ID NO: 187 | AUGCAGCCGAGGACAUUGACG | SEQ ID NO: 1129 | UCAAUGUCCUCGGCUGCAUAG | SEQ ID NO: 2071 | AUGCAGCCGAGGACAUUGA | 0.4939 | |
| MA0188 | SEQ ID NO: 188 | GCAGCCGAGGACAUUGACGAG | SEQ 10 NO: 1130 | CGUCAAUGUCCUCGGCUGCAU | SEQ ID NO: 2072 | GCAGCCGAGGACAUUGACG | 0.4111 | |
| MA0189 | SEQ ID NO: 189 | CAGCCGAGGACAUUGACGAGU | SEQ ID NO: 1131 | UCGUCAAUGUCCUCGGCUGCA | SEQ ID NO: 2073 | CAGCCGAGGACAUUGACGA | 0.3772 | |
| MA0190 | SEQ ID NO: 190 | AGCCGAGGACAUUGACGAGUG | SEQ ID NO: 1132 | CUCGUCAAUGUCCUCGGCUGC | SEQ ID NO: 2074 | AGCCGAGGACAUUGACGAG | 0.5403 | * |
| MA0191 | SEQ ID NO: 191 | GCCGAGGACAUUGACGAGUGC | SEQ ID NO: 1133 | ACUCGUCAAUGUCCUCGGCUG | SEQ ID NO: 2075 | GCCGAGGACAUUGACGAGU | 0.5877 | * |
| MA0192 | SEQ ID NO: 192 | CCGAGGACAUUGACGAGUGCC | SEQ ID NO: 1134 | CACUCGUCAAUGUCCUCGGCU | SEQ ID NO: 2076 | CCGAGGACAUUGACGAGUG | 0.5236 | * |
| MA0193 | SEQ ID NO: 193 | CGAGGACAUUGACGAGUGCCA | SEQ ID NO: 1135 | GCACUCGUCAAUGUCCUCGGC | SEQ ID NO: 2077 | CGAGGACAUUGACGAGUGC | 0.5510 | |
| MA0194 | SEQ ID NO: 194 | AGUGCCAGGUGGCCCCGGGAG | SEQ ID NO: 1136 | CCCGGGGCCACCUGGCACUCG | SEQ ID NO: 2078 | AGUGCCAGGUGGCCCCGGG | 0.4975 | |
| MA0195 | SEQ ID NO: 195 | GGAGAGGCGCCCACCUGCGAC | SEQ ID NO: 1137 | CGCAGGUGGGCGCCUCUCCCG | SEQ ID NO: 2079 | GGAGAGGCGCCCACCUGCG | 0.5445 | * |
| MA0196 | SEQ ID NO: 196 | GAGAGGCGCCCACCUGCGACC | SEQ ID NO: 1138 | UCGCAGGUGGGCGCCUCUCCC | SEQ ID NO: 2080 | GAGAGGCGCCCACCUGCGA | 0.5389 | * |
| MA0197 | SEQ ID NO: 197 | CCCACCUGCGACCACCACUGC | SEQ ID NO: 1139 | AGUGGUGGUCGCAGGUGGGCG | SEQ ID NO: 2081 | CCCACCUGCGACCACCACU | 0.5541 | * |
| MA0198 | SEQ ID NO: 198 | GCGACCACCACUGCCACAACC | SEQ ID NO: 1140 | UUGUGGCAGUGGUGGUCGCAG | SEQ ID NO: 2082 | GCGACCACCACUGCCACAA | 0.4922 | * |
| MA0199 | SEQ ID NO: 199 | CGACCACCACUGCCACAACCA | SEQ ID NO: 1141 | GUUGUGGCAGUGGUGGUCGCA | SEQ ID NO: 2083 | CGACCACCACUGCCACAAC | 0.4492 | |
| MA0200 | SEQ ID NO: 200 | ACCACCACUGCCACAACCACC | SEQ ID NO: 1142 | UGGUUGUGGCAGUGGUGGUCG | SEQ ID NO: 2084 | ACCACCACUGCCACAACCA | 0.4760 | |
| MA0201 | SEQ ID NO: 201 | CCACCACUGCCACAACCACCU | SEQ ID NO: 1143 | GUGGUUGUGGCAGUGGUGGUC | SEQ ID NO: 2085 | CCACCACUGCCACAACCAC | 0.4698 | * |
| MA0202 | SEQ ID NO: 202 | ACCACUGCCACAACCACCUGG | SEQ ID NO: 1144 | AGGUGGUUGUGGCAGUGGUGG | SEQ ID NO: 2086 | ACCACUGCCACAACCACCU | 0.5277 | * |
| MA0203 | SEQ ID NO: 203 | CCACUGCCACAACCACCUGGG | SEQ ID NO: 1145 | CAGGUGGUUGUGGCAGUGGUG | SEQ ID NO: 2087 | CCACUGCCACAACCACCUG | 0.4527 | |
| MA0204 | SEQ ID NO: 204 | ACUGCCACAACCACCUGGGCG | SEQ ID NO: 1146 | CCCAGGUGGUUGUGGCAGUGG | SEQ ID NO: 2088 | ACUGCCACAACCACCUGGG | 0.5410 | * |
| MA0205 | SEQ ID NO: 205 | GCCACAACCACCUGGGCGGUU | SEQ ID NO: 1147 | CCGCCCAGGUGGUUGUGGCAG | SEQ ID NO: 2089 | GCCACAACCACCUGGGCGG | 0.5442 | * |
| MA0206 | SEQ ID NO: 206 | CACAACCACCUGGGCGGUUUC | SEQ ID NO: 1148 | AACCGCCCAGGUGGUUGUGGC | SEQ ID NO: 2090 | CACAACCACCUGGGCGGUU | 0.5043 | |
| MA0207 | SEQ ID NO: 207 | ACAACCACCUGGGCGGUUUCU | SEQ ID NO: 1149 | AAACCGCCCAGGUGGUUGUGG | SEQ ID NO: 2091 | ACAACCACCUGGGCGGUUU | 0.3048 | |
| MA0208 | SEQ ID NO: 208 | CAACCACCUGGGCGGUUUCUA | SEQ ID NO: 1150 | GAAACCGCCCAGGUGGUUGUG | SEQ ID NO: 2092 | CAACCACCUGGGCGGUUUC | 0.2445 | |
| MA0209 | SEQ ID NO: 209 | CCACCUGGGCGGUUUCUACUG | SEQ ID NO: 1151 | GUAGAAACCGCCCAGGUGGUU | SEQ ID NO: 2093 | CCACCUGGGCGGUUUCUAC | 0.5105 | * |
| MA0210 | SEQ ID NO: 210 | GGGCGGUUUCUACUGCUCCUG | SEQ ID NO: 1152 | GGAGCAGUAGAAACCGCCCAG | SEQ ID NO: 2094 | GGGCGGUUUCUACUGCUCC | 0.4838 | |
| MA0211 | SEQ ID NO: 211 | GGCGGUUUCUACUGCUCCUGC | SEQ ID NO: 1153 | AGGAGCAGUAGAAACCGCCCA | SEQ ID NO: 2095 | GGCGGUUUCUACUGCUCCU | 0.2747 | |
| MA0212 | SEQ ID NO: 212 | GCGGUUUCUACUGCUCCUGCC | SEQ ID NO: 1154 | CAGGAGCAGUAGAAACCGCCC | SEQ ID NO: 2096 | GCGGUUUCUACUGCUCCUG | 0.2057 | |
| MA0213 | SEQ ID NO: 213 | CGGUUUCUACUGCUCCUGCCG | SEQ ID NO: 1155 | GCAGGAGCAGUAGAAACCGCC | SEQ ID NO: 2097 | CGGUUUCUACUGCUCCUGC | 0.5748 | |
| MA0214 | SEQ ID NO: 214 | GUUUCUACUGCUCCUGCCGCG | SEQ ID NO: 1156 | CGGCAGGAGCAGUAGAAACCG | SEQ ID NO: 2098 | GUUUCUACUGCUCCUGCCG | 0.3691 | |
| MA0215 | SEQ ID NO: 215 | GCUCCUGCCGCGCAGGCUACG | SEQ ID NO: 1157 | UAGCCUGCGCGGCAGGAGCAG | SEQ ID NO: 2099 | GCUCCUGCCGCGCAGGCUA | 0.5681 | * |
| MA0216 | SEQ ID NO: 216 | CUGCCGCGCAGGCUACGUCCU | SEQ ID NO: 1158 | GACGUAGCCUGCGCGGCAGGA | SEQ ID NO: 2100 | CUGCCGCGCAGGCUACGUC | 0.4764 | * |
| MA0217 | SEQ ID NO: 217 | GGCUACGUCCUGCACCGUAAC | SEQ ID NO: 1159 | UACGGUGCAGGACGUAGCCUG | SEQ ID NO: 2101 | GGCUACGUCCUGCACCGUA | 0.1601 | |
| MA0218 | SEQ ID NO: 218 | GCUACGUCCUGCACCGUAACA | SEQ ID NO: 1160 | UUACGGUGCAGGACGUAGCCU | SEQ ID NO: 2102 | GCUACGUCCUGCACCGUAA | 0.3361 | |
| MA0219 | SEQ ID NO: 219 | CUACGUCCUGCACCGUAACAA | SEQ ID NO: 1161 | GUUACGGUGCAGGACGUAGCC | SEQ ID NO: 2103 | CUACGUCCUGCACCGUAAC | 0.3681 | |
| MA0220 | SEQ ID NO: 220 | ACGUCCUGCACCGUAACAAGC | SEQ ID NO: 1162 | UUGUUACGGUGCAGGACGUAG | SEQ ID NO: 2104 | ACGUCCUGCACCGUAACAA | 0.3200 | |

**[Table 1-6]**

| double stranded nucleic acid number | SEQ ID NO: | sense strand sequence (5' --->3') | SEQ ID NO: | antisense strand sequence (5' --->3') | SEQ ID NO: | target MASP2 mRNA sequence | relative expression level | note |
|---|---|---|---|---|---|---|---|---|
| MA0221 | SEQ ID NO: 221 | CGUCCUGCACCGUAACAAGCG | SEQ ID NO: 1163 | CUUGUUACGGUGCAGGACGUA | SEQ ID NO: 2105 | CGUCCUGCACCGUAACAAG | 0.3384 | |
| MA0222 | SEQ ID NO: 222 | CUGCACCGUAACAAGCGCACC | SEQ ID NO: 1164 | UGCGCUUGUUACGGUGCAGGA | SEQ ID NO: 2106 | CUGCACCGUAACAAGCGCA | 0.4278 | |
| MA0223 | SEQ ID NO: 223 | GCACCGUAACAAGCGCACCUG | SEQ ID NO: 1165 | GGUGCGCUUGUUACGGUGCAG | SEQ ID NO: 2107 | GCACCGUAACAAGCGCACC | 0.3737 | |
| MA0224 | SEQ ID NO: 224 | CACCGUAACAAGCGCACCUGC | SEQ ID NO: 1166 | AGGUGCGCUUGUUACGGUGCA | SEQ ID NO: 2108 | CACCGUAACAAGCGCACCU | 0.5596 | |
| MA0225 | SEQ ID NO: 225 | CCGUAACAAGCGCACCUGCUC | SEQ ID NO: 1167 | GCAGGUGCGCUUGUUACGGUG | SEQ ID NO: 2109 | CCGUAACAAGCGCACCUGC | 0.5756 | |
| MA0226 | SEQ ID NO: 226 | CGUAACAAGCGCACCUGCUCA | SEQ ID NO: 1168 | AGCAGGUGCGCUUGUUACGGU | SEQ ID NO: 2110 | CGUAACAAGCGCACCUGCU | 0.4483 | |
| MA0227 | SEQ ID NO: 227 | CAAGCGCACCUGCUCAGCCCU | SEQ ID NO: 1169 | GGCUGAGCAGGUGCGCUUGUU | SEQ ID NO: 2111 | CAAGCGCACCUGCUCAGCC | 0.5864 | * |
| MA0228 | SEQ ID NO: 228 | GCCAGGUCUUCACCCAGAGGU | SEQ ID NO: 1170 | CUCUGGGUGAAGACCUGGCCG | SEQ ID NO: 2112 | GCCAGGUCUUCACCCAGAG | 0.4282 | |
| MA0229 | SEQ ID NO: 229 | AGGUCUUCACCCAGAGGUCUG | SEQ ID NO: 1171 | GACCUCUGGGUGAAGACCUGG | SEQ ID NO: 2113 | AGGUCUUCACCCAGAGGUC | 0.4223 | |
| MA0230 | SEQ ID NO: 230 | ACCCAGAGGUCUGGGGAGCUC | SEQ ID NO: 1172 | GCUCCCCAGACCUCUGGGUGA | SEQ ID NO: 2114 | ACCCAGAGGUCUGGGGAGC | 0.5581 | * |
| MA0231 | SEQ ID NO: 231 | CCCAGAGGUCUGGGGAGCUCA | SEQ ID NO: 1173 | AGCUCCCCAGACCUCUGGGUG | SEQ ID NO: 2115 | CCCAGAGGUCUGGGGAGCU | 0.4251 | |
| MA0232 | SEQ ID NO: 232 | CCAGAGGUCUGGGGAGCUCAG | SEQ ID NO: 1174 | GAGCUCCCCAGACCUCUGGGU | SEQ ID NO: 2116 | CCAGAGGUCUGGGGAGCUC | 0.4255 | |
| MA0233 | SEQ ID NO: 233 | CAGAGGUCUGGGGAGCUCAGC | SEQ ID NO: 1175 | UGAGCUCCCCAGACCUCUGGG | SEQ ID NO: 2117 | CAGAGGUCUGGGGAGCUCA | 0.3198 | |
| MA0234 | SEQ ID NO: 234 | AGAGGUCUGGGGAGCUCAGCA | SEQ ID NO: 1176 | CUGAGCUCCCCAGACCUCUGG | SEQ ID NO: 2118 | AGAGGUCUGGGGAGCUCAG | 0.5290 | * |
| MA0235 | SEQ ID NO: 235 | GGUCUGGGGAGCUCAGCAGCC | SEQ ID NO: 1177 | CUGCUGAGCUCCCCAGACCUC | SEQ ID NO: 2119 | GGUCUGGGGAGCUCAGCAG | 0.5204 | * |
| MA0236 | SEQ ID NO: 236 | UGGGGAGCUCAGCAGCCCUGA | SEQ ID NO: 1178 | AGGGCUGCUGAGCUCCCCAGA | SEQ ID NO: 2120 | UGGGGAGCUCAGCAGCCCU | 0.4872 | |
| MA0237 | SEQ ID NO: 237 | GGGGAGCUCAGCAGCCCUGAA | SEQ ID NO: 1179 | CAGGGCUGCUGAGCUCCCCAG | SEQ ID NO: 2121 | GGGGAGCUCAGCAGCCCUG | 0.4882 | * |
| MA0238 | SEQ ID NO: 238 | GGAGCUCAGCAGCCCUGAAUA | SEQ ID NO: 1180 | UUCAGGGCUGCUGAGCUCCCC | SEQ ID NO: 2122 | GGAGCUCAGCAGCCCUGAA | 0.4859 | * |
| MA0239 | SEQ ID NO: 239 | GAGCUCAGCAGCCCUGAAUAC | SEQ ID NO: 1181 | AUUCAGGGCUGCUGAGCUCCC | SEQ ID NO: 2123 | GAGCUCAGCAGCCCUGAAU | 0.3060 | |
| MA0240 | SEQ ID NO: 240 | GCUCAGCAGCCCUGAAUACCC | SEQ ID NO: 1182 | GUAUUCAGGGCUGCUGAGCUC | SEQ ID NO: 2124 | GCUCAGCAGCCCUGAAUAC | 0.3323 | |
| MA0241 | SEQ ID NO: 241 | CAGCAGCCCUGAAUACCCACG | SEQ ID NO: 1183 | UGGGUAUUCAGGGCUGCUGAG | SEQ ID NO: 2125 | CAGCAGCCCUGAAUACCCA | 0.3482 | |
| MA0242 | SEQ ID NO: 242 | AGCAGCCCUGAAUACCCACGG | SEQ ID NO: 1184 | GUGGGUAUUCAGGGCUGCUGA | SEQ ID NO: 2126 | AGCAGCCCUGAAUACCCAC | 0.4790 | * |
| MA0243 | SEQ ID NO: 24 3 | GCAGCCCUGAAUACCCACGGC | SEQ ID NO: 1185 | CGUGGGUAUUCAGGGCUGCUG | SEQ ID NO: 2127 | GCAGCCCUGAAUACCCACG | 0.4401 | |
| MA0244 | SEQ ID NO: 244 | CUGAAUACCCACGGCCGUAUC | SEQ ID NO: 1186 | UACGGCCGUGGGUAUUCAGGG | SEQ ID NO: 2128 | CUGAAUACCCACGGCCGUA | 0.4632 | * |
| MA0245 | SEQ ID NO: 245 | CCCACGGCCGUAUCCCAAACU | SEQ ID NO: 1187 | UUUGGGAUACGGCCGUGGGUA | SEQ ID NO: 2129 | CCCACGGCCGUAUCCCAAA | 0.3487 | |
| MA0246 | SEQ ID NO: 246 | CCACGGCCGUAUCCCAAACUC | SEQ ID NO: 1188 | GUUUGGGAUACGGCCGUGGGU | SEQ ID NO: 2130 | CCACGGCCGUAUCCCAAAC | 0.5145 | * |
| MA0247 | SEQ ID NO: 247 | GGCCGUAUCCCAAACUCUCCA | SEQ ID NO: 1189 | GAGAGUUUGGGAUACGGCCGU | SEQ ID NO: 2131 | GGCCGUAUCCCAAACUCUC | 0.3928 | |
| MA0248 | SEQ ID NO: 248 | GCCGUAUCCCAAACUCUCCAG | SEQ ID NO: 1190 | GGAGAGUUUGGGAUACGGCCG | SEQ ID NO: 2132 | GCCGUAUCCCAAACUCUCC | 0.2896 | |
| MA0249 | SEQ ID NO: 249 | CGUAUCCCAAACUCUCCAGUU | SEQ ID NO: 1191 | CUGGAGAGUUUGGGAUACGGC | SEQ ID NO: 2133 | CGUAUCCCAAACUCUCCAG | 0.5478 | * |
| MA0250 | SEQ ID NO: 250 | GUAUCCCAAACUCUCCAGUUG | SEQ ID NO: 1192 | ACUGGAGAGUUUGGGAUACGG | SEQ ID NO: 2134 | GUAUCCCAAACUCUCCAGU | 0.3549 | |
| MA0251 | SEQ ID NO: 251 | UAUCCCAAACUCUCCAGUUGC | SEQ ID NO: 1193 | AACUGGAGAGUUUGGGAUACG | SEQ ID NO: 2135 | UAUCCCAAACUCUCCAGUU | 0.5827 | * |
| MA0252 | SEQ ID NO: 252 | CCCAAACUCUCCAGUUGCACU | SEQ ID NO: 1194 | UGCAACUGGAGAGUUUGGGAU | SEQ ID NO: 2136 | CCCAAACUCUCCAGUUGCA | 0.4890 | |
| MA0253 | SEQ ID NO: 253 | CAAACUCUCCAGUUGCACUUA | SEQ ID NO: 1195 | AGUGCAACUGGAGAGUUUGGG | SEQ ID NO: 2137 | CAAACUCUCCAGUUGCACU | 0.2475 | |
| MA0254 | SEQ ID NO: 254 | AAACUCUCCAGUUGCACUUAC | SEQ ID NO: 1196 | AAGUGCAACUGGAGAGUUUGG | SEQ ID NO: 2138 | AAACUCUCCAGUUGCACUU | 0.4343 | |
| MA0255 | SEQ ID NO: 255 | AACUCUCCAGUUGCACUUACA | SEQ ID NO: 1197 | UAAGUGCAACUGGAGAGUUUG | SEQ ID NO: 2139 | AACUCUCCAGUUGCACUUA | 0.4009 | |
| MA0256 | SEQ ID NO: 256 | ACUCUCCAGUUGCACUUACAG | SEQ ID NO: 1198 | GUAAGUGCAACUGGAGAGUUU | SEQ ID NO: 2140 | ACUCUCCAGUUGCACUUAC | 0.4885 | |
| MA0257 | SEQ ID NO: 257 | CUCUCCAGUUGCACUUACAGC | SEQ ID NO: 1199 | UGUAAGUGCAACUGGAGAGUU | SEQ ID NO: 2141 | CUCUCCAGUUGCACUUACA | 0.4848 | |
| MA0258 | SEQ ID NO: 258 | UCCAGUUGCACUUACAGCAUC | SEQ ID NO: 1200 | UGCUGUAAGUGCAACUGGAGA | SEQ ID NO: 2142 | UCCAGUUGCACUUACAGCA | 0.4608 | |
| MA0259 | SEQ ID NO: 259 | CCAGUUGCACUUACAGCAUCA | SEQ ID NO: 1201 | AUGCUGUAAGUGCAACUGGAG | SEQ ID NO: 2143 | CCAGUUGCACUUACAGCAU | 0.1664 | |
| MA0260 | SEQ ID NO: 260 | CAGUUGCACUUACAGCAUCAG | SEQ ID NO: 1202 | GAUGCUGUAAGUGCAACUGGA | SEQ ID NO: 2144 | CAGUUGCACUUACAGCAUC | 0.2499 | |
| MA0261 | SEQ ID NO: 261 | AGUUGCACUUACAGCAUCAGC | SEQ ID NO: 1203 | UGAUGCUGUAAGUGCAACUGG | SEQ ID NO: 2145 | AGUUGCACUUACAGCAUCA | 0.1704 | |
| MA0262 | SEQ ID NO: 262 | GUUGCACUUACAGCAUCAGCC | SEQ ID NO: 1204 | CUGAUGCUGUAAGUGCAACUG | SEQ ID NO: 2146 | GUUGCACUUACAGCAUCAG | 0.3034 | |
| MA0263 | SEQ ID NO: 263 | CACUUACAGCAUCAGCCUGGA | SEQ ID NO: 1205 | CAGGCUGAUGCUGUAAGUGCA | SEQ ID NO: 2147 | CACUUACAGCAUCAGCCUG | 0.4214 | |
| MA0264 | SEQ ID NO: 264 | CUUACAGCAUCAGCCUGGAGG | SEQ ID NO: 1206 | UCCAGGCUGAUGCUGUAAGUG | SEQ ID NO: 2148 | CUUACAGCAUCAGCCUGGA | 0.5230 | |

**[Table 1-7]**

| double stranded nucleic acid number | SEQ ID NO: | sense strand sequence (5'--->3') | SEQ ID NO: | antisense strand sequence (5'--->3') | SEQ ID NO: | target MASP2 mRNA sequence | relative expression level | note |
|---|---|---|---|---|---|---|---|---|
| MA0265 | SEQ ID NO: 265 | GCAUCAGCCUGGAGGAGGGGU | SEQ ID NO: 1207 | CCCUCCUCCAGGCUGAUGCUG | SEQ ID NO: 2149 | GCAUCAGCCUGGAGGAGGG | 0.5629 | * |
| MA0266 | SEQ ID NO: 266 | UCAGCCUGGAGGAGGGGUUCA | SEQ ID NO: 1208 | AACCCCUCCUCCAGGCUGAUG | SEQ ID NO: 2150 | UCAGCCUGGAGGAGGGGUU | 0.5551 | |
| MA0267 | SEQ ID NO: 267 | CCUGGAGGAGGGGUUCAGUGU | SEQ ID NO: 1209 | ACUGAACCCCUCCUCCAGGCU | SEQ ID NO: 2151 | CCUGGAGGAGGGGUUCAGU | 0.5903 | * |
| MA0268 | SEQ ID NO: 268 | CUGGAGGAGGGGUUCAGUGUC | SEQ ID NO: 1210 | CACUGAACCCCUCCUCCAGGC | SEQ ID NO: 2152 | CUGGAGGAGGGGUUCAGUG | 0.4717 | * |
| MA0269 | SEQ ID NO: 269 | UGGAGGAGGGGUUCAGUGUCA | SEQ ID NO: 1211 | ACACUGAACCCCUCCUCCAGG | SEQ ID NO: 2153 | UGGAGGAGGGGUUCAGUGU | 0.4069 | |
| MA0270 | SEQ ID NO: 270 | GGAGGAGGGGUUCAGUGUCAU | SEQ ID NO: 1212 | GACACUGAACCCCUCCUCCAG | SEQ ID NO: 2154 | GGAGGAGGGGUUCAGUGUC | 0.5705 | |
| MA0271 | SEQ ID NO: 271 | GAGGAGGGGUUCAGUGUCAUU | SEQ ID NO: 1213 | UGACACUGAACCCCUCCUCCA | SEQ ID NO: 2155 | GAGGAGGGGUUCAGUGUCA | 0.5201 | * |
| MA0272 | SEQ ID NO: 272 | AGGAGGGGUUCAGUGUCAUUC | SEQ ID NO: 1214 | AUGACACUGAACCCCUCCUCC | SEQ ID NO: 2156 | AGGAGGGGUUCAGUGUCAU | 0.4697 | |
| MA0273 | SEQ ID NO: 273 | GGAGGGGUUCAGUGUCAUUCU | SEQ ID NO: 1215 | AAUGACACUGAACCCCUCCUC | SEQ ID NO: 2157 | GGAGGGGUUCAGUGUCAUU | 0.3886 | |
| MA0274 | SEQ ID NO: 274 | GAGGGGUUCAGUGUCAUUCUG | SEQ ID NO: 1216 | GAAUGACACUGAACCCCUCCU | SEQ ID NO: 2158 | GAGGGGUUCAGUGUCAUUC | 0.3832 | |
| MA0275 | SEQ ID NO: 275 | GGGGUUCAGUGUCAUUCUGGA | SEQ ID NO: 1217 | CAGAAUGACACUGAACCCCUC | SEQ ID NO: 2159 | GGGGUUCAGUGUCAUUCUG | 0.2600 | |
| MA0276 | SEQ ID NO: 276 | GGUUCAGUGUCAUUCUGGACU | SEQ ID NO: 1218 | UCCAGAAUGACACUGAACCCC | SEQ ID NO: 2160 | GGUUCAGUGUCAUUCUGGA | 0.4765 | |
| MA0277 | SEQ ID NO: 277 | GUUCAGUGUCAUUCUGGACUU | SEQ ID NO: 1219 | GUCCAGAAUGACACUGAACCC | SEQ ID NO: 2161 | GUUCAGUGUCAUUCUGGAC | 0.4706 | |
| MA0278 | SEQ ID NO: 278 | UCAGUGUCAUUCUGGACUUUG | SEQ ID NO: 1220 | AAGUCCAGAAUGACACUGAAC | SEQ ID NO: 2162 | UCAGUGUCAUUCUGGACUU | 0.3636 | |
| MA0279 | SEQ ID NO: 279 | CAGUGUCAUUCUGGACUUUGU | SEQ ID NO: 1221 | AAAGUCCAGAAUGACACUGAA | SEQ ID NO: 2163 | CAGUGUCAUUCUGGACUUU | 0.4409 | |
| MA0280 | SEQ ID NO: 280 | AGUGUCAUUCUGGACUUUGUG | SEQ ID NO: 1222 | CAAAGUCCAGAAUGACACUGA | SEQ ID NO: 2164 | AGUGUCAUUCUGGACUUUG | 0.2410 | |
| MA0281 | SEQ ID NO: 281 | GUGUCAUUCUGGACUUUGUGG | SEQ ID NO: 1223 | ACAAAGUCCAGAAUGACACUG | SEQ ID NO: 2165 | GUGUCAUUCUGGACUUUGU | 0.5820 | |
| MA0282 | SEQ ID NO: 282 | GACUUUGUGGAGUCCUUCGAU | SEQ ID NO: 1224 | CGAAGGACUCCACAAAGUCCA | SEQ ID NO: 2166 | GACUUUGUGGAGUCCUUCG | 0.4390 | |
| MA0283 | SEQ ID NO: 283 | ACUUUGUGGAGUCCUUCGAUG | SEQ ID NO: 1225 | UCGAAGGACUCCACAAAGUCC | SEQ ID NO: 2167 | ACUUUGUGGAGUCCUUCGA | 0.4999 | |
| MA0284 | SEQ ID NO: 284 | UUGUGGAGUCCUUCGAUGUGG | SEQ ID NO: 1226 | ACAUCGAAGGACUCCACAAAG | SEQ ID NO: 2168 | UUGUGGAGUCCUUCGAUGU | 0.4765 | |
| MA0285 | SEQ ID NO: 285 | UGUGGAGUCCUUCGAUGUGGA | SEQ ID NO: 1227 | CACAUCGAAGGACUCCACAAA | SEQ ID NO: 2169 | UGUGGAGUCCUUCGAUGUG | 0.5529 | * |
| MA0286 | SEQ ID NO: 286 | UGGAGUCCUUCGAUGUGGAGA | SEQ ID NO: 1228 | UCCACAUCGAAGGACUCCACA | SEQ ID NO: 2170 | UGGAGUCCUUCGAUGUGGA | 0.4499 | |
| MA0287 | SEQ ID NO: 287 | GAGUCCUUCGAUGUGGAGACA | SEQ ID NO: 1229 | UCUCCACAUCGAAGGACUCCA | SEQ ID NO: 2171 | GAGUCCUUCGAUGUGGAGA | 0.4828 | |
| MA0288 | SEQ **ID** NO: 288 | AGUCCUUCGAUGUGGAGACAC | SEQ ID NO: 1230 | GUCUCCACAUCGAAGGACUCC | SEQ ID NO: 2172 | AGUCCUUCGAUGUGGAGAC | 0.4890 | * |
| MA0289 | SEQ ID NO: 289 | GUCCUUCGAUGUGGAGACACA | SEQ ID NO: 1231 | UGUCUCCACAUCGAAGGACUC | SEQ ID NO: 2173 | GUCCUUCGAUGUGGAGACA | 0.5328 | |
| MA0290 | SEQ ID NO: 290 | CCUUCGAUGUGGAGACACACC | SEQ ID NO: | UGUGUCUCCACAUCGAAGGAC | SEQ ID NO: 2174 | CCUUCGAUGUGGAGACACA | 0.2342 | |
| MA0291 | SEQ ID NO: 291 | CUUCGAUGUGGAGACACACCC | SEQ ID NO: 1233 | GUGUGUCUCCACAUCGAAGGA | SEQ ID NO: 2175 | CUUCGAUGUGGAGACACAC | 0.3618 | |
| MA0292 | SEQ ID NO: 292 | AUGUGGAGACACACCCUGAAA | SEQ ID NO: 1234 | UCAGGGUGUGUCUCCACAUCG | SEQ ID NO: 2176 | AUGUGGAGACACACCCUGA | 0.3168 | |
| MA0293 | SEQ ID NO: 293 | UGUGGAGACACACCCUGAAAC | SEQ ID NO: 1235 | UUCAGGGUGUGUCUCCACAUC | SEQ ID NO: 2177 | UGUGGAGACACACCCUGAA | 0.2669 | |
| MA0294 | SEQ ID NO: 294 | GUGGAGACACACCCUGAAACC | SEQ ID NO: 1236 | UUUCAGGGUGUGUCUCCACAU | SEQ ID NO: 2178 | GUGGAGACACACCCUGAAA | 0.1774 | |
| MA0295 | SEQ ID NO: 295 | GGAGACACACCCUGAAACCCU | SEQ ID NO: 1237 | GGUUUCAGGGUGUGUCUCCAC | SEQ ID NO: 2179 | GGAGACACACCCUGAAACC | 0.4785 | * |
| MA0296 | SEQ ID NO: 296 | AGACACACCCUGAAACCCUGU | SEQ ID NO: 1238 | AGGGUUUCAGGGUGUGUCUCC | SEQ ID NO: 2180 | AGACACACCCUGAAACCCU | 0.4949 | * |
| MA0297 | SEQ ID NO: 297 | ACACACCCUGAAACCCUGUGU | SEQ ID NO: 1239 | ACAGGGUUUCAGGGUGUGUCU | SEQ ID NO: 2181 | ACACACCCUGAAACCCUGU | 0.4851 | * |
| MA0298 | SEQ ID NO: 298 | CACACCCUGAAACCCUGUGUC | SEQ ID NO: 1240 | CACAGGGUUUCAGGGUGUGUC | SEQ ID NO: 2182 | CACACCCUGAAACCCUGUG | 0.5144 | * |
| MA0299 | SEQ ID NO: 299 | CACCCUGAAACCCUGUGUCCC | SEQ ID NO: 1241 | GACACAGGGUUUCAGGGUGUG | SEQ ID NO: 2183 | CACCCUGAAACCCUGUGUC | 0.3305 | |
| MA0300 | SEQ ID NO: 300 | ACCCUGAAACCCUGUGUCCCU | SEQ ID NO: 1242 | GGACACAGGGUUUCAGGGUGU | SEQ ID NO: 2184 | ACCCUGAAACCCUGUGUCC | 0.4366 | |
| MA0301 | SEQ ID NO: 301 | CCCUGAAACCCUGUGUCCCUA | SEQ ID NO: 1243 | GGGACACAGGGUUUCAGGGUG | SEQ ID NO: 2185 | CCCUGAAACCCUGUGUCCC | 0.5978 | * |
| MA0302 | SEQ ID NO: 302 | CCUGAAACCCUGUGUCCCUAC | SEQ ID NO: 1244 | AGGGACACAGGGUUUCAGGGU | SEQ ID NO: 2186 | CCUGAAACCCUGUGUCCCU | 0.4734 | |
| MA0303 | SEQ ID NO: 303 | CUGAAACCCUGUGUCCCUACG | SEQ ID NO: 1245 | UAGGGACACAGGGUUUCAGGG | SEQ ID NO: 2187 | CUGAAACCCUGUGUCCCUA | 0.2764 | |
| MA0304 | SEQ ID NO: 304 | GAAACCCUGUGUCCCUACGAC | SEQ ID NO: 1246 | CGUAGGGACACAGGGUUUCAG | SEQ ID NO: 2188 | GAAACCCUGUGUCCCUACG | 0.5062 | * |
| MA0305 | SEQ ID NO: 305 | ACCCUGUGUCCCUACGACUUU | SEQ ID NO: 1247 | AGUCGUAGGGACACAGGGUUU | SEQ ID NO: 2189 | ACCCUGUGUCCCUACGACU | 0.3557 | |
| MA0306 | SEQ ID NO: 306 | CCCUGUGUCCCUACGACUUUC | SEQ ID NO: 1248 | AAGUCGUAGGGACACAGGGUU | SEQ ID NO: 2190 | CCCUGUGUCCCUACGACUU | 0.4268 | |
| MA0307 | SEQ ID NO: 307 | CCUGUGUCCCUACGACUUUCU | SEQ ID NO: 1249 | AAAGUCGUAGGGACACAGGGU | SEQ ID NO: 2191 | CCUGUGUCCCUACGACUUU | 0.3444 | |
| MA0308 | SEQ ID NO: 308 | CUGUGUCCCUACGACUUUCUC | SEQ ID NO: 1250 | GAAAGUCGUAGGGACACAGGG | SEQ ID NO: 2192 | CUGUGUCCCUACGACUUUC | 0.2530 | |

**[Table 1-8]**

| double stranded nucleic acid number | SEQ ID NO: | sense strand sequence (5'--->3') | SEQ ID NO: | antisense strand sequence (5'--->3') | SEQ ID NO: | target MASP2 mRNA sequence | relative expression level | note |
|---|---|---|---|---|---|---|---|---|
| MA0309 | SEQ ID NO: 309 | UGUGUCCCUACGACUUUCUCA | SEQ ID NO: 1251 | AGAAAGUCGUAGGGACACAGG | SEQ ID NO: 2193 | UGUGUCCCUACGACUUUCU | 0.2532 | |
| MA0310 | SEQ ID NO: 310 | GUGUCCCUACGACUUUCUCAA | SEQ ID NO: 1252 | GAGAAAGUCGUAGGGACACAG | SEQ ID NO: 2194 | GUGUCCCUACGACUUUCUC | 0.4717 | |
| MA0311 | SEQ ID NO: 311 | UGUCCCUACGACUUUCUCAAG | SEQ ID NO: 1253 | UGAGAAAGUCGUAGGGACACA | SEQ ID NO: 2195 | UGUCCCUACGACUUUCUCA | 0.2522 | |
| MA0312 | SEQ ID NO: 312 | GUCCCUACGACUUUCUCAAGA | SEQ ID NO: 1254 | UUGAGAAAGUCGUAGGGACAC | SEQ ID NO: 2196 | GUCCCUACGACUUUCUCAA | 0.2807 | |
| MA0313 | SEQ ID NO: 313 | CCCUACGACUUUCUCAAGAUU | SEQ ID NO: 1255 | UCUUGAGAAAGUCGUAGGGAC | SEQ ID NO : 2197 | CCCUACGACUUUCUCAAGA | 0.4546 | |
| MA0314 | SEQ ID NO: 314 | CUACGACUUUCUCAAGAUUCA | SEQ ID NO: 1256 | AAUCUUGAGAAAGUCGUAGGG | SEQ ID NO: 2198 | CUACGACUUUCUCAAGAUU | 0.3930 | |
| MA0315 | SEQ ID NO: 315 | ACGACUUUCUCAAGAUUCAAA | SEQ ID NO: 1257 | UGAAUCUUGAGAAAGUCGUAG | SEQ ID NO: 2199 | ACGACUUUCUCAAGAUUCA | 0.5138 | * |
| MA0316 | SEQ ID NO: 316 | GACUUUCUCAAGAUUCAAACA | SEQ ID NO: 1258 | UUUGAAUCUUGAGAAAGUCGU | SEQ ID NO: 2200 | GACUUUCUCAAGAUUCAAA | 0.3933 | |
| MA0317 | SEQ ID NO: 317 | ACUUUCUCAAGAUUCAAACAG | SEQ ID NO: 1259 | GUUUGAAUCUUGAGAAAGUCG | SEQ ID NO: 2201 | ACUUUCUCAAGAUUCAAAC | 0.2619 | |
| MA0318 | SEQ ID NO: 318 | CUUUCUCAAGAUUCAAACAGA | SEQ ID NO: 1260 | UGUUUGAAUCUUGAGAAAGUC | SEQ ID NO: 2202 | CUUUCUCAAGAUUCAAACA | 0.3187 | |
| MA0319 | SEQ ID NO: 319 | UUCUCAAGAUUCAAACAGACA | SEQ ID NO: 1261 | UCUGUUUGAAUCUUGAGAAAG | SEQ ID NO : 2203 | UUCUCAAGAUUCAAACAGA | 0.3236 | |
| MA0320 | SEQ ID NO: 320 | UCUCAAGAUUCAAACAGACAG | SEQ ID NO: 1262 | GUCUGUUUGAAUCUUGAGAAA | SEQ ID NO: 2204 | UCUCAAGAUUCAAACAGAC | 0.5444 | * |
| MA0321 | SEQ ID NO: 321 | CUCAAGAUUCAAACAGACAGA | SEQ ID NO: 1263 | UGUCUGUUUGAAUCUUGAGAA | SEQ ID NO: 2205 | CUCAAGAUUCAAACAGACA | 0.3206 | |
| MA0322 | SEQ ID NO: 322 | UCAAGAUUCAAACAGACAGAG | SEQ ID NO: 1264 | CUGUCUGUUUGAAUCUUGAGA | SEQ ID NO: 2206 | UCAAGAUUCAAACAGACAG | 0.5993 | * |
| MA0323 | SEQ ID NO: 323 | CAAGAUUCAAACAGACAGAGA | SEQ ID NO: 1265 | UCUGUCUGUUUGAAUCUUGAG | SEQ ID NO: 2207 | CAAGAUUCAAACAGACAGA | 0.3521 | |
| MA0324 | SEQ ID NO: 324 | AAGAUUCAAACAGACAGAGAA | SEQ ID NO: 1266 | CUCUGUCUGUUUGAAUCUUGA | SEQ ID NO: 2208 | AAGAUUCAAACAGACAGAG | 0.5235 | * |
| MA0325 | SEQ ID NO: 325 | AGAUUCAAACAGACAGAGAAG | SEQ ID NO: 1267 | UCUCUGUCUGUUUGAAUCUUG | SEQ ID NO: 2209 | AGAUUCAAACAGACAGAGA | 0.4590 | * |
| MA0326 | SEQ ID NO: 326 | GAUUCAAACAGACAGAGAAGA | SEQ ID NO: 1268 | UUCUCUGUCUGUUUGAAUCUU | SEQ ID NO: 2210 | GAUUCAAACAGACAGAGAA | 0.3784 | |
| MA0327 | SEQ ID NO: 327 | CAAACAGACAGAGAAGAACAU | SEQ ID NO: 1269 | GUUCUUCUCUGUCUGUUUGAA | SEQ ID NO : 2211 | CAAACAGACAGAGAAGAAC | 0.5463 | * |
| MA0328 | SEQ ID NO: 328 | AAACAGACAGAGAAGAACAUG | SEQ ID NO: 1270 | UGUUCUUCUCUGUCUGUUUGA | SEQ ID NO: 2212 | AAACAGACAGAGAAGAACA | 0.3174 | |
| MA0329 | SEQ ID NO: 329 | AACAGACAGAGAAGAACAUGG | SEQ ID NO: 1271 | AUGUUCUUCUCUGUCUGUUUG | SEQ ID NO: 2213 | AACAGACAGAGAAGAACAU | 0.3144 | |
| MA0330 | SEQ ID NO: 330 | GACAGAGAAGAACAUGGCCCA | SEQ ID NO: 1272 | GGCCAUGUUCUUCUCUGUCUG | SEQ ID NO: 2214 | GACAGAGAAGAACAUGGCC | 0.3940 | |
| MA0331 | SEQ ID NO: 331 | CAGAGAAGAACAUGGCCCAUU | SEQ ID NO: 1273 | UGGGCCAUGUUCUUCUCUGUC | SEQ ID NO: 2215 | CAGAGAAGAACAUGGCCCA | 0.4098 | |
| MA0332 | SEQ ID NO: 332 | AGAAGAACAUGGCCCAUUCUG | SEQ ID NO: 1274 | GAAUGGGCCAUGUUCUUCUCU | SEQ ID NO: 2216 | AGAAGAACAUGGCCCAUUC | 0.3539 | |
| MA0333 | SEQ ID NO: 333 | GGCCCAUUCUGUGGGAAGACA | SEQ ID NO: 1275 | UCUUCCCACAGAAUGGGCCAU | SEQ ID NO: 2217 | GGCCCAUUCUGUGGGAAGA | 0.3980 | |
| MA0334 | SEQ ID NO: 334 | GCCCAUUCUGUGGGAAGACAU | SEQ ID NO: 1276 | GUCUUCCCACAGAAUGGGCCA | SEQ ID NO: 2218 | GCCCAUUCUGUGGGAAGAC | 0.3482 | |
| MA0335 | SEQ ID NO: 335 | CCCAUUCUGUGGGAAGACAUU | SEQ ID NO: 1277 | UGUCUUCCCACAGAAUGGGCC | SEQ ID NO: 2219 | CCCAUUCUGUGGGAAGACA | 0.3857 | |
| MA0336 | SEQ ID NO: 336 | CCAUUCUGUGGGAAGACAUUG | SEQ ID NO: 1278 | AUGUCUUCCCACAGAAUGGGC | SEQ ID NO: 2220 | CCAUUCUGUGGGAAGACAU | 0.2614 | |
| MA0337 | SEQ ID NO: 337 | CAUUCUGUGGGAAGACAUUGC | SEQ ID NO: 1279 | AAUGUCUUCCCACAGAAUGGG | SEQ ID NO: 2221 | CAUUCUGUGGGAAGACAUU | 0.2040 | |
| MA0338 | SEQ ID NO: 338 | GACAUUGCCCCACAGGAUUGA | SEQ ID NO: 1280 | AAUCCUGUGGGGCAAUGUCUU | SEQ ID NO: 2222 | GACAUUGCCCCACAGGAUU | 0.4934 | * |
| MA0339 | SEQ ID NO: 339 | GCCCCACAGGAUUGAAACAAA | SEQ ID NO: 1281 | UGUUUCAAUCCUGUGGGGCAA | SEQ ID NO: 2223 | GCCCCACAGGAUUGAAACA | 0.4485 | |
| MA0340 | SEQ ID NO: 340 | CCCACAGGAUUGAAACAAAAA | SEQ ID NO: 1282 | UUUGUUUCAAUCCUGUGGGGC | SEQ ID NO: 2224 | CCCACAGGAUUGAAACAAA | 0.1891 | |
| MA0341 | SEQ ID NO: 341 | CCACAGGAUUGAAACAAAAAG | SEQ ID NO: 1283 | UUUUGUUUCAAUCCUGUGGGG | SEQ ID NO: 2225 | CCACAGGAUUGAAACAAAA | 0.2010 | |
| MA0342 | SEQ ID NO: 342 | CACAGGAUUGAAACAAAAAGC | SEQ ID NO: 1284 | UUUUUGUUUCAAUCCUGUGGG | SEQ ID NO: 2226 | CACAGGAUUGAAACAAAAA | 0.1805 | |
| MA0343 | SEQ ID NO: 343 | ACAGGAUUGAAACAAAAAGCA | SEQ ID NO: 1285 | CUUUUUGUUUCAAUCCUGUGG | SEQ ID NO: 2227 | ACAGGAUUGAAACAAAAAG | 0.3219 | |
| MA0344 | SEQ ID NO: 344 | GGAUUGAAACAAAAAGCAACA | SEQ ID NO: 1286 | UUGCUUUUUGUUUCAAUCCUG | SEQ ID NO: 2228 | GGAUUGAAACAAAAAGCAA | 0.2485 | |
| MA0345 | SEQ ID NO: 345 | GAUUGAAACAAAAAGCAACAC | SEQ ID NO: 1287 | GUUGCUUUUUGUUUCAAUCCU | SEQ ID NO: 2229 | GAUUGAAACAAAAAGCAAC | 0.4567 | |
| MA0346 | SEQ ID NO: 346 | AUUGAAACAAAAAGCAACACG | SEQ ID NO: 1288 | UGUUGCUUUUUGUUUCAAUCC | SEQ ID NO: 2230 | AUUGAAACAAAAAGCAACA | 0.3183 | |
| MA0347 | SEQ ID NO: 347 | GAAACAAAAAGCAACACGGUG | SEQ ID NO: 1289 | CCGUGUUGCUUUUUGUUUCAA | SEQ ID NO: 2231 | GAAACAAAAAGCAACACGG | 0.3736 | |
| MA0348 | SEQ ID NO: 348 | AAACAAAAAGCAACACGGUGA | SEQ ID NO: 1290 | ACCGUGUUGCUUUUUGUUUCA | SEQ ID NO: 2232 | AAACAAAAAGCAACACGGU | 0.3500 | |
| MA0349 | SEQ ID NO: 349 | ACAAAAAGCAACACGGUGACC | SEQ ID NO: 1291 | UCACCGUGUUGCUUUUUGUUU | SEQ ID NO: 2233 | ACAAAAAGCAACACGGUGA | 0.3531 | |
| MA0350 | SEQ ID NO: 350 | CAAAAAGCAACACGGUGACCA | SEQ ID NO: 1292 | GUCACCGUGUUGCUUUUUGUU | SEQ ID NO: 2234 | CAAAAAGCAACACGGUGAC | 0.5065 | |
| MA0351 | SEQ ID NO: 351 | AAAAGCAACACGGUGACCAUC | SEQ ID NO: 1293 | UGGUCACCGUGUUGCUUUUUG | SEQ ID NO: 2235 | AAAAGCAACACGGUGACCA | 0.5195 | |
| MA0352 | SEQ ID NO: 352 | AAAGCAACACGGUGACCAUCA | SEQ ID NO: 1294 | AUGGUCACCGUGUUGCUUUUU | SEQ ID NO: 2236 | AAAGCAACACGGUGACCAU | 0.5191 | |

**[Table 1-9]**

| double stranded nucleic acid number | SEQ ID NO: | sense strand sequence (5'--->3') | SEQ ID NO: | antisense strand sequence (5'--->3') | SEQ ID NO: | target MASP2 mRNA sequence | relative expression level | note |
|---|---|---|---|---|---|---|---|---|
| MA0353 | SEQ ID NO: 353 | AAGCAACACGGUGACCAUCAC | SEQ ID NO: 1295 | GAUGGUCACCGUGUUGCUUUU | SEQ ID NO: 2237 | AAGCAACACGGUGACCAUC | 0.5662 | * |
| MA0354 | SEQ ID NO: 354 | ACACGGUGACCAUCACCUUUG | SEQ ID NO: 1296 | AAGGUGAUGGUCACCGUGUUG | SEQ ID NO: 2238 | ACACGGUGACCAUCACCUU | 0.3121 | |
| MA0355 | SEQ ID NO: 355 | CACGGUGACCAUCACCUUUGU | SEQ ID NO: 1297 | AAAGGUGAUGGUCACCGUGUU | SEQ ID NO: 2239 | CACGGUGACCAUCACCUUU | 0.3430 | |
| MA0356 | SEQ ID NO: 356 | ACGGUGACCAUCACCUUUGUC | SEQ ID NO: 1298 | CAAAGGUGAUGGUCACCGUGU | SEQ ID NO: 2240 | ACGGUGACCAUCACCUUUG | 0.2989 | |
| MA0357 | SEQ ID NO: 357 | CGGUGACCAUCACCUUUGUCA | SEQ ID NO: 1299 | ACAAAGGUGAUGGUCACCGUG | SEQ ID NO: 2241 | CGGUGACCAUCACCUUUGU | 0.1661 | |
| MA0358 | SEQ ID NO: 358 | GGUGACCAUCACCUUUGUCAC | SEQ ID NO: 1300 | GACAAAGGUGAUGGUCACCGU | SEQ ID NO: 2242 | GGUGACCAUCACCUUUGUC | 0.5138 | * |
| MA0359 | SEQ ID NO: 359 | GUGACCAUCACCUUUGUCACA | SEQ ID NO: 1301 | UGACAAAGGUGAUGGUCACCG | SEQ ID NO: 2243 | GUGACCAUCACCUUUGUCA | 0.5498 | * |
| MA0360 | SEQ ID NO: 360 | GACCAUCACCUUUGUCACAGA | SEQ 10 NO: 1302 | UGUGACAAAGGUGAUGGUCAC | SEQ ID NO: 2244 | GACCAUCACCUUUGUCACA | 0.4169 | |
| MA0361 | SEQ ID NO: 361 | ACCAUCACCUUUGUCACAGAU | SEQ ID NO: 1303 | CUGUGACAAAGGUGAUGGUCA | SEQ ID NO: 2245 | ACCAUCACCUUUGUCACAG | 0.4109 | |
| MA0362 | SEQ ID NO: 362 | CCAUCACCUUUGUCACAGAUG | SEQ ID NO: 1304 | UCUGUGACAAAGGUGAUGGUC | SEQ ID NO: 2246 | CCAUCACCUUUGUCACAGA | 0.2872 | |
| MA0363 | SEQ ID NO: 363 | CAUCACCUUUGUCACAGAUGA | SEQ ID NO: 1305 | AUCUGUGACAAAGGUGAUGGU | SEQ ID NO: 2247 | CAUCACCUUUGUCACAGAU | 0.2772 | |
| MA0364 | SEQ ID NO: 364 | UCACCUUUGUCACAGAUGAAU | SEQ ID NO: 1306 | UCAUCUGUGACAAAGGUGAUG | SEQ ID NO: 2248 | UCACCUUUGUCACAGAUGA | 0.3394 | |
| MA0365 | SEQ ID NO: 365 | ACCUUUGUCACAGAUGAAUCA | SEQ ID NO: 1307 | AUUCAUCUGUGACAAAGGUGA | SEQ ID NO: 2249 | ACCUUUGUCACAGAUGAAU | 0.4653 | * |
| MA0366 | SEQ ID NO: 366 | CCUUUGUCACAGAUGAAUCAG | SEQ ID NO: 1308 | GAUUCAUCUGUGACAAAGGUG | SEQ ID NO: 2250 | CCUUUGUCACAGAUGAAUC | 0.5571 | * |
| MA0367 | SEQ ID NO: 367 | CUUUGUCACAGAUGAAUCAGG | SEQ ID NO: 1309 | UGAUUCAUCUGUGACAAAGGU | SEQ ID NO: 2251 | CUUUGUCACAGAUGAAUCA | 0.5002 | * |
| MA0368 | SEQ ID NO: 368 | UGUCACAGAUGAAUCAGGAGA | SEQ ID NO: 1310 | UCCUGAUUCAUCUGUGACAAA | SEQ ID NO: 2252 | UGUCACAGAUGAAUCAGGA | 0.3262 | |
| MA0369 | SEQ ID NO: 369 | UCACAGAUGAAUCAGGAGACC | SEQ ID NO: 1311 | UCUCCUGAUUCAUCUGUGACA | SEQ ID NO: 2253 | UCACAGAUGAAUCAGGAGA | 0.4704 | * |
| MA0370 | SEQ ID NO: 370 | ACAGAUGAAUCAGGAGACCAC | SEQ ID NO: 1312 | GGUCUCCUGAUUCAUCUGUGA | SEQ ID NO: 2254 | ACAGAUGAAUCAGGAGACC | 0.2196 | |
| MA0371 | SEQ ID NO: 371 | CAGAUGAAUCAGGAGACCACA | SEQ ID NO: 1313 | UGGUCUCCUGAUUCAUCUGUG | SEQ ID NO: 2255 | CAGAUGAAUCAGGAGACCA | 0.2379 | |
| MA0372 | SEQ ID NO: 372 | AGAUGAAUCAGGAGACCACAC | SEQ ID NO: 1314 | GUGGUCUCCUGAUUCAUCUGU | SEQ ID NO: 2256 | AGAUGAAUCAGGAGACCAC | 0.2312 | |
| MA0373 | SEQ ID NO: 373 | CAGGAGACCACACAGGCUGGA | SEQ ID NO: 1315 | CAGCCUGUGUGGUCUCCUGAU | SEQ ID NO: 2257 | CAGGAGACCACACAGGCUG | 0.3972 | |
| MA0374 | SEQ ID NO: 374 | GAGACCACACAGGCUGGAAGA | SEQ ID NO: 1316 | UUCCAGCCUGUGUGGUCUCCU | SEQ ID NO: 2258 | GAGACCACACAGGCUGGAA | 0.3681 | |
| MA0375 | SEQ ID NO: 375 | AGACCACACAGGCUGGAAGAU | SEQ ID NO: 1317 | CUUCCAGCCUGUGUGGUCUCC | SEQ ID NO: 2259 | AGACCACACAGGCUGGAAG | 0.1920 | |
| MA0376 | SEQ ID NO: 376 | GACCACACAGGCUGGAAGAUC | SEQ ID NO: 1318 | UCUUCCAGCCUGUGUGGUCUC | SEQ ID NO: 2260 | GACCACACAGGCUGGAAGA | 0.3744 | |
| MA0377 | SEQ ID NO: 377 | ACCACACAGGCUGGAAGAUCC | SEQ ID NO: 1319 | AUCUUCCAGCCUGUGUGGUCU | SEQ ID NO: 2261 | ACCACACAGGCUGGAAGAU | 0.0886 | |
| MA0378 | SEQ ID NO: 378 | CACACAGGCUGGAAGAUCCAC | SEQ ID NO: 1320 | GGAUCUUCCAGCCUGUGUGGU | SEQ ID NO: 2262 | CACACAGGCUGGAAGAUCC | 0.5133 | * |
| MA0379 | SEQ ID NO: 379 | ACAGGCUGGAAGAUCCACUAC | SEQ ID NO: 1321 | AGUGGAUCUUCCAGCCUGUGU | SEQ ID NO: 2263 | ACAGGCUGGAAGAUCCACU | 0.4863 | |
| MA0380 | SEQ ID NO: 380 | CAGGCUGGAAGAUCCACUACA | SEQ ID NO: 1322 | UAGUGGAUCUUCCAGCCUGUG | SEQ ID NO: 2264 | CAGGCUGGAAGAUCCACUA | 0.3014 | |
| MA0381 | SEQ ID NO: 381 | AGGCUGGAAGAUCCACUACAC | SEQ ID NO: 1323 | GUAGUGGAUCUUCCAGCCUGU | SEQ ID NO: 2265 | AGGCUGGAAGAUCCACUAC | 0.5415 | * |
| MA0382 | SEQ ID NO: 382 | GGCUGGAAGAUCCACUACACG | SEQ ID NO: 1324 | UGUAGUGGAUCUUCCAGCCUG | SEQ ID NO: 2266 | GGCUGGAAGAUCCACUACA | 0.3350 | |
| MA0383 | SEQ ID NO: 383 | GCUGGAAGAUCCACUACACGA | SEQ ID NO: 1325 | GUGUAGUGGAUCUUCCAGCCU | SEQ ID NO: 2267 | GCUGGAAGAUCCACUACAC | 0.5410 | * |
| MA0384 | SEQ ID NO: 384 | CUGGAAGAUCCACUACACGAG | SEQ ID NO: 1326 | CGUGUAGUGGAUCUUCCAGCC | SEQ ID NO: 2268 | CUGGAAGAUCCACUACACG | 0.2077 | |
| MA0385 | SEQ ID NO: 385 | UGGAAGAUCCACUACACGAGC | SEQ ID NO: 1327 | UCGUGUAGUGGAUCUUCCAGC | SEQ ID NO: 2269 | UGGAAGAUCCACUACACGA | 0.3152 | |
| MA0386 | SEQ ID NO: 386 | GGAAGAUCCACUACACGAGCA | SEQ ID NO: 1328 | CUCGUGUAGUGGAUCUUCCAG | SEQ ID NO: 2270 | GGAAGAUCCACUACACGAG | 0.4167 | |
| MA0387 | SEQ ID NO: 387 | AGAUCCACUACACGAGCACAG | SEQ ID NO: 1329 | GUGCUCGUGUAGUGGAUCUUC | SEQ ID NO: 2271 | AGAUCCACUACACGAGCAC | 0.4602 | * |
| MA0388 | SEQ ID NO: 388 | GAUCCACUACACGAGCACAGC | SEQ ID NO: 1330 | UGUGCUCGUGUAGUGGAUCUU | SEQ ID NO: 2272 | GAUCCACUACACGAGCACA | 0.3816 | |
| MA0389 | SEQ ID NO: 389 | CCACUACACGAGCACAGCGCA | SEQ ID NO: 1331 | CGCUGUGCUCGUGUAGUGGAU | SEQ ID NO: 2273 | CCACUACACGAGCACAGCG | 0.4488 | |
| MA0390 | SEQ ID NO: 390 | ACUACACGAGCACAGCGCAGC | SEQ ID NO: 1332 | UGCGCUGUGCUCGUGUAGUGG | SEQ ID NO: 2274 | ACUACACGAGCACAGCGCA | 0.5738 | * |
| MA0391 | SEQ ID NO: 391 | CUACACGAGCACAGCGCAGCC | SEQ ID NO: 1333 | CUGCGCUGUGCUCGUGUAGUG | SEQ ID NO: 2275 | CUACACGAGCACAGCGCAG | 0.3150 | |
| MA0392 | SEQ ID NO: 392 | ACAGCGCAGCCUUGCCCUUAU | SEQ ID NO: 1334 | AAGGGCAAGGCUGCGCUGUGC | SEQ ID NO: 2276 | ACAGCGCAGCCUUGCCCUU | 0.4304 | |
| MA0393 | SEQ ID NO: 393 | CAGCGCAGCCUUGCCCUUAUC | SEQ ID NO: 1335 | UAAGGGCAAGGCUGCGCUGUG | SEQ ID NO: 2277 | CAGCGCAGCCUUGCCCUUA | 0.5605 | |
| MA0394 | SEQ ID NO: 394 | AGCGCAGCCUUGCCCUUAUCC | SEQ ID NO: 1336 | AUAAGGGCAAGGCUGCGCUGU | SEQ ID NO: 2278 | AGCGCAGCCUUGCCCUUAU | 0.4667 | |
| MA0395 | SEQ ID NO: 395 | GCGCAGCCUUGCCCUUAUCCG | SEQ ID NO: 1337 | GAUAAGGGCAAGGCUGCGCUG | SEQ ID NO: 2279 | GCGCAGCCUUGCCCUUAUC | 0.4770 | * |
| MA0396 | SEQ ID NO: 396 | CAGCCUUGCCCUUAUCCGAUG | SEQ ID NO: 1338 | UCGGAUAAGGGCAAGGCUGCG | SEQ ID NO: 2280 | CAGCCUUGCCCUUAUCCGA | 0.4466 | |

**[Table 1-10]**

| double stranded nucleic acid number | SEQ ID NO: | sense strand sequence (5'--->3') | SEQ ID NO: | antisense strand sequence (5'--->3') | SEQ ID NO: | target MASP2 mRNA sequence | relative expression level | note |
|---|---|---|---|---|---|---|---|---|
| MA0397 | SEQ ID NO: 397 | AGCCUUGCCCUUAUCCGAUGG | SEQ ID NO: 1339 | AUCGGAUAAGGGCAAGGCUGC | SEQ ID NO: 2281 | AGCCUUGCCCUUAUCCGAU | 0.2460 | |
| MA0398 | SEQ ID NO: 398 | GCCUUGCCCUUAUCCGAUGGC | SEQ ID NO: 1340 | CAUCGGAUAAGGGCAAGGCUG | SEQ ID NO: 2282 | GCCUUGCCCUUAUCCGAUG | 0.5001 | * |
| MA0399 | SEQ ID NO: 399 | GCCCUUAUCCGAUGGCGCCAC | SEQ ID NO: 1341 | GGCGCCAUCGGAUAAGGGCAA | SEQ ID NO: 2283 | GCCCUUAUCCGAUGGCGCC | 0.5112 | * |
| MA0400 | SEQ ID NO: 400 | AUCCGAUGGCGCCACCUAAUG | SEQ ID NO: 1342 | UUAGGUGGCGCCAUCGGAUAA | SEQ ID NO: 2284 | AUCCGAUGGCGCCACCUAA | 0.3946 | |
| MA0401 | SEQ ID NO: 401 | UCCGAUGGCGCCACCUAAUGG | SEQ ID NO: 1343 | AUUAGGUGGCGCCAUCGGAUA | SEQ ID NO: 2285 | UCCGAUGGCGCCACCUAAU | 0.5387 | |
| MA0402 | SEQ ID NO: 402 | CGAUGGCGCCACCUAAUGGCC | SEQ ID NO: 1344 | CCAUUAGGUGGCGCCAUCGGA | SEQ ID NO: 2286 | CGAUGGCGCCACCUAAUGG | 0.5710 | * |
| MA0403 | SEQ ID NO: 403 | UGGCGCCACCUAAUGGCCACG | SEQ ID NO: 1345 | UGGCCAUUAGGUGGCGCCAUC | SEQ ID NO: 2287 | UGGCGCCACCUAAUGGCCA | 0.4455 | |
| MA0404 | SEQ ID NO: 404 | GGCGCCACCUAAUGGCCACGU | SEQ ID NO: 1346 | GUGGCCAUUAGGUGGCGCCAU | SEQ ID NO: 2288 | GGCGCCACCUAAUGGCCAC | 0.5239 | * |
| MA0405 | SEQ ID NO: 405 | CCACCUAAUGGCCACGUUUCA | SEQ ID NO: 1347 | AAACGUGGCCAUUAGGUGGCG | SEQ ID NO: 2289 | CCACCUAAUGGCCACGUUU | 0.4512 | |
| MA0406 | SEQ ID NO: 406 | CACCUAAUGGCCACGUUUCAC | SEQ ID NO: 1348 | GAAACGUGGCCAUUAGGUGGC | SEQ ID NO: 2290 | CACCUAAUGGCCACGUUUC | 0.4201 | |
| MA0407 | SEQ ID NO: 407 | ACCUAAUGGCCACGUUUCACC | SEQ ID NO: 1349 | UGAAACGUGGCCAUUAGGUGG | SEQ ID NO: 2291 | ACCUAAUGGCCACGUUUCA | 0.3158 | |
| MA0408 | SEQ ID NO: 408 | UAAUGGCCACGUUUCACCUGU | SEQ ID NO: 1350 | AGGUGAAACGUGGCCAUUAGG | SEQ ID NO: 2292 | UAAUGGCCACGUUUCACCU | 0.4647 | |
| MA0409 | SEQ ID NO: 409 | AUGGCCACGUUUCACCUGUGC | SEQ ID NO: 1351 | ACAGGUGAAACGUGGCCAUUA | SEQ ID NO: 2293 | AUGGCCACGUUUCACCUGU | 0.2814 | |
| MA0410 | SEQ ID NO: 410 | UGGCCACGUUUCACCUGUGCA | SEQ ID NO: 1352 | CACAGGUGAAACGUGGCCAUU | SEQ ID NO: 2294 | UGGCCACGUUUCACCUGUG | 0.4897 | * |
| MA0411 | SEQ ID NO: 411 | GGCCACGUUUCACCUGUGCAA | SEQ ID NO: 1353 | GCACAGGUGAAACGUGGCCAU | SEQ ID NO: 2295 | GGCCACGUUUCACCUGUGC | 0.5642 | * |
| MA0412 | SEQ ID NO: 412 | CACGUUUCACCUGUGCAAGCC | SEQ ID NO: 1354 | CUUGCACAGGUGAAACGUGGC | SEQ ID NO: 2296 | CACGUUUCACCUGUGCAAG | 0.3378 | |
| MA0413 | SEQ ID NO: 413 | ACGUUUCACCUGUGCAAGCCA | SEQ ID NO: 1355 | GCUUGCACAGGUGAAACGUGG | SEQ ID NO: 2297 | ACGUUUCACCUGUGCAAGC | 0.3783 | |
| MA0414 | SEQ ID NO: 414 | CGUUUCACCUGUGCAAGCCAA | SEQ ID NO: 1356 | GGCUUGCACAGGUGAAACGUG | SEQ ID NO: 2298 | CGUUUCACCUGUGCAAGCC | 0.4860 | |
| MA0415 | SEQ ID NO: 415 | UUUCACCUGUGCAAGCCAAAU | SEQ ID NO: 1357 | UUGGCUUGCACAGGUGAAACG | SEQ ID NO: 2299 | UUUCACCUGUGCAAGCCAA | 0.5422 | * |
| MA0416 | SEQ ID NO: 416 | UUCACCUGUGCAAGCCAAAUA | SEQ ID NO: 1358 | UUUGGCUUGCACAGGUGAAAC | SEQ ID NO: 2300 | UUCACCUGUGCAAGCCAAA | 0.4283 | |
| MA0417 | SEQ ID NO: 417 | CACCUGUGCAAGCCAAAUACA | SEQ ID NO: 1359 | UAUUUGGCUUGCACAGGUGAA | SEQ ID NO: 2301 | CACCUGUGCAAGCCAAAUA | 0.2541 | |
| MA0418 | SEQ ID NO: 418 | ACCUGUGCAAGCCAAAUACAU | SEQ ID NO: 1360 | GUAUUUGGCUUGCACAGGUGA | SEQ ID NO: 2302 | ACCUGUGCAAGCCAAAUAC | 0.3453 | |
| MA0419 | SEQ ID NO: 419 | CCUGUGCAAGCCAAAUACAUC | SEQ ID NO: 1361 | UGUAUUUGGCUUGCACAGGUG | SEQ ID NO: 2303 | CCUGUGCAAGCCAAAUACA | 0.5007 | |
| MA0420 | SEQ ID NO: 420 | CUGUGCAAGCCAAAUACAUCC | SEQ ID NO: 1362 | AUGUAUUUGGCUUGCACAGGU | SEQ ID NO: 2304 | CUGUGCAAGCCAAAUACAU | 0.4071 | |
| MA0421 | SEQ ID NO: 421 | UGUGCAAGCCAAAUACAUCCU | SEQ ID NO: 1363 | GAUGUAUUUGGCUUGCACAGG | SEQ ID NO: 2305 | UGUGCAAGCCAAAUACAUC | 0.3048 | |
| MA0422 | SEQ ID NO: 422 | GUGCAAGCCAAAUACAUCCUG | SEQ ID NO: 1364 | GGAUGUAUUUGGCUUGCACAG | SEQ ID NO: 2306 | GUGCAAGCCAAAUACAUCC | 0.5079 | * |
| MA0423 | SEQ ID NO: 423 | CAAGCCAAAUACAUCCUGAAA | SEQ ID NO: 1365 | UCAGGAUGUAUUUGGCUUGCA | SEQ ID NO: 2307 | CAAGCCAAAUACAUCCUGA | 0.5448 | |
| MA0424 | SEQ ID NO: 424 | AAGCCAAAUACAUCCUGAAAG | SEQ ID NO: 1366 | UUCAGGAUGUAUUUGGCUUGC | SEQ ID NO: 2308 | AAGCCAAAUACAUCCUGAA | 0.1603 | |
| MA0425 | SEQ ID NO: 425 | AGCCAAAUACAUCCUGAAAGA | SEQ ID NO: 1367 | UUUCAGGAUGUAUUUGGCUUG | SEQ ID NO: 2309 | AGCCAAAUACAUCCUGAAA | 0.3699 | |
| MA0426 | SEQ ID NO: 426 | GCCAAAUACAUCCUGAAAGAC | SEQ ID NO: 1368 | CUUUCAGGAUGUAUUUGGCUU | SEQ ID NO: 2310 | GCCAAAUACAUCCUGAAAG | 0.4184 | |
| MA0427 | SEQ ID NO: 427 | CCAAAUACAUCCUGAAAGACA | SEQ ID NO: 1369 | UCUUUCAGGAUGUAUUUGGCU | SEQ ID NO: 2311 | CCAAAUACAUCCUGAAAGA | 0.2512 | |
| MA0428 | SEQ ID NO: 428 | CAAAUACAUCCUGAAAGACAG | SEQ ID NO: 1370 | GUCUUUCAGGAUGUAUUUGGC | SEQ ID NO: 2312 | CAAAUACAUCCUGAAAGAC | 0.5064 | |
| MA0429 | SEQ ID NO: 429 | AAAUACAUCCUGAAAGACAGC | SEQ ID NO: 1371 | UGUCUUUCAGGAUGUAUUUGG | SEQ ID NO: 2313 | AAAUACAUCCUGAAAGACA | 0.2717 | |
| MA0430 | SEQ ID NO: 430 | CAUCCUGAAAGACAGCUUCUC | SEQ ID NO: 1372 | GAAGCUGUCUUUCAGGAUGUA | SEQ ID NO: 2314 | CAUCCUGAAAGACAGCUUC | 0.4665 | |
| MA0431 | SEQ ID NO: 431 | UCCUGAAAGACAGCUUCUCCA | SEQ ID NO: 1373 | GAGAAGCUGUCUUUCAGGAUG | SEQ ID NO: 2315 | UCCUGAAAGACAGCUUCUC | 0.5761 | * |
| MA0432 | SEQ ID NO: 432 | CUGAAAGACAGCUUCUCCAUC | SEQ ID NO: 1374 | UGGAGAAGCUGUCUUUCAGGA | SEQ ID NO: 2316 | CUGAAAGACAGCUUCUCCA | 0.5338 | * |
| MA0433 | SEQ ID NO: 433 | UGAAAGACAGCUUCUCCAUCU | SEQ ID NO: 1375 | AUGGAGAAGCUGUCUUUCAGG | SEQ ID NO: 2317 | UGAAAGACAGCUUCUCCAU | 0.3153 | |
| MA0434 | SEQ ID NO: 434 | GAAAGACAGCUUCUCCAUCUU | SEQ ID NO: 1376 | GAUGGAGAAGCUGUCUUUCAG | SEQ ID NO: 2318 | GAAAGACAGCUUCUCCAUC | 0.4992 | |
| MA0435 | SEQ ID NO: 435 | AGACAGCUUCUCCAUCUUUUG | SEQ ID NO: 1377 | AAAGAUGGAGAAGCUGUCUUU | SEQ ID NO: 2319 | AGACAGCUUCUCCAUCUUU | 0.5014 | |
| MA0436 | SEQ ID NO: 436 | GACAGCUUCUCCAUCUUUUGC | SEQ ID NO: 1378 | AAAAGAUGGAGAAGCUGUCUU | SEQ ID NO: 2320 | GACAGCUUCUCCAUCUUUU | 0.5467 | * |
| MA0437 | SEQ ID NO: 437 | GCUUCUCCAUCUUUUGCGAGA | SEQ ID NO: 1379 | UCGCAAAAGAUGGAGAAGCUG | SEQ ID NO: 2321 | GCUUCUCCAUCUUUUGCGA | 0.2675 | |
| MA0438 | SEQ ID NO: 438 | CUCCAUCUUUUGCGAGACUGG | SEQ ID NO: 1380 | AGUCUCGCAAAAGAUGGAGAA | SEQ ID NO: 2322 | CUCCAUCUUUUGCGAGACU | 0.4516 | |
| MA0439 | SEQ ID NO: 439 | CCAUCUUUUGCGAGACUGGCU | SEQ ID NO: 1381 | CCAGUCUCGCAAAAGAUGGAG | SEQ ID NO: 2323 | CCAUCUUUUGCGAGACUGG | 0.1952 | |
| MA0440 | SEQ ID NO: 440 | CAUCUUUUGCGAGACUGGCUA | SEQ ID NO: 1382 | GCCAGUCUCGCAAAAGAUGGA | SEQ ID NO: 2324 | CAUCUUUUGCGAGACUGGC | 0.4113 | |

**[Table 1-11]**

| double stranded nucleic acid number | SEQ ID NO: | sense strand sequence (5'--->3') | SEQ ID NO: | antisense strand sequence (5'--->3') | SEQ ID NO: | target MASP2 mRNA sequence | relative expression level | note |
|---|---|---|---|---|---|---|---|---|
| MA0441 | SEQ ID NO: 441 | AUCUUUUGCGAGACUGGCUAU | SEQ ID NO: 1383 | AGCCAGUCUCGCAAAAGAUGG | SEQ ID NO: 2325 | AUCUUUUGCGAGACUGGCU | 0.5191 | * |
| MA0442 | SEQ ID NO: 442 | GCGAGACUGGCUAUGAGCUUC | SEQ ID NO: 1384 | AGCUCAUAGCCAGUCUCGCAA | SEQ ID NO: 2326 | GCGAGACUGGCUAUGAGCU | 0.4694 | * |
| MA0443 | SEQ ID NO: 443 | CGAGACUGGCUAUGAGCUUCU | SEQ ID NO: 1385 | AAGCUCAUAGCCAGUCUCGCA | SEQ ID NO: 2327 | CGAGACUGGCUAUGAGCUU | 0.3900 | |
| MA0444 | SEQ ID NO: 444 | AGACUGGCUAUGAGCUUCUGC | SEQ ID NO: 1386 | AGAAGCUCAUAGCCAGUCUCG | SEQ ID NO: 2328 | AGACUGGCUAUGAGCUUCU | 0.4448 | |
| MA0445 | SEQ ID NO: 445 | CUGGCUAUGAGCUUCUGCAAG | SEQ ID NO: 1387 | UGCAGAAGCUCAUAGCCAGUC | SEQ ID NO: 2329 | CUGGCUAUGAGCUUCUGCA | 0.3588 | |
| MA0446 | SEQ ID NO: 446 | GGCUAUGAGCUUCUGCAAGGU | SEQ ID NO: 1388 | CUUGCAGAAGCUCAUAGCCAG | SEQ ID NO: 2330 | GGCUAUGAGCUUCUGCAAG | 0.4931 | * |
| MA0447 | SEQ ID NO: 447 | GCUAUGAGCUUCUGCAAGGUC | SEQ ID NO: 1389 | CCUUGCAGAAGCUCAUAGCCA | SEQ ID NO: 2331 | GCUAUGAGCUUCUGCAAGG | 0.4732 | * |
| MA0448 | SEQ ID NO: 448 | GAGCUUCUGCAAGGUCACUUG | SEQ ID NO: 1390 | AGUGACCUUGCAGAAGCUCAU | SEQ ID NO: 2332 | GAGCUUCUGCAAGGUCACU | 0.3265 | |
| MA0449 | SEQ ID NO: 449 | AGCUUCUGCAAGGUCACUUGC | SEQ ID NO: 1391 | AAGUGACCUUGCAGAAGCUCA | SEQ ID NO: 2333 | AGCUUCUGCAAGGUCACUU | 0.5674 | * |
| MA0450 | SEQ ID NO: 450 | GCAAGGUCACUUGCCCCUGAA | SEQ ID NO: 1392 | CAGGGGCAAGUGACCUUGCAG | SEQ ID NO: 2334 | GCAAGGUCACUUGCCCCUG | 0.5893 | * |
| MA0451 | SEQ ID NO: 451 | CAAGGUCACUUGCCCCUGAAA | SEQ ID NO: 1393 | UCAGGGGCAAGUGACCUUGCA | SEQ ID NO: 2335 | CAAGGUCACUUGCCCCUGA | 0.3773 | |
| MA0452 | SEQ ID NO: 452 | AAGGUCACUUGCCCCUGAAAU | SEQ ID NO: 1394 | UUCAGGGGCAAGUGACCUUGC | SEQ ID NO: 2336 | AAGGUCACUUGCCCCUGAA | 0.4152 | |
| MA0453 | SEQ ID NO: 453 | AGGUCACUUGCCCCUGAAAUC | SEQ ID NO: 1395 | UUUCAGGGGCAAGUGACCUUG | SEQ ID NO: 2337 | AGGUCACUUGCCCCUGAAA | 0.3239 | |
| MA0454 | SEQ ID NO: 454 | GGUCACUUGCCCCUGAAAUCC | SEQ ID NO: 1396 | AUUUCAGGGGCAAGUGACCUU | SEQ ID NO: 2338 | GGUCACUUGCCCCUGAAAU | 0.2725 | |
| MA0455 | SEQ ID NO: 455 | GUCACUUGCCCCUGAAAUCCU | SEQ ID NO: 1397 | GAUUUCAGGGGCAAGUGACCU | SEQ ID NO: 2339 | GUCACUUGCCCCUGAAAUC | 0.2651 | |
| MA0456 | SEQ ID NO: 456 | CUUGCCCCUGAAAUCCUUUAC | SEQ ID NO: 1398 | AAAGGAUUUCAGGGGCAAGUG | SEQ ID NO: 2340 | CUUGCCCCUGAAAUCCUUU | 0.5417 | |
| MA0457 | SEQ ID NO: 457 | UUGCCCCUGAAAUCCUUUACU | SEQ ID NO: 1399 | UAAAGGAUUUCAGGGGCAAGU | SEQ ID NO: 2341 | UUGCCCCUGAAAUCCUUUA | 0.5754 | * |
| MA0458 | SEQ ID NO: 458 | UGCCCCUGAAAUCCUUUACUG | SEQ ID NO: 1400 | GUAAAGGAUUUCAGGGGCAAG | SEQ ID NO: 2342 | UGCCCCUGAAAUCCUUUAC | 0.5572 | * |
| MA0459 | SEQ ID NO: 459 | GCCCCUGAAAUCCUUUACUGC | SEQ ID NO: 1401 | AGUAAAGGAUUUCAGGGGCAA | SEQ ID NO: 2343 | GCCCCUGAAAUCCUUUACU | 0.3631 | |
| MA0460 | SEQ ID NO: 460 | CCCCUGAAAUCCUUUACUGCA | SEQ ID NO: 1402 | CAGUAAAGGAUUUCAGGGGCA | SEQ ID NO: 2344 | CCCCUGAAAUCCUUUACUG | 0.1982 | |
| MA0461 | SEQ ID NO: 461 | CCCUGAAAUCCUUUACUGCAG | SEQ ID NO: 1403 | GCAGUAAAGGAUUUCAGGGGC | SEQ ID NO: 2345 | CCCUGAAAUCCUUUACUGC | 0.3964 | |
| MA0462 | SEQ ID NO: 462 | CCUGAAAUCCUUUACUGCAGU | SEQ ID NO: 1404 | UGCAGUAAAGGAUUUCAGGGG | SEQ ID NO: 2346 | CCUGAAAUCCUUUACUGCA | 0.4854 | |
| MA0463 | SEQ ID NO: 463 | GAAAUCCUUUACUGCAGUUUG | SEQ ID NO: 1405 | AACUGCAGUAAAGGAUUUCAG | SEQ ID NO: 2347 | GAAAUCCUUUACUGCAGUU | 0.2896 | |
| MA0464 | SEQ ID NO: 464 | AAAUCCUUUACUGCAGUUUGU | SEQ ID NO: 1406 | AAACUGCAGUAAAGGAUUUCA | SEQ ID NO: 2348 | AAAUCCUUUACUGCAGUUU | 0.4440 | |
| MA0465 | SEQ ID NO: 465 | AUCCUUUACUGCAGUUUGUCA | SEQ ID NO: 1407 | ACAAACUGCAGUAAAGGAUUU | SEQ ID NO: 2349 | AUCCUUUACUGCAGUUUGU | 0.3501 | |
| MA0466 | SEQ ID NO: 466 | UCCUUUACUGCAGUUUGUCAG | SEQ ID NO: 1408 | GACAAACUGCAGUAAAGGAUU | SEQ ID NO: 2350 | UCCUUUACUGCAGUUUGUC | 0.5265 | * |
| MA0467 | SEQ ID NO: 467 | CCUUUACUGCAGUUUGUCAGA | SEQ ID NO: 1409 | UGACAAACUGCAGUAAAGGAU | SEQ ID NO: 2351 | CCUUUACUGCAGUUUGUCA | 0.3027 | |
| MA0468 | SEQ ID NO: 468 | CUUUACUGCAGUUUGUCAGAA | SEQ ID NO: 1410 | CUGACAAACUGCAGUAAAGGA | SEQ ID NO: 2352 | CUUUACUGCAGUUUGUCAG | 0.4220 | |
| MA0469 | SEQ ID NO: 469 | UACUGCAGUUUGUCAGAAAGA | SEQ ID NO: 1411 | UUUCUGACAAACUGCAGUAAA | SEQ ID NO: 2353 | UACUGCAGUUUGUCAGAAA | 0.4121 | |
| MA0470 | SEQ ID NO: 470 | ACUGCAGUUUGUCAGAAAGAU | SEQ ID NO: 1412 | CUUUCUGACAAACUGCAGUAA | SEQ ID NO: 2354 | ACUGCAGUUUGUCAGAAAG | 0.4037 | |
| MA0471 | SEQ ID NO: 471 | CUGCAGUUUGUCAGAAAGAUG | SEQ ID NO: 1413 | UCUUUCUGACAAACUGCAGUA | SEQ ID NO: 2355 | CUGCAGUUUGUCAGAAAGA | 0.2964 | |
| MA0472 | SEQ ID NO: 472 | UGCAGUUUGUCAGAAAGAUGG | SEQ ID NO: 1414 | AUCUUUCUGACAAACUGCAGU | SEQ ID NO: 2356 | UGCAGUUUGUCAGAAAGAU | 0.3959 | |
| MA0473 | SEQ ID NO: 473 | CAGUUUGUCAGAAAGAUGGAU | SEQ ID NO: 1415 | CCAUCUUUCUGACAAACUGCA | SEQ ID NO: 2357 | CAGUUUGUCAGAAAGAUGG | 0.4549 | |
| MA0474 | SEQ ID NO: 474 | GUUUGUCAGAAAGAUGGAUCU | SEQ ID NO: 1416 | AUCCAUCUUUCUGACAAACUG | SEQ ID NO: 2358 | GUUUGUCAGAAAGAUGGAU | 0.3267 | |
| MA0475 | SEQ ID NO: 475 | UUGUCAGAAAGAUGGAUCUUG | SEQ ID NO: 1417 | AGAUCCAUCUUUCUGACAAAC | SEQ ID NO: 2359 | UUGUCAGAAAGAUGGAUCU | 0.4131 | |
| MA0476 | SEQ ID NO: 476 | CAGAAAGAUGGAUCUUGGGAC | SEQ ID NO: 1418 | CCCAAGAUCCAUCUUUCUGAC | SEQ ID NO: 2360 | CAGAAAGAUGGAUCUUGGG | 0.5620 | * |
| MA0477 | SEQ ID NO: 477 | AGAAAGAUGGAUCUUGGGACC | SEQ ID NO: 1419 | UCCCAAGAUCCAUCUUUCUGA | SEQ ID NO: 2361 | AGAAAGAUGGAUCUUGGGA | 0.4532 | |
| MA0478 | SEQ ID NO: 478 | GAAAGAUGGAUCUUGGGACCG | SEQ ID NO: 1420 | GUCCCAAGAUCCAUCUUUCUG | SEQ ID NO: 2362 | GAAAGAUGGAUCUUGGGAC | 0.5746 | * |
| MA0479 | SEQ ID NO: 479 | AGAUGGAUCUUGGGACCGGCC | SEQ ID NO: 1421 | CCGGUCCCAAGAUCCAUCUUU | SEQ ID NO: 2363 | AGAUGGAUCUUGGGACCGG | 0.4889 | * |
| MA0480 | SEQ ID NO: 480 | GAUGGAUCUUGGGACCGGCCA | SEQ ID NO: 1422 | GCCGGUCCCAAGAUCCAUCUU | SEQ ID NO: 2364 | GAUGGAUCUUGGGACCGGC | 0.4425 | |
| MA0481 | SEQ ID NO: 481 | CCAAUGCCCGCGUGCAGCAUU | SEQ ID NO: 1423 | UGCUGCACGCGGGCAUUGGCC | SEQ ID NO: 2365 | CCAAUGCCCGCGUGCAGCA | 0.1766 | |
| MA0482 | SEQ ID NO: 482 | CAAUGCCCGCGUGCAGCAUUG | SEQ ID NO: 1424 | AUGCUGCACGCGGGCAUUGGC | SEQ ID NO: 2366 | CAAUGCCCGCGUGCAGCAU | 0.1303 | |
| MA0483 | SEQ ID NO: 483 | AAUGCCCGCGUGCAGCAUUGU | SEQ ID NO: 1425 | AAUGCUGCACGCGGGCAUUGG | SEQ ID NO: 2367 | AAUGCCCGCGUGCAGCAUU | 0.3310 | |
| MA0484 | SEQ ID NO: 484 | CCCGCGUGCAGCAUUGUUGAC | SEQ ID NO: 1426 | CAACAAUGCUGCACGCGGGCA | SEQ ID NO: 2368 | CCCGCGUGCAGCAUUGUUG | 0.4186 | |

**[Table 1-12]**

| double stranded nucleic acid number | SEQ ID NO: | sense strand sequence (5' --->3') | SEQ ID NO: | antisense strand sequence (5' →3') | SEQ ID NO: | target MASP2 mRNA sequence | relative expression level | note |
|---|---|---|---|---|---|---|---|---|
| MA0485 | SEQ ID NO: 485 | CCGCGUGCAGCAUUGUUGACU | SEQ ID NO: 1427 | UCAACAAUGCUGCACGCGGGC | SEQ ID NO: 2369 | CCGCGUGCAGCAUUGUUGA | 0.4219 | |
| MA0486 | SEQ ID NO: 486 | CGUGCAGCAUUGUUGACUGUG | SEQ ID NO: 1428 | CAGUCAACAAUGCUGCACGCG | SEQ ID NO: 2370 | CGUGCAGCAUUGUUGACUG | 0.3188 | |
| MA0487 | SEQ ID NO: 487 | GUGCAGCAUUGUUGACUGUGG | SEQ ID NO: 1429 | ACAGUCAACAAUGCUGCACGC | SEQ ID NO: 2371 | GUGCAGCAUUGUUGACUGU | 0.4787 | |
| MA0488 | SEQ ID NO: 488 | GCAGCAUUGUUGACUGUGGCC | SEQ ID NO: 1430 | CCACAGUCAACAAUGCUGCAC | SEQ ID NO: 2372 | GCAGCAUUGUUGACUGUGG | 0.5918 | * |
| MA0489 | SEQ ID NO: 489 | GCAUUGUUGACUGUGGCCCUC | SEQ ID NO: 1431 | GGGCCACAGUCAACAAUGCUG | SEQ ID NO: 2373 | GCAUUGUUGACUGUGGCCC | 0.5045 | * |
| MA0490 | SEQ ID NO: 490 | CAUUGUUGACUGUGGCCCUCC | SEQ ID NO: 1432 | AGGGCCACAGUCAACAAUGCU | SEQ ID NO: 2374 | CAUUGUUGACUGUGGCCCU | 0.4851 | |
| MA0491 | SEQ ID NO: 491 | AUUGUUGACUGUGGCCCUCCU | SEQ ID NO: 1433 | GAGGGCCACAGUCAACAAUGC | SEQ ID NO: 2375 | AUUGUUGACUGUGGCCCUC | 0.5925 | * |
| MA0492 | SEQ ID NO: 492 | UGACUGUGGCCCUCCUGAUGA | SEQ ID NO: 1434 | AUCAGGAGGGCCACAGUCAAC | SEQ ID NO: 2376 | UGACUGUGGCCCUCCUGAU | 0.3410 | |
| MA0493 | SEQ ID NO: 493 | GACUGUGGCCCUCCUGAUGAU | SEQ ID NO: 1435 | CAUCAGGAGGGCCACAGUCAA | SEQ ID NO: 2377 | GACUGUGGCCCUCCUGAUG | 0.3529 | |
| MA0494 | SEQ ID NO: 494 | ACUGUGGCCCUCCUGAUGAUC | SEQ ID NO: 1436 | UCAUCAGGAGGGCCACAGUCA | SEQ ID NO: 2378 | ACUGUGGCCCUCCUGAUGA | 0.2114 | |
| MA0495 | SEQ ID NO: 495 | CUGUGGCCCUCCUGAUGAUCU | SEQ ID NO: 1437 | AUCAUCAGGAGGGCCACAGUC | SEQ ID NO: 2379 | CUGUGGCCCUCCUGAUGAU | 0.2853 | |
| MA0496 | SEQ ID NO: 496 | UGGCCCUCCUGAUGAUCUACC | SEQ ID NO: 1438 | UAGAUCAUCAGGAGGGCCACA | SEQ ID NO: 2380 | UGGCCCUCCUGAUGAUCUA | 0.3154 | |
| MA0497 | SEQ ID NO: 497 | GCCCUCCUGAUGAUCUACCCA | SEQ ID NO: 1439 | GGUAGAUCAUCAGGAGGGCCA | SEQ ID NO: 2381 | GCCCUCCUGAUGAUCUACC | 0.5766 | * |
| MA0498 | SEQ ID NO: 498 | CCUCCUGAUGAUCUACCCAGU | SEQ ID NO: 1440 | UGGGUAGAUCAUCAGGAGGGC | SEQ ID NO: 2382 | CCUCCUGAUGAUCUACCCA | 0.4269 | |
| MA0499 | SEQ ID NO: 499 | UCCUGAUGAUCUACCCAGUGG | SEQ ID NO: 1441 | ACUGGGUAGAUCAUCAGGAGG | SEQ ID NO: 2383 | UCCUGAUGAUCUACCCAGU | 0.5275 | * |
| MA0500 | SEQ ID NO: 500 | CCUGAUGAUCUACCCAGUGGC | SEQ ID NO: 1442 | CACUGGGUAGAUCAUCAGGAG | SEQ ID NO: 2384 | CCUGAUGAUCUACCCAGUG | 0.5850 | * |
| MA0501 | SEQ ID NO: 501 | CUGAUGAUCUACCCAGUGGCC | SEQ ID NO: 1443 | CCACUGGGUAGAUCAUCAGGA | SEQ ID NO: 2385 | CUGAUGAUCUACCCAGUGG | 0.4049 | |
| MA0502 | SEQ ID NO: 502 | UACCCAGUGGCCGAGUGGAGU | SEQ ID NO: 1444 | UCCACUCGGCCACUGGGUAGA | SEQ ID NO: 2386 | UACCCAGUGGCCGAGUGGA | 0.5202 | |
| MA0503 | SEQ ID NO: 503 | ACCCAGUGGCCGAGUGGAGUA | SEQ ID NO: 1445 | CUCCACUCGGCCACUGGGUAG | SEQ ID NO: 2387 | ACCCAGUGGCCGAGUGGAG | 0.5126 | * |
| MA0504 | SEQ ID NO: 504 | CCCAGUGGCCGAGUGGAGUAC | SEQ ID NO: 1446 | ACUCCACUCGGCCACUGGGUA | SEQ ID NO: 2388 | CCCAGUGGCCGAGUGGAGU | 0.2501 | |
| MA0505 | SEQ ID NO: 505 | CCAGUGGCCGAGUGGAGUACA | SEQ ID NO: 1447 | UACUCCACUCGGCCACUGGGU | SEQ ID NO: 2389 | CCAGUGGCCGAGUGGAGUA | 0.4064 | |
| MA0506 | SEQ ID NO: 506 | GUGGCCGAGUGGAGUACAUCA | SEQ ID NO: 1448 | AUGUACUCCACUCGGCCACUG | SEQ ID NO: 2390 | GUGGCCGAGUGGAGUACAU | 0.2857 | |
| MA0507 | SEQ ID NO: 507 | GGCCGAGUGGAGUACAUCACA | SEQ ID NO: 1449 | UGAUGUACUCCACUCGGCCAC | SEQ ID NO: 2391 | GGCCGAGUGGAGUACAUCA | 0.2613 | |
| MA0508 | SEQ ID NO: 508 | GCCGAGUGGAGUACAUCACAG | SEQ ID NO: 1450 | GUGAUGUACUCCACUCGGCCA | SEQ ID NO: 2392 | GCCGAGUGGAGUACAUCAC | 0.2870 | |
| MA0509 | SEQ ID NO: 509 | CCGAGUGGAGUACAUCACAGG | SEQ ID NO: 1451 | UGUGAUGUACUCCACUCGGCC | SEQ ID NO: 2393 | CCGAGUGGAGUACAUCACA | 0.4004 | |
| MA0510 | SEQ ID NO: 510 | CGAGUGGAGUACAUCACAGGU | SEQ ID NO: 1452 | CUGUGAUGUACUCCACUCGGC | SEQ ID NO: 2394 | CGAGUGGAGUACAUCACAG | 0.4004 | |
| MA0511 | SEQ ID NO: 511 | GAGUGGAGUACAUCACAGGUC | SEQ ID NO: 1453 | CCUGUGAUGUACUCCACUCGG | SEQ ID NO: 2395 | GAGUGGAGUACAUCACAGG | 0.5938 | * |
| MA0512 | SEQ ID NO: 512 | AGUGGAGUACAUCACAGGUCC | SEQ ID NO: 1454 | ACCUGUGAUGUACUCCACUCG | SEQ ID NO: 2396 | AGUGGAGUACAUCACAGGU | 0.4559 | |
| MA0513 | SEQ ID NO: 513 | GUGGAGUACAUCACAGGUCCU | SEQ ID NO: 1455 | GACCUGUGAUGUACUCCACUC | SEQ ID NO: 2397 | GUGGAGUACAUCACAGGUC | 0.5007 | * |
| MA0514 | SEQ ID NO: 514 | GGAGUACAUCACAGGUCCUGG | SEQ ID NO: 1456 | AGGACCUGUGAUGUACUCCAC | SEQ ID NO: 2398 | GGAGUACAUCACAGGUCCU | 0.4028 | |
| MA0515 | SEQ ID NO: 515 | GAGUACAUCACAGGUCCUGGA | SEQ ID NO: 1457 | CAGGACCUGUGAUGUACUCCA | SEQ ID NO: 2399 | GAGUACAUCACAGGUCCUG | 0.3880 | |
| MA0516 | SEQ ID NO: 516 | GUACAUCACAGGUCCUGGAGU | SEQ ID NO: 1458 | UCCAGGACCUGUGAUGUACUC | SEQ ID NO: 2400 | GUACAUCACAGGUCCUGGA | 0.3831 | |
| MA0517 | SEQ ID NO: 517 | CACAGGUCCUGGAGUGACCAC | SEQ ID NO: 1459 | GGUCACUCCAGGACCUGUGAU | SEQ ID NO: 2401 | CACAGGUCCUGGAGUGACC | 0.4413 | * |
| MA0518 | SEQ ID NO: 518 | ACAGGUCCUGGAGUGACCACC | SEQ ID NO: 1460 | UGGUCACUCCAGGACCUGUGA | SEQ ID NO: 2402 | ACAGGUCCUGGAGUGACCA | 0.3835 | |
| MA0519 | SEQ ID NO: 519 | CCUGGAGUGACCACCUACAAA | SEQ ID NO: 1461 | UGUAGGUGGUCACUCCAGGAC | SEQ ID NO: 2403 | CCUGGAGUGACCACCUACA | 0.5596 | * |
| MA0520 | SEQ ID NO: 520 | UGGAGUGACCACCUACAAAGC | SEQ ID NO: 1462 | UUUGUAGGUGGUCACUCCAGG | SEQ ID NO: 2404 | UGGAGUGACCACCUACAAA | 0.4134 | |
| MA0521 | SEQ ID NO: 521 | GGAGUGACCACCUACAAAGCU | SEQ ID NO: 1463 | CUUUGUAGGUGGUCACUCCAG | SEQ ID NO: 2405 | GGAGUGACCACCUACAAAG | 0.3734 | |
| MA0522 | SEQ ID NO: 522 | GAGUGACCACCUACAAAGCUG | SEQ ID NO: 1464 | GCUUUGUAGGUGGUCACUCCA | SEQ ID NO: 2406 | GAGUGACCACCUACAAAGC | 0.5727 | * |
| MA0523 | SEQ ID NO: 523 | AGUGACCACCUACAAAGCUGU | SEQ ID NO: 1465 | AGCUUUGUAGGUGGUCACUCC | SEQ ID NO: 2407 | AGUGACCACCUACAAAGCU | 0.3071 | |
| MA0524 | SEQ ID NO: 524 | UGACCACCUACAAAGCUGUGA | SEQ ID NO: 1466 | ACAGCUUUGUAGGUGGUCACU | SEQ ID NO: 2408 | UGACCACCUACAAAGCUGU | 0.3420 | |
| MA0525 | SEQ ID NO: 525 | CACCUACAAAGCUGUGAUUCA | SEQ ID NO: 1467 | AAUCACAGCUUUGUAGGUGGU | SEQ ID NO: 2409 | CACCUACAAAGCUGUGAUU | 0.5218 | * |
| MA0526 | SEQ ID NO: 526 | CCUACAAAGCUGUGAUUCAGU | SEQ ID NO: 1468 | UGAAUCACAGCUUUGUAGGUG | SEQ ID NO: 2410 | CCUACAAAGCUGUGAUUCA | 0.2914 | |
| MA0527 | SEQ ID NO: 527 | CUACAAAGCUGUGAUUCAGUA | SEQ ID NO: 1469 | CUGAAUCACAGCUUUGUAGGU | SEQ ID NO: 2411 | CUACAAAGCUGUGAUUCAG | 0.5212 | * |
| MA0528 | SEQ ID NO: 528 | ACAAAGCUGUGAUUCAGUACA | SEQ ID NO: 1470 | UACUGAAUCACAGCUUUGUAG | SEQ ID NO: 2412 | ACAAAGCUGUGAUUCAGUA | 0.2584 | |

**[Table 1-13**

| double stranded nucleic acid number | SEQ ID NO: | sense strand sequence (5'--->3') | SEQ ID NO: | antisense strand sequence (5' --->3') | SEQ ID NO: | target MASP2 mRNA sequence | relative expression level | note |
|---|---|---|---|---|---|---|---|---|
| MA0529 | SEQ ID NO: 529 | CAAAGCUGUGAUUCAGUACAG | SEQ ID NO: 1471 | GUACUGAAUCACAGCUUUGUA | SEQ ID NO: 2413 | CAAAGCUGUGAUUCAGUAC | 0.3258 | |
| MA0530 | SEQ ID NO: 530 | AAAGCUGUGAUUCAGUACAGC | SEQ ID NO: 1472 | UGUACUGAAUCACAGCUUUGU | SEQ ID NO: 2414 | AAAGCUGUGAUUCAGUACA | 0.2658 | |
| MA0531 | SEQ ID NO: 531 | AGCUGUGAUUCAGUACAGCUG | SEQ ID NO: 1473 | GCUGUACUGAAUCACAGCUUU | SEQ ID NO: 2415 | AGCUGUGAUUCAGUACAGC | 0.4886 | * |
| MA0532 | SEQ ID NO: 532 | GAUUCAGUACAGCUGUGAAGA | SEQ ID NO: 1474 | UUCACAGCUGUACUGAAUCAC | SEQ ID NO: 2416 | GAUUCAGUACAGCUGUGAA | 0.4636 | |
| MA0533 | SEQ ID NO: 533 | UUCAGUACAGCUGUGAAGAGA | SEQ ID NO: 1475 | UCUUCACAGCUGUACUGAAUC | SEQ ID NO: 2417 | UUCAGUACAGCUGUGAAGA | 0.4362 | |
| MA0534 | SEQ ID NO: 534 | UCAGUACAGCUGUGAAGAGAC | SEQ ID NO: 1476 | CUCUUCACAGCUGUACUGAAU | SEQ ID NO: 2418 | UCAGUACAGCUGUGAAGAG | 0.2338 | |
| MA0535 | SEQ ID NO: 535 | CAGUACAGCUGUGAAGAGACC | SEQ ID NO: 1477 | UCUCUUCACAGCUGUACUGAA | SEQ ID NO: 2419 | CAGUACAGCUGUGAAGAGA | 0.3281 | |
| MA0536 | SEQ ID NO: 536 | GUACAGCUGUGAAGAGACCUU | SEQ ID NO: 1478 | GGUCUCUUCACAGCUGUACUG | SEQ ID NO: 2420 | GUACAGCUGUGAAGAGACC | 0.2368 | |
| MA0537 | SEQ ID NO: 537 | ACAGCUGUGAAGAGACCUUCU | SEQ ID NO: 1479 | AAGGUCUCUUCACAGCUGUAC | SEQ ID NO: 2421 | ACAGCUGUGAAGAGACCUU | 0.3683 | |
| MA0538 | SEQ ID NO: 538 | CAGCUGUGAAGAGACCUUCUA | SEQ ID NO: 1480 | GAAGGUCUCUUCACAGCUGUA | SEQ ID NO: 2422 | CAGCUGUGAAGAGACCUUC | 0.3346 | |
| MA0539 | SEQ ID NO: 539 | AGCUGUGAAGAGACCUUCUAC | SEQ ID NO: 1481 | AGAAGGUCUCUUCACAGCUGU | SEQ ID NO: 2423 | AGCUGUGAAGAGACCUUCU | 0.4532 | * |
| MA0540 | SEQ ID NO: 540 | UGUGAAGAGACCUUCUACACA | SEQ ID NO: 1482 | UGUAGAAGGUCUCUUCACAGC | SEQ ID NO: 2424 | UGUGAAGAGACCUUCUACA | 0.2455 | |
| MA0541 | SEQ ID NO: 541 | GUGAAGAGACCUUCUACACAA | SEQ ID NO: 1483 | GUGUAGAAGGUCUCUUCACAG | SEQ ID NO: 2425 | GUGAAGAGACCUUCUACAC | 0.1206 | |
| MA0542 | SEQ ID NO: 542 | UGAAGAGACCUUCUACACAAU | SEQ ID NO: 1484 | UGUGUAGAAGGUCUCUUCACA | SEQ ID NO: 2426 | UGAAGAGACCUUCUACACA | 0.4039 | |
| MA0543 | SEQ ID NO: 543 | GAAGAGACCUUCUACACAAUG | SEQ ID NO: 1485 | UUGUGUAGAAGGUCUCUUCAC | SEQ ID NO: 2427 | GAAGAGACCUUCUACACAA | 0.0873 | |
| MA0544 | SEQ ID NO: 544 | AAGAGACCUUCUACACAAUGA | SEQ ID NO: 1486 | AUUGUGUAGAAGGUCUCUUCA | SEQ ID NO: 2428 | AAGAGACCUUCUACACAAU | 0.3674 | |
| MA0545 | SEQ ID NO: 545 | AGAGACCUUCUACACAAUGAA | SEQ ID NO: 1487 | CAUUGUGUAGAAGGUCUCUUC | SEQ ID NO: 2429 | AGAGACCUUCUACACAAUG | 0.5259 | |
| MA0546 | SEQ ID NO: 546 | GAGACCUUCUACACAAUGAAA | SEQ ID NO: 1488 | UCAUUGUGUAGAAGGUCUCUU | SEQ ID NO: 2430 | GAGACCUUCUACACAAUGA | 0.4628 | |
| MA0547 | SEQ ID NO: 547 | AGACCUUCUACACAAUGAAAG | SEQ ID NO: 1489 | UUCAUUGUGUAGAAGGUCUCU | SEQ ID NO: 2431 | AGACCUUCUACACAAUGAA | 0.2071 | |
| MA0548 | SEQ ID NO: 548 | GACCUUCUACACAAUGAAAGU | SEQ ID NO: 1490 | UUUCAUUGUGUAGAAGGUCUC | SEQ ID NO: 2432 | GACCUUCUACACAAUGAAA | 0.3355 | |
| MA0549 | SEQ ID NO: 549 | ACCUUCUACACAAUGAAAGUG | SEQ ID NO: 1491 | CUUUCAUUGUGUAGAAGGUCU | SEQ ID NO: 2433 | ACCUUCUACACAAUGAAAG | 0.3961 | |
| MA0550 | SEQ ID NO: 550 | CCUUCUACACAAUGAAAGUGA | SEQ ID NO: 1492 | ACUUUCAUUGUGUAGAAGGUC | SEQ ID NO: 2434 | CCUUCUACACAAUGAAAGU | 0.2661 | |
| MA0551 | SEQ ID NO: 551 | CUUCUACACAAUGAAAGUGAA | SEQ ID NO: 1493 | CACUUUCAUUGUGUAGAAGGU | SEQ ID NO: 2435 | CUUCUACACAAUGAAAGUG | 0.4225 | |
| MA0552 | SEQ ID NO: 552 | UUCUACACAAUGAAAGUGAAU | SEQ ID NO: 1494 | UCACUUUCAUUGUGUAGAAGG | SEQ ID NO: 2436 | UUCUACACAAUGAAAGUGA | 0.4160 | |
| MA0553 | SEQ ID NO: 553 | UCUACACAAUGAAAGUGAAUG | SEQ ID NO: 1495 | UUCACUUUCAUUGUGUAGAAG | SEQ ID NO: 2437 | UCUACACAAUGAAAGUGAA | 0.2817 | |
| MA0554 | SEQ ID NO: 554 | CUACACAAUGAAAGUGAAUGA | SEQ ID NO: 1496 | AUUCACUUUCAUUGUGUAGAA | SEQ ID NO: 2438 | CUACACAAUGAAAGUGAAU | 0.5685 | * |
| MA0555 | SEQ ID NO: 555 | ACACAAUGAAAGUGAAUGAUG | SEQ ID NO: 1497 | UCAUUCACUUUCAUUGUGUAG | SEQ ID NO: 2439 | ACACAAUGAAAGUGAAUGA | 0.1776 | |
| MA0556 | SEQ ID NO: 556 | CACAAUGAAAGUGAAUGAUGG | SEQ ID NO: 1498 | AUCAUUCACUUUCAUUGUGUA | SEQ ID NO: 2440 | CACAAUGAAAGUGAAUGAU | 0.3592 | |
| MA0557 | SEQ ID NO: 557 | CAAUGAAAGUGAAUGAUGGUA | SEQ ID NO: 1499 | CCAUCAUUCACUUUCAUUGUG | SEQ ID NO: 2441 | CAAUGAAAGUGAAUGAUGG | 0.1996 | |
| MA0558 | SEQ ID NO: 558 | AAUGAAAGUGAAUGAUGGUAA | SEQ ID NO: 1500 | ACCAUCAUUCACUUUCAUUGU | SEQ ID NO: 2442 | AAUGAAAGUGAAUGAUGGU | 0.3948 | |
| MA0559 | SEQ ID NO: 559 | AUGAAAGUGAAUGAUGGUAAA | SEQ ID NO: 1501 | UACCAUCAUUCACUUUCAUUG | SEQ ID NO: 2443 | AUGAAAGUGAAUGAUGGUA | 0.4001 | |
| MA0560 | SEQ ID NO: 560 | GAAAGUGAAUGAUGGUAAAUA | SEQ ID NO: 1502 | UUUACCAUCAUUCACUUUCAU | SEQ ID NO: 2444 | GAAAGUGAAUGAUGGUAAA | 0.2653 | |
| MA0561 | SEQ ID NO: 561 | AAGUGAAUGAUGGUAAAUAUG | SEQ ID NO: 1503 | UAUUUACCAUCAUUCACUUUC | SEQ ID NO: 2445 | AAGUGAAUGAUGGUAAAUA | 0.4991 | |
| MA0562 | SEQ ID NO: 562 | AGUGAAUGAUGGUAAAUAUGU | SEQ ID NO: 1504 | AUAUUUACCAUCAUUCACUUU | SEQ ID NO: 2446 | AGUGAAUGAUGGUAAAUAU | 0.2388 | |
| MA0563 | SEQ ID NO: 563 | GUGAAUGAUGGUAAAUAUGUG | SEQ ID NO: 1505 | CAUAUUUACCAUCAUUCACUU | SEQ ID NO: 2447 | GUGAAUGAUGGUAAAUAUG | 0.3418 | |
| MA0564 | SEQ ID NO: 564 | UGAAUGAUGGUAAAUAUGUGU | SEQ ID NO: 1506 | ACAUAUUUACCAUCAUUCACU | SEQ ID NO: 2448 | UGAAUGAUGGUAAAUAUGU | 0.5088 | * |
| MA0565 | SEQ ID NO: 565 | GAAUGAUGGUAAAUAUGUGUG | SEQ ID NO: 1507 | CACAUAUUUACCAUCAUUCAC | SEQ ID NO: 2449 | GAAUGAUGGUAAAUAUGUG | 0.2323 | |
| MA0566 | SEQ ID NO: 566 | GGUAAAUAUGUGUGUGAGGCU | SEQ ID NO: 1508 | CCUCACACACAUAUUUACCAU | SEQ ID NO: 2450 | GGUAAAUAUGUGUGUGAGG | 0.4557 | |
| MA0567 | SEQ ID NO: 567 | AUAUGUGUGUGAGGCUGAUGG | SEQ ID NO: 1509 | AUCAGCCUCACACACAUAUUU | SEQ ID NO: 2451 | AUAUGUGUGUGAGGCUGAU | 0.3283 | |
| MA0568 | SEQ ID NO: 568 | UGUGUGUGAGGCUGAUGGAUU | SEQ ID NO: 1510 | UCCAUCAGCCUCACACACAUA | SEQ ID NO: 2452 | UGUGUGUGAGGCUGAUGGA | 0.3241 | |
| MA0569 | SEQ ID NO: 569 | GUGUGUGAGGCUGAUGGAUUC | SEQ ID NO: 1511 | AUCCAUCAGCCUCACACACAU | SEQ ID NO: 2453 | GUGUGUGAGGCUGAUGGAU | 0.1097 | |
| MA0570 | SEQ ID NO: 570 | UGUGUGAGGCUGAUGGAUUCU | SEQ ID NO: 1512 | AAUCCAUCAGCCUCACACACA | SEQ ID NO: 2454 | UGUGUGAGGCUGAUGGAUU | 0.2347 | |
| MA0571 | SEQ ID NO: 571 | GUGUGAGGCUGAUGGAUUCUG | SEQ ID NO: 1513 | GAAUCCAUCAGCCUCACACAC | SEQ ID NO: 2455 | GUGUGAGGCUGAUGGAUUC | 0.2909 | |
| MA0572 | SEQ ID NO: 572 | UGUGAGGCUGAUGGAUUCUGG | SEQ ID NO: 1514 | AGAAUCCAUCAGCCUCACACA | SEQ ID NO: 2456 | UGUGAGGCUGAUGGAUUCU | 0.3250 | |

**[Table 1-14]**

| double stranded nucleic acid number | SEQ ID NO: | sense strand sequence (5'--->3') | SEQ ID NO: | antisense strand sequence (5'--->3') | SEQ ID NO : | target MASP2 mRNA sequence | relative expression level | note |
|---|---|---|---|---|---|---|---|---|
| MA0573 | SEQ ID NO: 573 | GUGAGGCUGAUGGAUUCUGGA | SEQ ID NO: 1515 | CAGAAUCCAUCAGCCUCACAC | SEQ ID NO: 2457 | GUGAGGCUGAUGGAUUCUG | 0.4694 | |
| MA0574 | SEQ ID NO: 574 | GAGGCUGAUGGAUUCUGGACG | SEQ ID NO: 1516 | UCCAGAAUCCAUCAGCCUCAC | SEQ ID NO: 2458 | GAGGCUGAUGGAUUCUGGA | 0.3183 | |
| MA0575 | SEQ ID NO: 575 | GGCUGAUGGAUUCUGGACGAG | SEQ ID NO: 1517 | CGUCCAGAAUCCAUCAGCCUC | SEQ ID NO: 2459 | GGCUGAUGGAUUCUGGACG | 0.4488 | |
| MA0576 | SEQ ID NO: 576 | GCUGAUGGAUUCUGGACGAGC | SEQ ID NO: 1518 | UCGUCCAGAAUCCAUCAGCCU | SEQ ID NO: 2460 | GCUGAUGGAUUCUGGACGA | 0.2778 | |
| MA0577 | SEQ ID NO: 577 | GAUGGAUUCUGGACGAGCUCC | SEQ ID NO: 1519 | AGCUCGUCCAGAAUCCAUCAG | SEQ ID NO: 2461 | GAUGGAUUCUGGACGAGCU | 0.2956 | |
| MA0578 | SEQ ID NO: 578 | GAUUCUGGACGAGCUCCAAAG | SEQ ID NO: 1520 | UUGGAGCUCGUCCAGAAUCCA | SEQ ID NO: 2462 | GAUUCUGGACGAGCUCCAA | 0.3395 | |
| MA0579 | SEQ ID NO: 579 | AUUCUGGACGAGCUCCAAAGG | SEQ ID NO: 1521 | UUUGGAGCUCGUCCAGAAUCC | SEQ ID NO: 2463 | AUUCUGGACGAGCUCCAAA | 0.3768 | |
| MA0580 | SEQ ID NO: 580 | CUGGACGAGCUCCAAAGGAGA | SEQ ID NO: 1522 | UCCUUUGGAGCUCGUCCAGAA | SEQ ID NO: 2464 | CUGGACGAGCUCCAAAGGA | 0.2355 | |
| MA0581 | SEQ ID NO: 581 | UGGACGAGCUCCAAAGGAGAA | SEQ ID NO: 1523 | CUCCUUUGGAGCUCGUCCAGA | SEQ ID NO: 2465 | UGGACGAGCUCCAAAGGAG | 0.2817 | |
| MA0582 | SEQ ID NO: 582 | GGACGAGCUCCAAAGGAGAAA | SEQ ID NO: 1524 | UCUCCUUUGGAGCUCGUCCAG | SEQ ID NO: 2466 | GGACGAGCUCCAAAGGAGA | 0.1939 | |
| MA0583 | SEQ ID NO: 583 | GACGAGCUCCAAAGGAGAAAA | SEQ ID NO: 1525 | UUCUCCUUUGGAGCUCGUCCA | SEQ ID NO: 2467 | GACGAGCUCCAAAGGAGAA | 0.4187 | |
| MA0584 | SEQ ID NO: 584 | CGAGCUCCAAAGGAGAAAAAU | SEQ ID NO: 1526 | UUUUCUCCUUUGGAGCUCGUC | SEQ ID NO: 2468 | CGAGCUCCAAAGGAGAAAA | 0.4469 | |
| MA0585 | SEQ ID NO: 585 | GAGCUCCAAAGGAGAAAAAUC | SEQ ID NO: 1527 | UUUUUCUCCUUUGGAGCUCGU | SEQ ID NO: 2469 | GAGCUCCAAAGGAGAAAAA | 0.3593 | |
| MA0586 | SEQ ID NO: 586 | AGCUCCAAAGGAGAAAAAUCA | SEQ ID NO: 1528 | AUUUUUCUCCUUUGGAGCUCG | SEQ ID NO: 2470 | AGCUCCAAAGGAGAAAAAU | 0.4459 | |
| MA0587 | SEQ ID NO: 587 | GCUCCAAAGGAGAAAAAUCAC | SEQ ID NO: 1529 | GAUUUUUCUCCUUUGGAGCUC | SEQ ID NO: 2471 | GCUCCAAAGGAGAAAAAUC | 0.4857 | * |
| MA0588 | SEQ ID NO: 588 | CUCCAAAGGAGAAAAAUCACU | SEQ ID NO: 1530 | UGAUUUUUCUCCUUUGGAGCU | SEQ ID NO: 2472 | CUCCAAAGGAGAAAAAUCA | 0.1871 | |
| MA0589 | SEQ ID NO: 589 | CCAAAGGAGAAAAAUCACUCC | SEQ ID NO: 1531 | AGUGAUUUUUCUCCUUUGGAG | SEQ ID NO: 2473 | CCAAAGGAGAAAAAUCACU | 0.3475 | |
| MA0590 | SEQ ID NO: 590 | AGGAGAAAAAUCACUCCCAGU | SEQ ID NO: 1532 | UGGGAGUGAUUUUUCUCCUUU | SEQ ID NO: 2474 | AGGAGAAAAAUCACUCCCA | 0.4639 | |
| MA0591 | SEQ ID NO: 591 | GGAGAAAAAUCACUCCCAGUC | SEQ ID NO: 1533 | CUGGGAGUGAUUUUUCUCCUU | SEQ ID NO: 2475 | GGAGAAAAAUCACUCCCAG | 0.4155 | |
| MA0592 | SEQ ID NO: 592 | AGAAAAAUCACUCCCAGUCUG | SEQ ID NO: 1534 | GACUGGGAGUGAUUUUUCUCC | SEQ ID NO: 2476 | AGAAAAAUCACUCCCAGUC | 0.3251 | |
| MA0593 | SEQ ID NO: 593 | GAAAAAUCACUCCCAGUCUGU | SEQ ID NO: 1535 | AGACUGGGAGUGAUUUUUCUC | SEQ ID NO: 2477 | GAAAAAUCACUCCCAGUCU | 0.3061 | |
| MA0594 | SEQ ID NO: 594 | AAAAAUCACUCCCAGUCUGUG | SEQ ID NO: 1536 | CAGACUGGGAGUGAUUUUUCU | SEQ ID NO: 2478 | AAAAAUCACUCCCAGUCUG | 0.5931 | * |
| MA0595 | SEQ ID NO: 595 | AAAAUCACUCCCAGUCUGUGA | SEQ ID NO: 1537 | ACAGACUGGGAGUGAUUUUUC | SEQ ID NO: 2479 | AAAAUCACUCCCAGUCUGU | 0.5196 | * |
| MA0596 | SEQ ID NO: 596 | CUCCCAGUCUGUGAGCCUGUU | SEQ ID NO: 1538 | CAGGCUCACAGACUGGGAGUG | SEQ ID NO: 2480 | CUCCCAGUCUGUGAGCCUG | 0.4157 | |
| MA0597 | SEQ ID NO: 597 | UCCCAGUCUGUGAGCCUGUUU | SEQ ID NO: 1539 | ACAGGCUCACAGACUGGGAGU | SEQ ID NO: 2481 | UCCCAGUCUGUGAGCCUGU | 0.4504 | |
| MA0598 | SEQ ID NO: 598 | CCCAGUCUGUGAGCCUGUUUG | SEQ ID NO: 1540 | AACAGGCUCACAGACUGGGAG | SEQ ID NO: 2482 | CCCAGUCUGUGAGCCUGUU | 0.4836 | * |
| MA0599 | SEQ ID NO: 599 | CAGUCUGUGAGCCUGUUUGUG | SEQ ID NO: 1541 | CAAACAGGCUCACAGACUGGG | SEQ ID NO: 2483 | CAGUCUGUGAGCCUGUUUG | 0.4572 | |
| MA0600 | SEQ ID NO: 600 | CUGUGAGCCUGUUUGUGGACU | SEQ ID NO: 1542 | UCCACAAACAGGCUCACAGAC | SEQ ID NO: 2484 | CUGUGAGCCUGUUUGUGGA | 0.3947 | |
| MA0601 | SEQ ID NO: 601 | UGAGCCUGUUUGUGGACUAUC | SEQ ID NO: 1543 | UAGUCCACAAACAGGCUCACA | SEQ ID NO: 2485 | UGAGCCUGUUUGUGGACUA | 0.5071 | * |
| MA0602 | SEQ ID NO: 602 | GCCUGUUUGUGGACUAUCAGC | SEQ ID NO: 1544 | UGAUAGUCCACAAACAGGCUC | SEQ ID NO: 2486 | GCCUGUUUGUGGACUAUCA | 0.4543 | |
| MA0603 | SEQ ID NO: 603 | CCUGUUUGUGGACUAUCAGCC | SEQ ID NO: 1545 | CUGAUAGUCCACAAACAGGCU | SEQ ID NO: 2487 | CCUGUUUGUGGACUAUCAG | 0.5234 | * |
| MA0604 | SEQ ID NO: 604 | CUGUUUGUGGACUAUCAGCCC | SEQ ID NO: 1546 | GCUGAUAGUCCACAAACAGGC | SEQ ID NO: 2488 | CUGUUUGUGGACUAUCAGC | 0.4479 | * |
| MA0605 | SEQ ID NO: 605 | UGGACUAUCAGCCCGCACAAC | SEQ ID NO: 1547 | UGUGCGGGCUGAUAGUCCACA | SEQ ID NO: 2489 | UGGACUAUCAGCCCGCACA | 0.4976 | * |
| MA0606 | SEQ ID NO: 606 | ACUAUCAGCCCGCACAACAGG | SEQ ID NO: 1548 | UGUUGUGCGGGCUGAUAGUCC | SEQ ID NO: 2490 | ACUAUCAGCCCGCACAACA | 0.4484 | |
| MA0607 | SEQ ID NO: 607 | CUAUCAGCCCGCACAACAGGA | SEQ ID NO: 1549 | CUGUUGUGCGGGCUGAUAGUC | SEQ ID NO: 2491 | CUAUCAGCCCGCACAACAG | 0.2952 | |
| MA0608 | SEQ ID NO: 608 | CCGCACAACAGGAGGGCGUAU | SEQ ID NO: 1550 | ACGCCCUCCUGUUGUGCGGGC | SEQ ID NO: 2492 | CCGCACAACAGGAGGGCGU | 0.3233 | |
| MA0609 | SEQ ID NO: 609 | CGCACAACAGGAGGGCGUAUA | SEQ ID NO: 1551 | UACGCCCUCCUGUUGUGCGGG | SEQ ID NO: 2493 | CGCACAACAGGAGGGCGUA | 0.2263 | |
| MA0610 | SEQ ID NO: 610 | GCACAACAGGAGGGCGUAUAU | SEQ ID NO: 1552 | AUACGCCCUCCUGUUGUGCGG | SEQ ID NO: 2494 | GCACAACAGGAGGGCGUAU | 0.4521 | |
| MA0611 | SEQ ID NO: 611 | CACAACAGGAGGGCGUAUAUA | SEQ ID NO: 1553 | UAUACGCCCUCCUGUUGUGCG | SEQ ID NO: 2495 | CACAACAGGAGGGCGUAUA | 0.1227 | |
| MA0612 | SEQ ID NO: 612 | ACAACAGGAGGGCGUAUAUAU | SEQ ID NO: 1554 | AUAUACGCCCUCCUGUUGUGC | SEQ ID NO: 2496 | ACAACAGGAGGGCGUAUAU | 0.0915 | |
| MA0613 | SEQ ID NO: 613 | CAACAGGAGGGCGUAUAUAUG | SEQ ID NO: 1555 | UAUAUACGCCCUCCUGUUGUG | SEQ ID NO: 2497 | CAACAGGAGGGCGUAUAUA | 0.3367 | |
| MA0614 | SEQ ID NO: 614 | AACAGGAGGGCGUAUAUAUGG | SEQ ID NO: 1556 | AUAUAUACGCCCUCCUGUUGU | SEQ ID NO: 2498 | AACAGGAGGGCGUAUAUAU | 0.4134 | |
| MA0615 | SEQ ID NO: 615 | GCGUAUAUAUGGAGGGCAAAA | SEQ ID NO: 1557 | UUGCCCUCCAUAUAUACGCCC | SEQ ID NO: 2499 | GCGUAUAUAUGGAGGGCAA | 0.3278 | |
| MA0616 | SEQ ID NO: 616 | CGUAUAUAUGGAGGGCAAAAG | SEQ ID NO: 1558 | UUUGCCCUCCAUAUAUACGCC | SEQ ID NO: 2500 | CGUAUAUAUGGAGGGCAAA | 0.4800 | |

**[Table 1-15]**

| double stranded nucleic acid number | SEQ ID NO: | sense strand sequence (5'--->3') | SEQ ID NO: | antisense strand sequence (5'--->3') | SEQ ID NO: | target MASP2 mRNA sequence | relative expression level | note |
|---|---|---|---|---|---|---|---|---|
| MA0617 | SEQ ID NO: 617 | AUAUGGAGGGCAAAAGGCAAA | SEQ ID NO: 1559 | UGCCUUUUGCCCUCCAUAUAU | SEQ ID NO: 2501 | AUAUGGAGGGCAAAAGGCA | 0.4880 | * |
| MA0618 | SEQ ID NO: 618 | UAUGGAGGGCAAAAGGCAAAA | SEQ ID NO: 1560 | UUGCCUUUUGCCCUCCAUAUA | SEQ ID NO: 2502 | UAUGGAGGGCAAAAGGCAA | 0.3547 | |
| MA0619 | SEQ ID NO: 619 | GGAGGGCAAAAGGCAAAACCU | SEQ ID NO: 1561 | GUUUUGCCUUUUGCCCUCCAU | SEQ ID NO: 2503 | GGAGGGCAAAAGGCAAAAC | 0.4989 | |
| MA0620 | SEQ ID NO: 620 | AGGGCAAAAGGCAAAACCUGG | SEQ ID NO: 1562 | AGGUUUUGCCUUUUGCCCUCC | SEQ ID NO: 2504 | AGGGCAAAAGGCAAAACCU | 0.4328 | |
| MA0621 | SEQ ID NO: 621 | GGGCAAAAGGCAAAACCUGGU | SEQ ID NO: 1563 | CAGGUUUUGCCUUUUGCCCUC | SEQ ID NO: 2505 | GGGCAAAAGGCAAAACCUG | 0.2519 | |
| MA0622 | SEQ ID NO: 622 | GGCAAAAGGCAAAACCUGGUG | SEQ ID NO: 1564 | CCAGGUUUUGCCUUUUGCCCU | SEQ ID NO: 2506 | GGCAAAAGGCAAAACCUGG | 0.1468 | |
| MA0623 | SEQ ID NO: 623 | GCAAAAGGCAAAACCUGGUGA | SEQ ID NO: 1565 | ACCAGGUUUUGCCUUUUGCCC | SEQ ID NO: 2507 | GCAAAAGGCAAAACCUGGU | 0.2368 | |
| MA0624 | SEQ ID NO: 624 | AAAGGCAAAACCUGGUGAUUU | SEQ ID NO: 1566 | AUCACCAGGUUUUGCCUUUUG | SEQ ID NO: 2508 | AAAGGCAAAACCUGGUGAU | 0.4016 | |
| MA0625 | SEQ ID NO: 625 | AAGGCAAAACCUGGUGAUUUU | SEQ ID NO: 1567 | AAUCACCAGGUUUUGCCUUUU | SEQ ID NO: 2509 | AAGGCAAAACCUGGUGAUU | 0.3840 | |
| MA0626 | SEQ ID NO: 626 | AGGCAAAACCUGGUGAUUUUC | SEQ ID NO: 1568 | AAAUCACCAGGUUUUGCCUUU | SEQ ID NO: 2510 | AGGCAAAACCUGGUGAUUU | 0.3041 | |
| MA0627 | SEQ ID NO: 627 | GGCAAAACCUGGUGAUUUUCC | SEQ ID NO: 1569 | AAAAUCACCAGGUUUUGCCUU | SEQ ID NO: 2511 | GGCAAAACCUGGUGAUUUU | 0.2229 | |
| MA0628 | SEQ ID NO: 628 | GCAAAACCUGGUGAUUUUCCU | SEQ ID NO: 1570 | GAAAAUCACCAGGUUUUGCCU | SEQ ID NO: 2512 | GCAAAACCUGGUGAUUUUC | 0.3537 | |
| MA0629 | SEQ ID NO: 629 | AAAACCUGGUGAUUUUCCUUG | SEQ ID NO: 1571 | AGGAAAAUCACCAGGUUUUGC | SEQ ID NO: 2513 | AAAACCUGGUGAUUUUCCU | 0.1861 | |
| MA0630 | SEQ ID NO: 630 | AAACCUGGUGAUUUUCCUUGG | SEQ ID NO: 1572 | AAGGAAAAUCACCAGGUUUUG | SEQ ID NO: 2514 | AAACCUGGUGAUUUUCCUU | 0.2314 | |
| MA0631 | SEQ ID NO: 631 | ACCUGGUGAUUUUCCUUGGCA | SEQ ID NO: 1573 | CCAAGGAAAAUCACCAGGUUU | SEQ ID NO: 2515 | ACCUGGUGAUUUUCCUUGG | 0.5703 | * |
| MA0632 | SEQ ID NO: 632 | CCUGGUGAUUUUCCUUGGCAA | SEQ ID NO: 1574 | GCCAAGGAAAAUCACCAGGUU | SEQ ID NO: 2516 | CCUGGUGAUUUUCCUUGGC | 0.1702 | |
| MA0633 | SEQ ID NO: 633 | CUGGUGAUUUUCCUUGGCAAG | SEQ ID NO: 1575 | UGCCAAGGAAAAUCACCAGGU | SEQ ID NO: 2517 | CUGGUGAUUUUCCUUGGCA | 0.1609 | |
| MA0634 | SEQ ID NO: 634 | UGGUGAUUUUCCUUGGCAAGU | SEQ ID NO: 1576 | UUGCCAAGGAAAAUCACCAGG | SEQ ID NO: 2518 | UGGUGAUUUUCCUUGGCAA | 0.1500 | |
| MA0635 | SEQ ID NO: 635 | GGUGAUUUUCCUUGGCAAGUC | SEQ ID NO: 1577 | CUUGCCAAGGAAAAUCACCAG | SEQ ID NO: 2519 | GGUGAUUUUCCUUGGCAAG | 0.2815 | |
| MA0636 | SEQ ID NO: 636 | GUGAUUUUCCUUGGCAAGUCC | SEQ ID NO: 1578 | ACUUGCCAAGGAAAAUCACCA | SEQ ID NO: 2520 | GUGAUUUUCCUUGGCAAGU | 0.3443 | |
| MA0637 | SEQ ID NO: 637 | UGAUUUUCCUUGGCAAGUCCU | SEQ ID NO: 1579 | GACUUGCCAAGGAAAAUCACC | SEQ ID NO: 2521 | UGAUUUUCCUUGGCAAGUC | 0.4070 | |
| MA0638 | SEQ ID NO: 638 | GAUUUUCCUUGGCAAGUCCUG | SEQ ID NO: 1580 | GGACUUGCCAAGGAAAAUCAC | SEQ ID NO: 2522 | GAUUUUCCUUGGCAAGUCC | 0.3956 | |
| MA0639 | SEQ ID NO: 639 | UCCUUGGCAAGUCCUGAUAUU | SEQ ID NO: 1581 | UAUCAGGACUUGCCAAGGAAA | SEQ ID NO: 2523 | UCCUUGGCAAGUCCUGAUA | 0.3508 | |
| MA0640 | SEQ ID NO: 640 | CCUUGGCAAGUCCUGAUAUUA | SEQ ID NO: 1582 | AUAUCAGGACUUGCCAAGGAA | SEQ ID NO: 2524 | CCUUGGCAAGUCCUGAUAU | 0.2376 | |
| MA0641 | SEQ ID NO: 641 | CUUGGCAAGUCCUGAUAUUAG | SEQ ID NO: 1583 | AAUAUCAGGACUUGCCAAGGA | SEQ ID NO: 2525 | CUUGGCAAGUCCUGAUAUU | 0.3199 | |
| MA0642 | SEQ ID NO: 642 | UUGGCAAGUCCUGAUAUUAGG | SEQ ID NO: 1584 | UAAUAUCAGGACUUGCCAAGG | SEQ ID NO: 2526 | UUGGCAAGUCCUGAUAUUA | 0.4101 | |
| MA0643 | SEQ ID NO: 643 | GGCAAGUCCUGAUAUUAGGUG | SEQ ID NO: 1585 | CCUAAUAUCAGGACUUGCCAA | SEQ ID NO: 2527 | GGCAAGUCCUGAUAUUAGG | 0.1383 | |
| MA0644 | SEQ ID NO: 644 | GCAAGUCCUGAUAUUAGGUGG | SEQ ID NO: 1586 | ACCUAAUAUCAGGACUUGCCA | SEQ ID NO: 2528 | GCAAGUCCUGAUAUUAGGU | 0.2269 | |
| MA0645 | SEQ ID NO: 645 | AGUCCUGAUAUUAGGUGGAAC | SEQ ID NO: 1587 | UCCACCUAAUAUCAGGACUUG | SEQ ID NO: 2529 | AGUCCUGAUAUUAGGUGGA | 0.5441 | |
| MA0646 | SEQ ID NO: 646 | GUCCUGAUAUUAGGUGGAACC | SEQ ID NO: 1588 | UUCCACCUAAUAUCAGGACUU | SEQ ID NO: 2530 | GUCCUGAUAUUAGGUGGAA | 0.4556 | |
| MA0647 | SEQ ID NO: 647 | UCCUGAUAUUAGGUGGAACCA | SEQ ID NO: 1589 | GUUCCACCUAAUAUCAGGACU | SEQ ID NO: 2531 | UCCUGAUAUUAGGUGGAAC | 0.5311 | * |
| MA0648 | SEQ ID NO: 648 | CCUGAUAUUAGGUGGAACCAC | SEQ ID NO: 1590 | GGUUCCACCUAAUAUCAGGAC | SEQ ID NO: 2532 | CCUGAUAUUAGGUGGAACC | 0.3381 | |
| MA0649 | SEQ ID NO: 649 | GAUAUUAGGUGGAACCACAGC | SEQ ID NO: 1591 | UGUGGUUCCACCUAAUAUCAG | SEQ ID NO: 2533 | GAUAUUAGGUGGAACCACA | 0.1224 | |
| MA0650 | SEQ ID NO: 650 | AUUAGGUGGAACCACAGCAGC | SEQ ID NO: 1592 | UGCUGUGGUUCCACCUAAUAU | SEQ ID NO: 2534 | AUUAGGUGGAACCACAGCA | 0.5344 | * |
| MA0651 | SEQ ID NO: 651 | UUAGGUGGAACCACAGCAGCA | SEQ ID NO: 1593 | CUGCUGUGGUUCCACCUAAUA | SEQ ID NO: 2535 | UUAGGUGGAACCACAGCAG | 0.5391 | |
| MA0652 | SEQ ID NO: 652 | AGGUGGAACCACAGCAGCAGG | SEQ ID NO: 1594 | UGCUGCUGUGGUUCCACCUAA | SEQ ID NO: 2536 | AGGUGGAACCACAGCAGCA | 0.1599 | |
| MA0653 | SEQ ID NO: 653 | GGUGGAACCACAGCAGCAGGU | SEQ ID NO: 1595 | CUGCUGCUGUGGUUCCACCUA | SEQ ID NO: 2537 | GGUGGAACCACAGCAGCAG | 0.0858 | |
| MA0654 | SEQ ID NO: 654 | GUGGAACCACAGCAGCAGGUG | SEQ ID NO: 1596 | CCUGCUGCUGUGGUUCCACCU | SEQ ID NO: 2538 | GUGGAACCACAGCAGCAGG | 0.5782 | * |
| MA0655 | SEQ ID NO: 655 | GAACCACAGCAGCAGGUGCAC | SEQ ID NO: 1597 | GCACCUGCUGCUGUGGUUCCA | SEQ ID NO: 2539 | GAACCACAGCAGCAGGUGC | 0.3427 | |
| MA0656 | SEQ ID NO: 656 | AACCACAGCAGCAGGUGCACU | SEQ ID NO: 1598 | UGCACCUGCUGCUGUGGUUCC | SEQ ID NO: 2540 | AACCACAGCAGCAGGUGCA | 0.3562 | |
| MA0657 | SEQ ID NO: 657 | ACCACAGCAGCAGGUGCACUU | SEQ ID NO: 1599 | GUGCACCUGCUGCUGUGGUUC | SEQ ID NO: 2541 | ACCACAGCAGCAGGUGCAC | 0.5252 | |
| MA0658 | SEQ ID NO: 658 | CCACAGCAGCAGGUGCACUUU | SEQ ID NO: 1600 | AGUGCACCUGCUGCUGUGGUU | SEQ ID NO: 2542 | CCACAGCAGCAGGUGCACU | 0.4436 | |
| MA0659 | SEQ ID NO: 659 | CACAGCAGCAGGUGCACUUUU | SEQ ID NO: 1601 | AAGUGCACCUGCUGCUGUGGU | SEQ ID NO: 2543 | CACAGCAGCAGGUGCACUU | 0.4283 | |
| MA0660 | SEQ ID NO: 660 | ACAGCAGCAGGUGCACUUUUA | SEQ ID NO: 1602 | AAAGUGCACCUGCUGCUGUGG | SEQ ID NO: 2544 | ACAGCAGCAGGUGCACUUU | 0.1498 | |

**[Table 1-16]**

| double stranded nucleic acid number | SEQ ID NO: | sense strand sequence (5'--->3') | SEQ ID NO: | antisense strand sequence (5'--->3') | SEQ ID NO: | target MASP2 mRNA sequence | relative expression level | note |
|---|---|---|---|---|---|---|---|---|
| MA0661 | SEQ ID NO: 661 | CAGCAGCAGGUGCACUUUUAU | SEQ ID NO: 1603 | AAAAGUGCACCUGCUGCUGUG | SEQ ID NO: 2545 | CAGCAGCAGGUGCACUUUU | 0.2579 | |
| MA0662 | SEQ ID NO: 662 | AGCAGCAGGUGCACUUUUAUA | SEQ ID NO: 1604 | UAAAAGUGCACCUGCUGCUGU | SEQ ID NO: 2546 | AGCAGCAGGUGCACUUUUA | 0.3752 | |
| MA0663 | SEQ ID NO: 663 | CAGCAGGUGCACUUUUAUAUG | SEQ ID NO: 1605 | UAUAAAAGUGCACCUGCUGCU | SEQ ID NO: 2547 | CAGCAGGUGCACUUUUAUA | 0.4298 | |
| MA0664 | SEQ ID NO: 664 | AGCAGGUGCACUUUUAUAUGA | SEQ ID NO: 1606 | AUAUAAAAGUGCACCUGCUGC | SEQ ID NO: 2548 | AGCAGGUGCACUUUUAUAU | 0.3065 | |
| MA0665 | SEQ ID NO: 665 | GCAGGUGCACUUUUAUAUGAC | SEQ ID NO: 1607 | CAUAUAAAAGUGCACCUGCUG | SEQ ID NO: 2549 | GCAGGUGCACUUUUAUAUG | 0.4590 | |
| MA0666 | SEQ ID NO: 666 | CAGGUGCACUUUUAUAUGACA | SEQ ID NO: 1608 | UCAUAUAAAAGUGCACCUGCU | SEQ ID NO: 2550 | CAGGUGCACUUUUAUAUGA | 0.0834 | |
| MA0667 | SEQ ID NO: 667 | AGGUGCACUUUUAUAUGACAA | SEQ ID NO: 1609 | GUCAUAUAAAAGUGCACCUGC | SEQ ID NO: 2551 | AGGUGCACUUUUAUAUGAC | 0.2991 | |
| MA0668 | SEQ ID NO: 668 | GGUGCACUUUUAUAUGACAAC | SEQ ID NO: 1610 | UGUCAUAUAAAAGUGCACCUG | SEQ ID NO: 2552 | GGUGCACUUUUAUAUGACA | 0.0999 | |
| MA0669 | SEQ ID NO: 669 | GUGCACUUUUAUAUGACAACU | SEQ ID NO: 1611 | UUGUCAUAUAAAAGUGCACCU | SEQ ID NO: 2553 | GUGCACUUUUAUAUGACAA | 0.2829 | |
| MA0670 | SEQ ID NO: 670 | GCACUUUUAUAUGACAACUGG | SEQ ID NO: 1612 | AGUUGUCAUAUAAAAGUGCAC | SEQ ID NO: 2554 | GCACUUUUAUAUGACAACU | 0.3893 | |
| MA0671 | SEQ ID NO: 671 | CACUUUUAUAUGACAACUGGG | SEQ ID NO: 1613 | CAGUUGUCAUAUAAAAGUGCA | SEQ ID NO: 2555 | CACUUUUAUAUGACAACUG | 0.4472 | * |
| MA0672 | SEQ ID NO: 672 | AUAUGACAACUGGGUCCUAAC | SEQ ID NO: 1614 | UAGGACCCAGUUGUCAUAUAA | SEQ ID NO: 2556 | AUAUGACAACUGGGUCCUA | 0.4865 | |
| MA0673 | SEQ ID NO: 673 | UAUGACAACUGGGUCCUAACA | SEQ ID NO: 1615 | UUAGGACCCAGUUGUCAUAUA | SEQ ID NO: 2557 | UAUGACAACUGGGUCCUAA | 0.2445 | |
| MA0674 | SEQ ID NO: 674 | CAACUGGGUCCUAACAGCUGC | SEQ ID NO: 1616 | AGCUGUUAGGACCCAGUUGUC | SEQ ID NO: 2558 | CAACUGGGUCCUAACAGCU | 0.1608 | |
| MA0675 | SEQ ID NO: 675 | AACUGGGUCCUAACAGCUGCU | SEQ ID NO: 1617 | CAGCUGUUAGGACCCAGUUGU | SEQ ID NO: 2559 | AACUGGGUCCUAACAGCUG | 0.3745 | |
| MA0676 | SEQ ID NO: 676 | ACUGGGUCCUAACAGCUGCUC | SEQ ID NO: 1618 | GCAGCUGUUAGGACCCAGUUG | SEQ ID NO: 2560 | ACUGGGUCCUAACAGCUGC | 0.4935 | * |
| MA0677 | SEQ ID NO: 677 | GGGUCCUAACAGCUGCUCAUG | SEQ ID NO: 1619 | UGAGCAGCUGUUAGGACCCAG | SEQ ID NO: 2561 | GGGUCCUAACAGCUGCUCA | 0.5722 | |
| MA0678 | SEQ ID NO: 678 | GGUCCUAACAGCUGCUCAUGC | SEQ ID NO: 1620 | AUGAGCAGCUGUUAGGACCCA | SEQ ID NO: 2562 | GGUCCUAACAGCUGCUCAU | 0.2446 | |
| MA0679 | SEQ ID NO: 679 | ACAGCUGCUCAUGCCGUCUAU | SEQ ID NO: 1621 | AGACGGCAUGAGCAGCUGUUA | SEQ ID NO: 2563 | ACAGCUGCUCAUGCCGUCU | 0.2732 | |
| MA0680 | SEQ ID NO: 680 | AGCUGCUCAUGCCGUCUAUGA | SEQ ID NO: 1622 | AUAGACGGCAUGAGCAGCUGU | SEQ ID NO: 2564 | AGCUGCUCAUGCCGUCUAU | 0.5858 | |
| MA0681 | SEQ ID NO: 681 | CUGCUCAUGCCGUCUAUGAGC | SEQ ID NO: 1623 | UCAUAGACGGCAUGAGCAGCU | SEQ ID NO: 2565 | CUGCUCAUGCCGUCUAUGA | 0.3854 | |
| MA0682 | SEQ ID NO: 682 | UCAUGCCGUCUAUGAGCAAAA | SEQ ID NO: 1624 | UUGCUCAUAGACGGCAUGAGC | SEQ ID NO: 2566 | UCAUGCCGUCUAUGAGCAA | 0.5233 | * |
| MA0683 | SEQ ID NO: 683 | UGCCGUCUAUGAGCAAAAACA | SEQ ID NO: 1625 | UUUUUGCUCAUAGACGGCAUG | SEQ ID NO: 2567 | UGCCGUCUAUGAGCAAAAA | 0.5085 | * |
| MA0684 | SEQ ID NO: 684 | CCGUCUAUGAGCAAAAACAUG | SEQ ID NO: 1626 | UGUUUUUGCUCAUAGACGGCA | SEQ ID NO: 2568 | CCGUCUAUGAGCAAAAACA | 0.2050 | |
| MA0685 | SEQ ID NO: 685 | CGUCUAUGAGCAAAAACAUGA | SEQ ID NO: 1627 | AUGUUUUUGCUCAUAGACGGC | SEQ ID NO: 2569 | CGUCUAUGAGCAAAAACAU | 0.2724 | |
| MA0686 | SEQ ID NO: 686 | UCUAUGAGCAAAAACAUGAUG | SEQ ID NO: 1628 | UCAUGUUUUUGCUCAUAGACG | SEQ ID NO: 2570 | UCUAUGAGCAAAAACAUGA | 0.3618 | |
| MA0687 | SEQ ID NO: 687 | CUAUGAGCAAAAACAUGAUGC | SEQ ID NO: 1629 | AUCAUGUUUUUGCUCAUAGAC | SEQ ID NO: 2571 | CUAUGAGCAAAAACAUGAU | 0.5215 | * |
| MA0688 | SEQ ID NO: 688 | UGAGCAAAAACAUGAUGCAUC | SEQ ID NO: 1630 | UGCAUCAUGUUUUUGCUCAUA | SEQ ID NO: 2572 | UGAGCAAAAACAUGAUGCA | 0.4025 | |
| MA0689 | SEQ ID NO: 689 | GAGCAAAAACAUGAUGCAUCC | SEQ ID NO: 1631 | AUGCAUCAUGUUUUUGCUCAU | SEQ ID NO: 2573 | GAGCAAAAACAUGAUGCAU | 0.4620 | |
| MA0690 | SEQ ID NO: 690 | GCAAAAACAUGAUGCAUCCGC | SEQ ID NO: 1632 | GGAUGCAUCAUGUUUUUGCUC | SEQ ID NO: 2574 | GCAAAAACAUGAUGCAUCC | 0.3825 | |
| MA0691 | SEQ ID NO: 691 | GAUGCAUCCGCCCUGGACAUU | SEQ ID NO: 1633 | UGUCCAGGGCGGAUGCAUCAU | SEQ ID NO: 2575 | GAUGCAUCCGCCCUGGACA | 0.3141 | |
| MA0692 | SEQ ID NO: 692 | AUGCAUCCGCCCUGGACAUUC | SEQ ID NO: 1634 | AUGUCCAGGGCGGAUGCAUCA | SEQ ID NO: 2576 | AUGCAUCCGCCCUGGACAU | 0.5124 | |
| MA0693 | SEQ ID NO: 693 | CCCUGGACAUUCGAAUGGGCA | SEQ ID NO: 1635 | CCCAUUCGAAUGUCCAGGGCG | SEQ ID NO: 2577 | CCCUGGACAUUCGAAUGGG | 0.4911 | |
| MA0694 | SEQ ID NO: 694 | GGACAUUCGAAUGGGCACCCU | SEQ ID NO: 1636 | GGUGCCCAUUCGAAUGUCCAG | SEQ ID NO: 2578 | GGACAUUCGAAUGGGCACC | 0.5227 | * |
| MA0695 | SEQ ID NO: 695 | ACAUUCGAAUGGGCACCCUGA | SEQ ID NO: 1637 | AGGGUGCCCAUUCGAAUGUCC | SEQ ID NO: 2579 | ACAUUCGAAUGGGCACCCU | 0.5385 | * |
| MA0696 | SEQ ID NO: 696 | CAUUCGAAUGGGCACCCUGAA | SEQ ID NO: 1638 | CAGGGUGCCCAUUCGAAUGUC | SEQ ID NO: 2580 | CAUUCGAAUGGGCACCCUG | 0.2598 | |
| MA0697 | SEQ ID NO: 697 | AUUCGAAUGGGCACCCUGAAA | SEQ ID NO: 1639 | UCAGGGUGCCCAUUCGAAUGU | SEQ ID NO: 2581 | AUUCGAAUGGGCACCCUGA | 0.3436 | |
| MA0698 | SEQ ID NO: 698 | UUCGAAUGGGCACCCUGAAAA | SEQ ID NO: 1640 | UUCAGGGUGCCCAUUCGAAUG | SEQ ID NO: 2582 | UUCGAAUGGGCACCCUGAA | 0.3559 | |
| MA0699 | SEQ ID NO: 699 | UCGAAUGGGCACCCUGAAAAG | SEQ ID NO: 1641 | UUUCAGGGUGCCCAUUCGAAU | SEQ ID NO: 2583 | UCGAAUGGGCACCCUGAAA | 0.5967 | * |
| MA0700 | SEQ ID NO: 700 | CGAAUGGGCACCCUGAAAAGA | SEQ ID NO: 1642 | UUUUCAGGGUGCCCAUUCGAA | SEQ ID NO: 2584 | CGAAUGGGCACCCUGAAAA | 0.2110 | |
| MA0701 | SEQ ID NO: 701 | GAAUGGGCACCCUGAAAAGAC | SEQ ID NO: 1643 | CUUUUUAGGGUGCCCAUUCGA | SEQ ID NO: 2585 | GAAUGGGCACCCUGAAAAG | 0.2843 | |
| MA0702 | SEQ ID NO: 702 | UGGGCACCCUGAAAAGACUAU | SEQ ID NO: 1644 | AGUCUUUUCAGGGUGCCCAUU | SEQ ID NO: 2586 | UGGGCACCCUGAAAAGACU | 0.4177 | |
| MA0703 | SEQ ID NO: 703 | GGGCACCCUGAAAAGACUAUC | SEQ ID NO: 1645 | UAGUCUUUUCAGGGUGCCCAU | SEQ ID NO: 2587 | GGGCACCCUGAAAAGACUA | 0.4237 | |
| MA0704 | SEQ ID NO: 704 | GGCACCCUGAAAAGACUAUCA | SEQ ID NO: 1646 | AUAGUCUUUUCAGGGUGCCCA | SEQ ID NO: 2588 | GGCACCCUGAAAAGACUAU | 0.2539 | |

**[Table 1-17]**

| double stranded nucleic acid number | SEQ ID NO: | sense strand sequence (5'--->3') | SEQ ID NO: | antisense strand sequence (5'--->3') | SEQ ID NO: | target MASP2 mRNA sequence | relative expression level | note |
|---|---|---|---|---|---|---|---|---|
| MA0705 | SEQ ID NO: 705 | GCACCCUGAAAAGACUAUCAC | SEQ ID NO: 1647 | GAUAGUCUUUUCAGGGUGCCC | SEQ ID NO: 2589 | GCACCCUGAAAAGACUAUC | 0.4681 | |
| MA0706 | SEQ ID NO: 706 | CACCCUGAAAAGACUAUCACC | SEQ ID NO: 1648 | UGAUAGUCUUUUCAGGGUGCC | SEQ ID NO: 2590 | CACCCUGAAAAGACUAUCA | 0.2546 | |
| MA0707 | SEQ ID NO: 707 | ACCCUGAAAAGACUAUCACCU | SEQ ID NO: 1649 | GUGAUAGUCUUUUCAGGGUGC | SEQ ID NO: 2591 | ACCCUGAAAAGACUAUCAC | 0.4901 | * |
| MA0708 | SEQ ID NO: 708 | CCCUGAAAAGACUAUCACCUC | SEQ ID NO: 1650 | GGUGAUAGUCUUUUCAGGGUG | SEQ ID NO: 2592 | CCCUGAAAAGACUAUCACC | 0.2992 | |
| MA0709 | SEQ ID NO: 709 | CCUGAAAAGACUAUCACCUCA | SEQ ID NO: 1651 | AGGUGAUAGUCUUUUCAGGGU | SEQ ID NO: 2593 | CCUGAAAAGACUAUCACCU | 0.1591 | |
| MA0710 | SEQ ID NO: 710 | CUGAAAAGACUAUCACCUCAU | SEQ ID NO: 1652 | GAGGUGAUAGUCUUUUCAGGG | SEQ ID NO: 2594 | CUGAAAAGACUAUCACCUC | 0.4286 | * |
| MA0711 | SEQ ID NO: 711 | UGAAAAGACUAUCACCUCAUU | SEQ ID NO: 1653 | UGAGGUGAUAGUCUUUUCAGG | SEQ ID NO: 2595 | UGAAAAGACUAUCACCUCA | 0.2791 | |
| MA0712 | SEQ ID NO: 712 | GAAAAGACUAUCACCUCAUUA | SEQ ID NO: 1654 | AUGAGGUGAUAGUCUUUUCAG | SEQ ID NO: 2596 | GAAAAGACUAUCACCUCAU | 0.5838 | * |
| MA0713 | SEQ ID NO: 713 | AAGACUAUCACCUCAUUAUAC | SEQ ID NO: 1655 | AUAAUGAGGUGAUAGUCUUUU | SEQ ID NO: 2597 | AAGACUAUCACCUCAUUAU | 0.3405 | |
| MA0714 | SEQ ID NO: 714 | AGACUAUCACCUCAUUAUACA | SEQ ID NO: 1656 | UAUAAUGAGGUGAUAGUCUUU | SEQ ID NO: 2598 | AGACUAUCACCUCAUUAUA | 0.1884 | |
| MA0715 | SEQ ID NO: 715 | GACUAUCACCUCAUUAUACAC | SEQ ID NO: 1657 | GUAUAAUGAGGUGAUAGUCUU | SEQ ID NO: 2599 | GACUAUCACCUCAUUAUAC | 0.4534 | |
| MA0716 | SEQ ID NO: 716 | ACUAUCACCUCAUUAUACACA | SEQ ID NO: 1658 | UGUAUAAUGAGGUGAUAGUCU | SEQ ID NO: 2600 | ACUAUCACCUCAUUAUACA | 0.1301 | |
| MA0717 | SEQ ID NO: 717 | CUAUCACCUCAUUAUACACAA | SEQ ID NO: 1659 | GUGUAUAAUGAGGUGAUAGUC | SEQ ID NO: 2601 | CUAUCACCUCAUUAUACAC | 0.1852 | |
| MA0718 | SEQ ID NO: 718 | AUCACCUCAUUAUACACAAGC | SEQ ID NO: 1660 | UUGUGUAUAAUGAGGUGAUAG | SEQ ID NO: 2602 | AUCACCUCAUUAUACACAA | 0.5239 | |
| MA0719 | SEQ ID NO: 719 | UCACCUCAUUAUACACAAGCC | SEQ ID NO: 1661 | CUUGUGUAUAAUGAGGUGAUA | SEQ ID NO: 2603 | UCACCUCAUUAUACACAAG | 0.5667 | |
| MA0720 | SEQ ID NO: 720 | ACCUCAUUAUACACAAGCCUG | SEQ ID NO: 1662 | GGCUUGUGUAUAAUGAGGUGA | SEQ ID NO: 2604 | ACCUCAUUAUACACAAGCC | 0.3569 | |
| MA0721 | SEQ ID NO: 721 | CCUCAUUAUACACAAGCCUGG | SEQ ID NO: 1663 | AGGCUUGUGUAUAAUGAGGUG | SEQ ID NO: 2605 | CCUCAUUAUACACAAGCCU | 0.4619 | * |
| MA0722 | SEQ ID NO: 722 | CUCAUUAUACACAAGCCUGGU | SEQ ID NO: 1664 | CAGGCUUGUGUAUAAUGAGGU | SEQ ID NO: 2606 | CUCAUUAUACACAAGCCUG | 0.1992 | |
| MA0723 | SEQ ID NO: 723 | UCAUUAUACACAAGCCUGGUC | SEQ ID NO: 1665 | CCAGGCUUGUGUAUAAUGAGG | SEQ ID NO: 2607 | UCAUUAUACACAAGCCUGG | 0.5752 | * |
| MA0724 | SEQ ID NO: 724 | CAUUAUACACAAGCCUGGUCU | SEQ ID NO: 1666 | ACCAGGCUUGUGUAUAAUGAG | SEQ ID NO: 2608 | CAUUAUACACAAGCCUGGU | 0.3232 | |
| MA0725 | SEQ ID NO: 725 | AUUAUACACAAGCCUGGUCUG | SEQ ID NO: 1667 | GACCAGGCUUGUGUAUAAUGA | SEQ ID NO: 2609 | AUUAUACACAAGCCUGGUC | 0.5738 | * |
| MA0726 | SEQ ID NO: 726 | UUAUACACAAGCCUGGUCUGA | SEQ ID NO: 1668 | AGACCAGGCUUGUGUAUAAUG | SEQ ID NO: 2610 | UUAUACACAAGCCUGGUCU | 0.5138 | * |
| MA0727 | SEQ ID NO: 727 | UAUACACAAGCCUGGUCUGAA | SEQ ID NO: 1669 | CAGACCAGGCUUGUGUAUAAU | SEQ ID NO: 2611 | UAUACACAAGCCUGGUCUG | 0.3517 | |
| MA0728 | SEQ ID NO: 728 | AUACACAAGCCUGGUCUGAAG | SEQ ID NO: 1670 | UCAGACCAGGCUUGUGUAUAA | SEQ ID NO: 2612 | AUACACAAGCCUGGUCUGA | 0.2272 | |
| MA0729 | SEQ ID NO: 729 | UACACAAGCCUGGUCUGAAGC | SEQ ID NO: 1671 | UUCAGACCAGGCUUGUGUAUA | SEQ ID NO: 2613 | UACACAAGCCUGGUCUGAA | 0.3259 | |
| MA0730 | SEQ ID NO: 730 | ACACAAGCCUGGUCUGAAGCU | SEQ ID NO: 1672 | CUUCAGACCAGGCUUGUGUAU | SEQ ID NO: 2614 | ACACAAGCCUGGUCUGAAG | 0.3315 | |
| MA0731 | SEQ ID NO: 731 | CACAAGCCUGGUCUGAAGCUG | SEQ ID NO: 1673 | GCUUCAGACCAGGCUUGUGUA | SEQ ID NO: 2615 | CACAAGCCUGGUCUGAAGC | 0.3870 | |
| MA0732 | SEQ ID NO: 732 | ACAAGCCUGGUCUGAAGCUGU | SEQ ID NO: 1674 | AGCUUCAGACCAGGCUUGUGU | SEQ ID NO: 2616 | ACAAGCCUGGUCUGAAGCU | 0.4855 | * |
| MA0733 | SEQ ID NO: 733 | CAAGCCUGGUCUGAAGCUGUU | SEQ ID NO: 1675 | CAGCUUCAGACCAGGCUUGUG | SEQ ID NO: 2617 | CAAGCCUGGUCUGAAGCUG | 0.3114 | |
| MA0734 | SEQ ID NO: 734 | AAGCCUGGUCUGAAGCUGUUU | SEQ ID NO: 1676 | ACAGCUUCAGACCAGGCUUGU | SEQ ID NO: 2618 | AAGCCUGGUCUGAAGCUGU | 0.3144 | |
| MA0735 | SEQ ID NO: 735 | AGCCUGGUCUGAAGCUGUUUU | SEQ ID NO: 1677 | AACAGCUUCAGACCAGGCUUG | SEQ ID NO: 2619 | AGCCUGGUCUGAAGCUGUU | 0.3286 | |
| MA0736 | SEQ ID NO: 736 | GCCUGGUCUGAAGCUGUUUUU | SEQ ID NO: 1678 | AAACAGCUUCAGACCAGGCUU | SEQ ID NO: 2620 | GCCUGGUCUGAAGCUGUUU | 0.5949 | * |
| MA0737 | SEQ ID NO: 737 | CCUGGUCUGAAGCUGUUUUUA | SEQ ID NO: 1619 | AAAACAGCUUCAGACCAGGCU | SEQ ID NO: 2621 | CCUGGUCUGAAGCUGUUUU | 0.5426 | * |
| MA0738 | SEQ ID NO: 738 | CUGGUCUGAAGCUGUUUUUAU | SEQ ID NO: 1680 | AAAAACAGCUUCAGACCAGGC | SEQ ID NO: 2622 | CUGGUCUGAAGCUGUUUUU | 0.2070 | |
| MA0739 | SEQ ID NO: 739 | UGGUCUGAAGCUGUUUUUAUA | SEQ ID NO: 1681 | UAAAAACAGCUUCAGACCAGG | SEQ ID NO: 2623 | UGGUCUGAAGCUGUUUUUA | 0.2202 | |
| MA0740 | SEQ ID NO: 740 | GGUCUGAAGCUGUUUUUAUAC | SEQ ID NO: 1682 | AUAAAAACAGCUUCAGACCAG | SEQ ID NO: 2624 | GGUCUGAAGCUGUUUUUAU | 0.3214 | |
| MA0741 | SEQ ID NO: 741 | GUCUGAAGCUGUUUUUAUACA | SEQ ID NO: 1683 | UAUAAAAACAGCUUCAGACCA | SEQ ID NO: 2625 | GUCUGAAGCUGUUUUUAUA | 0.1678 | |
| MA0742 | SEQ ID NO: 742 | CUGAAGCUGUUUUUAUACAUG | SEQ ID NO: 1684 | UGUAUAAAAACAGCUUCAGAC | SEQ ID NO: 2626 | CUGAAGCUGUUUUUAUACA | 0.2003 | |
| MA0743 | SEQ ID NO: 743 | UGAAGCUGUUUUUAUACAUGA | SEQ ID NO: 1685 | AUGUAUAAAAACAGCUUCAGA | SEQ ID NO: 2627 | UGAAGCUGUUUUUAUACAU | 0.4078 | |
| MA0744 | SEQ ID NO: 744 | GAAGCUGUUUUUAUACAUGAA | SEQ ID NO: 1686 | CAUGUAUAAAAACAGCUUCAG | SEQ ID NO: 2628 | GAAGCUGUUUUUAUACAUG | 0.3181 | |
| MA0745 | SEQ ID NO: 745 | AAGCUGUUUUUAUACAUGAAG | SEQ ID NO: 1687 | UCAUGUAUAAAAACAGCUUCA | SEQ ID NO: 2629 | AAGCUGUUUUUAUCAUGA | 0.3278 | |
| MA0746 | SEQ ID NO: 746 | UUUUAUACAUGAAGGUUAUAC | SEQ ID NO: 1688 | AUAACCUUCAUGUAUAAAAAC | SEQ ID NO: 2630 | UUUUAUACAUGAAGGUUAU | 0.4180 | |
| MA0747 | SEQ ID 7 NO: 747 | UAUACAUGAAGGUUAUACUCA | SEQ ID NO: 1689 | AGUAUAACCUUCAUGUAUAAA | SEQ ID NO: 2631 | UAUACAUGAAGGUUAUACU | 0.5284 | * |
| MA0748 | SEQ ID NO: 748 | ACAUGAAGGUUAUACUCAUGA | SEQ ID NO: 1690 | AUGAGUAUAACCUUCAUGUAU | SEQ ID NO: 2632 | ACAUGAAGGUUAUACUCAU | 0.2583 | |

**[Table 1-18]**

| double stranded nucleic acid number | SEQ ID NO: | sense strand sequence (5'--->3') | SEQ ID NO: | antisense strand sequence (5'--->3') | SEQ ID NO : | target MASP2 mRNA sequence | relative expression level | note |
|---|---|---|---|---|---|---|---|---|
| MA0749 | SEQ ID NO: 749 | UGAAGGUUAUACUCAUGAUGC | SEQ ID NO: 1691 | AUCAUGAGUAUAACCUUCAUG | SEQ ID NO: 2633 | UGAAGGUUAUACUCAUGAU | 0.4428 | |
| MA0750 | SEQ ID NO: 750 | GAAGGUUAUACUCAUGAUGCU | SEQ ID NO: 1692 | CAUCAUGAGUAUAACCUUCAU | SEQ ID NO: 2634 | GAAGGUUAUACUCAUGAUG | 0.5120 | * |
| MA0751 | SEQ ID NO: 751 | AGGUUAUACUCAUGAUGCUGG | SEQ ID NO: 1693 | AGCAUCAUGAGUAUAACCUUC | SEQ ID NO: 2635 | AGGUUAUACUCAUGAUGCU | 0.3862 | |
| MA0752 | SEQ ID NO: 752 | GUUAUACUCAUGAUGCUGGCU | SEQ ID NO: 1694 | CCAGCAUCAUGAGUAUAACCU | SEQ ID NO: 2636 | GUUAUACUCAUGAUGCUGG | 0.5673 | * |
| MA0753 | SEQ ID NO: 753 | UACUCAUGAUGCUGGCUUUGA | SEQ ID NO: 1695 | AAAGCCAGCAUCAUGAGUAUA | SEQ ID NO: 2637 | UACUCAUGAUGCUGGCUUU | 0.5018 | |
| MA0754 | SEQ ID NO: 754 | ACUCAUGAUGCUGGCUUUGAC | SEQ ID NO: 1696 | CAAAGCCAGCAUCAUGAGUAU | SEQ ID NO: 2638 | ACUCAUGAUGCUGGCUUUG | 0.4341 | * |
| MA0755 | SEQ ID NO: 755 | CUCAUGAUGCUGGCUUUGACA | SEQ ID NO: 1697 | UCAAAGCCAGCAUCAUGAGUA | SEQ ID NO: 2639 | CUCAUGAUGCUGGCUUUGA | 0.2301 | |
| MA0756 | SEQ ID NO: 756 | UCAUGAUGCUGGCUUUGACAA | SEQ ID NO: 1698 | GUCAAAGCCAGCAUCAUGAGU | SEQ ID NO: 2640 | UCAUGAUGCUGGCUUUGAC | 0.5801 | * |
| MA0757 | SEQ ID NO: 757 | CAUGAUGCUGGCUUUGACAAU | SEQ ID NO: 1699 | UGUCAAAGCCAGCAUCAUGAG | SEQ ID NO: 2641 | CAUGAUGCUGGCUUUGACA | 0.2522 | |
| MA0758 | SEQ ID NO: 758 | AUGAUGCUGGCUUUGACAAUG | SEQ ID NO: 1700 | UUGUCAAAGCCAGCAUCAUGA | SEQ ID NO: 2642 | AUGAUGCUGGCUUUGACAA | 0.1982 | |
| MA0759 | SEQ ID NO: 759 | UGAUGCUGGCUUUGACAAUGA | SEQ ID NO: 1701 | AUUGUCAAAGCCAGCAUCAUG | SEQ ID NO: 2643 | UGAUGCUGGCUUUGACAAU | 0.3565 | |
| MA0760 | SEQ ID NO: 760 | GAUGCUGGCUUUGACAAUGAC | SEQ ID NO: 1702 | CAUUGUCAAAGCCAGCAUCAU | SEQ ID NO: 2644 | GAUGCUGGCUUUGACAAUG | 0.0781 | |
| MA0761 | SEQ ID NO: 761 | AUGCUGGCUUUGACAAUGACA | SEQ ID NO: 1703 | UCAUUGUCAAAGCCAGCAUCA | SEQ ID NO: 2645 | AUGCUGGCUUUGACAAUGA | 0.2048 | |
| MA0762 | SEQ ID NO: 762 | GCUGGCUUUGACAAUGACAUA | SEQ ID NO: 1704 | UGUCAUUGUCAAAGCCAGCAU | SEQ ID NO: 2646 | GCUGGCUUUGACAAUGACA | 0.2943 | |
| MA0763 | SEQ ID NO: 763 | CUGGCUUUGACAAUGACAUAG | SEQ ID NO: 1705 | AUGUCAUUGUCAAAGCCAGCA | SEQ ID NO: 2647 | CUGGCUUUGACAAUGACAU | 0.1290 | |
| MA0764 | SEQ ID NO: 764 | UGGCUUUGACAAUGACAUAGC | SEQ ID NO: 1706 | UAUGUCAUUGUCAAAGCCAGC | SEQ ID NO: 2648 | UGGCUUUGACAAUGACAUA | 0.4822 | |
| MA0765 | SEQ ID NO: 765 | GGCUUUGACAAUGACAUAGCA | SEQ ID NO: 1707 | CUAUGUCAUUGUCAAAGCCAG | SEQ ID NO: 2649 | GGCUUUGACAAUGACAUAG | 0.2661 | |
| MA0766 | SEQ ID NO: 766 | GCUUUGACAAUGACAUAGCAC | SEQ ID NO: 1708 | GCUAUGUCAUUGUCAAAGCCA | SEQ ID NO: 2650 | GCUUUGACAAUGACAUAGC | 0.3189 | |
| MA0767 | SEQ ID NO: 767 | CUUUGACAAUGACAUAGCACU | SEQ ID NO: 1709 | UGCUAUGUCAUUGUCAAAGCC | SEQ ID NO: 2651 | CUUUGACAAUGACAUAGCA | 0.2030 | |
| MA0768 | SEQ ID NO: 768 | UUGACAAUGACAUAGCACUGA | SEQ ID NO: 1710 | AGUGCUAUGUCAUUGUCAAAG | SEQ ID NO: 2652 | UUGACAAUGACAUAGCACU | 0.5830 | * |
| MA0769 | SEQ ID NO: 769 | GACAAUGACAUAGCACUGAUU | SEQ ID NO: 1711 | UCAGUGCUAUGUCAUUGUCAA | SEQ ID NO: 2653 | GACAAUGACAUAGCACUGA | 0.2930 | |
| MA0770 | SEQ ID NO: 770 | ACAAUGACAUAGCACUGAUUA | SEQ ID NO: 1712 | AUCAGUGCUAUGUCAUUGUCA | SEQ ID NO: 2654 | ACAAUGACAUAGCACUGAU | 0.1669 | |
| MA0771 | SEQ ID NO: 771 | CAAUGACAUAGCACUGAUUAA | SEQ ID NO: 1713 | AAUCAGUGCUAUGUCAUUGUC | SEQ ID NO: 2655 | CAAUGACAUAGCACUGAUU | 0.2136 | |
| MA0772 | SEQ ID NO: 772 | AAUGACAUAGCACUGAUUAAA | SEQ ID NO: 1714 | UAAUCAGUGCUAUGUCAUUGU | SEQ ID NO: 2656 | AAUGACAUAGCACUGAUUA | 0.3583 | |
| MA0773 | SEQ ID NO: 773 | AUGACAUAGCACUGAUUAAAU | SEQ ID NO: 1715 | UUAAUCAGUGCUAUGUCAUUG | SEQ ID NO: 2657 | AUGACAUAGCACUGAUUAA | 0.1389 | |
| MA0774 | SEQ ID NO: 774 | UGACAUAGCACUGAUUAAAUU | SEQ ID NO: 1716 | UUUAAUCAGUGCUAUGUCAUU | SEQ ID NO: 2658 | UGACAUAGCACUGAUUAAA | 0.1580 | |
| MA0775 | SEQ ID NO: 775 | GACAUAGCACUGAUUAAAUUG | SEQ ID NO: 1717 | AUUUAAUCAGUGCUAUGUCAU | SEQ ID NO: 2659 | GACAUAGCACUGAUUAAAU | 0.1395 | |
| MA0776 | SEQ ID NO: 776 | ACAUAGCACUGAUUAAAUUGA | SEQ ID NO: 1718 | AAUUUAAUCAGUGCUAUGUCA | SEQ ID NO: 2660 | ACAUAGCACUGAUUAAAUU | 0.3979 | |
| MA0777 | SEQ ID NO: 777 | CAUAGCACUGAUUAAAUUGAA | SEQ ID NO: 1719 | CAAUUUAAUCAGUGCUAUGUC | SEQ ID NO: 2661 | CAUAGCACUGAUUAAAUUG | 0.2916 | |
| MA0778 | SEQ ID NO: 778 | AUAGCACUGAUUAAAUUGAAU | SEQ ID NO: 1720 | UCAAUUUAAUCAGUGCUAUGU | SEQ ID NO: 2662 | AUAGCACUGAUUAAAUUGA | 0.4196 | |
| MA0779 | SEQ ID NO: 779 | UAGCACUGAUUAAAUUGAAUA | SEQ ID NO: 1721 | UUCAAUUUAAUCAGUGCUAUG | SEQ ID NO: 2663 | UAGCACUGAUUAAAUUGAA | 0.3348 | |
| MA0780 | SEQ ID NO: 780 | AGCACUGAUUAAAUUGAAUAA | SEQ ID NO: 1722 | AUUCAAUUUAAUCAGUGCUAU | SEQ ID NO: 2664 | AGCACUGAUUAAAUUGAAU | 0.3641 | |
| MA0781 | SEQ ID NO: 781 | GCACUGAUUAAAUUGAAUAAC | SEQ ID NO: 1723 | UAUUCAAUUUAAUCAGUGCUA | SEQ ID NO: 2665 | GCACUGAUUAAAUUGAAUA | 0.2676 | |
| MA0782 | SEQ ID NO: 782 | CACUGAUUAAAUUGAAUAACA | SEQ ID NO: 1724 | UUAUUCAAUUUAAUCAGUGCU | SEQ ID NO: 2666 | CACUGAUUAAAUUGAAUAA | 0.2343 | |
| MA0783 | SEQ ID NO: 783 | CUGAUUAAAUUGAAUAACAAA | SEQ ID NO: 1725 | UGUUAUUCAAUUUAAUCAGUG | SEQ ID NO: 2667 | CUGAUUAAAUUGAAUAACA | 0.1117 | |
| MA0784 | SEQ ID NO: 784 | UGAUUAAAUUGAAUAACAAAG | SEQ ID NO: 1726 | UUGUUAUUCAAUUUAAUCAGU | SEQ ID NO: 2668 | UGAUUAAAUUGAAUAACAA | 0.2792 | |
| MA0785 | SEQ ID NO: 785 | GAUUAAAUUGAAUAACAAAGU | SEQ ID NO: 1727 | UUUGUUAUUCAAUUUAAUCAG | SEQ ID NO: 2669 | GAUUAAAUUGAAUAACAAA | 0.2171 | |
| MA0786 | SEQ ID NO: 786 | AAUUGAAUAACAAAGUUGUAA | SEQ ID NO: 1728 | ACAACUUUGUUAUUCAAUUUA | SEQ ID NO: 2670 | AAUUGAAUAACAAAGUUGU | 0.5295 | * |
| MA0787 | SEQ ID NO: 787 | AAUAACAAAGUUGUAAUCAAU | SEQ ID NO: 1729 | UGAUUACAACUUUGUUAUUCA | SEQ ID NO: 2671 | AAUAACAAAGUUGUAAUCA | 0.4232 | |
| MA0788 | SEQ ID NO: 788 | AUAACAAAGUUGUAAUCAAUA | SEQ ID NO: 1730 | UUGAUUACAACUUUGUUAUUC | SEQ ID NO: 2672 | AUAACAAAGUUGUAAUCAA | 0.2156 | |
| MA0789 | SEQ ID NO: 789 | UAACAAAGUUGUAAUCAAUAG | SEQ ID NO: 1731 | AUUGAUUACAACUUUGUUAUU | SEQ ID NO: 2673 | UAACAAAGUUGUAAUCAAU | 0.4481 | |
| MA0790 | SEQ ID NO: 790 | AACAAAGUUGUAAUCAAUAGC | SEQ ID NO: 1732 | UAUUGAUUACAACUUUGUUAU | SEQ ID NO: 2674 | AACAAAGUUGUAAUCAAUA | 0.5348 | * |
| MA0791 | SEQ ID NO: 791 | AAGUUGUAAUCAAUAGCAACA | SEQ ID NO: 1733 | UUGCUAUUGAUUACAACUUUG | SEQ ID NO: 2675 | AAGUUGUAAUCAAUAGCAA | 0.5833 | * |
| MA0792 | SEQ ID NO: 792 | GUUGUAAUCAAUAGCAACAUC | SEQ ID NO: 1734 | UGUUGCUAUUGAUUACAACUU | SEQ ID NO: 2676 | GUUGUAAUCAAUAGCAACA | 0.2408 | |

**[Table 1-19]**

| double stranded nucleic acid number | SEQ ID NO: | sense strand sequence (5'--->3') | SEQ ID NO: | antisense strand sequence (5'--->3') | SEQ ID NO: | target MASP2 mRNA sequence | relative expression level | note |
|---|---|---|---|---|---|---|---|---|
| MA0793 | SEQ ID NO: 793 | GUAAUCAAUAGCAACAUCACG | SEQ ID NO: 1735 | UGAUGUUGCUAUUGAUUACAA | SEQ ID NO: 2677 | GUAAUCAAUAGCAACAUCA | 0.5308 | * |
| MA0794 | SEQ ID NO: 794 | AAUCAAUAGCAACAUCACGCC | SEQ ID NO: 1736 | CGUGAUGUUGCUAUUGAUUAC | SEQ ID NO: 2678 | AAUCAAUAGCAACAUCACG | 0.5752 | * |
| MA0795 | SEQ ID NO: 795 | CUAUUUGUCUGCCAAGAAAAG | SEQ ID NO: 1737 | UUUCUUGGCAGACAAAUAGGC | SEQ ID NO: 2679 | CUAUUUGUCUGCCAAGAAA | 0.4156 | |
| MA0796 | SEQ ID NO: 796 | UAUUUGUCUGCCAAGAAAAGA | SEQ ID NO: 1738 | UUUUCUUGGCAGACAAAUAGG | SEQ ID NO: 2680 | UAUUUGUCUGCCAAGAAAA | 0.2340 | |
| MA0797 | SEQ ID NO: 797 | AUUUGUCUGCCAAGAAAAGAA | SEQ ID NO: 1739 | CUUUUCUUGGCAGACAAAUAG | SEQ ID NO: 2681 | AUUUGUCUGCCAAGAAAAG | 0.3825 | |
| MA0798 | SEQ ID NO: 798 | UGCCAAGAAAAGAAGCUGAAU | SEQ ID NO: 1740 | UCAGCUUCUUUUCUUGGCAGA | SEQ ID NO: 2682 | UGCCAAGAAAAGAAGCUGA | 0.5997 | * |
| MA0799 | SEQ ID NO: 799 | CCAAGAAAAGAAGCUGAAUCC | SEQ ID NO: 1741 | AUUCAGCUUCUUUUCUUGGCA | SEQ ID NO: 2683 | CCAAGAAAAGAAGCUGAAU | 0.4103 | |
| MA0800 | SEQ ID NO: 800 | GAAGCUGAAUCCUUUAUGAGG | SEQ ID NO: 1742 | UCAUAAAGGAUUCAGCUUCUU | SEQ ID NO: 2684 | GAAGCUGAAUCCUUUAUGA | 0.2991 | |
| MA0801 | SEQ ID NO: 801 | GCUGAAUCCUUUAUGAGGACA | SEQ ID NO: 1743 | UCCUCAUAAAGGAUUCAGCUU | SEQ ID NO: 2685 | GCUGAAUCCUUUAUGAGGA | 0.5691 | * |
| MA0802 | SEQ ID NO: 802 | CUGAAUCCUUUAUGAGGACAG | SEQ ID NO: 1744 | GUCCUCAUAAAGGAUUCAGCU | SEQ ID NO: 2686 | CUGAAUCCUUUAUGAGGAC | 0.5511 | * |
| MA0803 | SEQ ID NO: 803 | AGGACAGAUGACAUUGGAACU | SEQ ID NO: 1745 | UUCCAAUGUCAUCUGUCCUCA | SEQ ID NO: 2687 | AGGACAGAUGACAUUGGAA | 0.4492 | * |
| MA0804 | SEQ ID NO: 804 | GGACAGAUGACAUUGGAACUG | SEQ ID NO: 1746 | GUUCCAAUGUCAUCUGUCCUC | SEQ ID NO: 2688 | GGACAGAUGACAUUGGAAC | 0.5747 | |
| MA0805 | SEQ ID NO: 805 | CAUUGGAACUGCAUCUGGAUG | SEQ ID NO: 1747 | UCCAGAUGCAGUUCCAAUGUC | SEQ ID NO: 2689 | CAUUGGAACUGCAUCUGGA | 0.4047 | |
| MA0806 | SEQ ID NO: 806 | AACUGCAUCUGGAUGGGGAUU | SEQ ID NO: 1748 | UCCCCAUCCAGAUGCAGUUCC | SEQ ID NO: 2690 | AACUGCAUCUGGAUGGGGA | 0.5476 | * |
| MA0807 | SEQ ID NO: 807 | CUGCAUCUGGAUGGGGAUUAA | SEQ ID NO: 1749 | AAUCCCCAUCCAGAUGCAGUU | SEQ ID NO: 2691 | CUGCAUCUGGAUGGGGAUU | 0.4427 | |
| MA0808 | SEQ ID NO: 808 | GGAUGGGGAUUAACCCAAAGG | SEQ ID NO: 1750 | UUUGGGUUAAUCCCCAUCCAG | SEQ ID NO: 2692 | GGAUGGGGAUUAACCCAAA | 0.3251 | |
| MA0809 | SEQ ID NO: 809 | GGGGUUUUCUUGCUAGAAAUC | SEQ ID NO: 1751 | UUUCUAGCAAGAAAACCCCUU | SEQ ID NO: 2693 | GGGGUUUUCUUGCUAGAAA | 0.5848 | * |
| MA0810 | SEQ ID NO: 810 | GGGUUUUCUUGCUAGAAAUCU | SEQ ID NO: 1752 | AUUUCUAGCAAGAAAACCCCU | SEQ ID NO: 2694 | GGGUUUUCUUGCUAGAAAU | 0.5307 | * |
| MA0811 | SEQ ID NO: 811 | GGUUUUCUUGCUAGAAAUCUA | SEQ ID NO: 1753 | GAUUUCUAGCAAGAAAACCCC | SEQ ID NO: 2695 | GGUUUUCUUGCUAGAAAUC | 0.5842 | |
| MA0812 | SEQ ID NO: 812 | GUUUUCUUGCUAGAAAUCUAA | SEQ ID NO: 1754 | AGAUUUCUAGCAAGAAAACCC | SEQ ID NO: 2696 | GUUUUCUUGCUAGAAAUCU | 0.5979 | * |
| MA0813 | SEQ ID NO: 813 | UUUUCUUGCUAGAAAUCUAAU | SEQ ID NO: 1755 | UAGAUUUCUAGCAAGAAAACC | SEQ ID NO: 2697 | UUUUCUUGCUAGAAAUCUA | 0.3900 | |
| MA0814 | SEQ ID NO: 814 | UUUCUUGCUAGAAAUCUAAUG | SEQ ID NO: 1756 | UUAGAUUUCUAGCAAGAAAAC | SEQ ID NO: 2698 | UUUCUUGCUAGAAAUCUAA | 0.3701 | |
| MA0815 | SEQ ID NO: 81 5 | CUUGCUAGAAAUCUAAUGUAU | SEQ ID NO: 1757 | ACAUUAGAUUUCUAGCAAGAA | SEQ ID NO: 2699 | CUUGCUAGAAAUCUAAUGU | 0.5529 | * |
| MA0816 | SEQ ID NO: 816 | UUGCUAGAAAUCUAAUGUAUG | SEQ ID NO: 1758 | UACAUUAGAUUUCUAGCAAGA | SEQ ID NO: 2700 | UUGCUAGAAAUCUAAUGUA | 0.4379 | |
| MA0817 | SEQ ID NO: 817 | UGCUAGAAAUCUAAUGUAUGU | SEQ ID NO: 1759 | AUACAUUAGAUUUCUAGCAAG | SEQ ID NO: 2701 | UGCUAGAAAUCUAAUGUAU | 0.4668 | * |
| MA0818 | SEQ ID NO: 818 | AUCUAAUGUAUGUCGACAUAC | SEQ ID NO: 1760 | AUGUCGACAUACAUUAGAUUU | SEQ ID NO: 2702 | AUCUAAUGUAUGUCGACAU | 0.5457 | * |
| MA0819 | SEQ ID NO: 819 | UCUAAUGUAUGUCGACAUACC | SEQ ID NO: 1761 | UAUGUCGACAUACAUUAGAUU | SEQ ID NO: 2703 | UCUAAUGUAUGUCGACAUA | 0.5453 | |
| MA0820 | SEQ ID NO: 820 | UAAUGUAUGUCGACAUACCGA | SEQ ID NO: 1762 | GGUAUGUCGACAUACAUUAGA | SEQ ID NO: 2704 | UAAUGUAUGUCGACAUACC | 0.5130 | * |
| MA0821 | SEQ ID NO: 821 | AUGUAUGUCGACAUACCGAUU | SEQ ID NO: 1763 | UCGGUAUGUCGACAUACAUUA | SEQ ID NO: 2705 | AUGUAUGUCGACAUACCGA | 0.3913 | |
| MA0822 | SEQ ID NO: 822 | UGUAUGUCGACAUACCGAUUG | SEQ ID NO: 1764 | AUCGGUAUGUCGACAUACAUU | SEQ ID NO: 2706 | UGUAUGUCGACAUACCGAU | 0.4948 | |
| MA0823 | SEQ ID NO: 823 | AUGUCGACAUACCGAUUGUUG | SEQ ID NO: 1765 | ACAAUCGGUAUGUCGACAUAC | SEQ ID NO: 2707 | AUGUCGACAUACCGAUUGU | 0.5940 | * |
| MA0824 | SEQ ID NO: 824 | UGUCGACAUACCGAUUGUUGA | SEQ ID NO: 1766 | AACAAUCGGUAUGUCGACAUA | SEQ ID NO: 2708 | UGUCGACAUACCGAUUGUU | 0.5752 | * |
| MA0825 | SEQ ID NO: 825 | CCGAUUGUUGACCAUCAAAAA | SEQ ID NO: 1767 | UUUGAUGGUCAACAAUCGGUA | SEQ ID NO: 2709 | CCGAUUGUUGACCAUCAAA | 0.5365 | * |
| MA0826 | SEQ ID NO: 826 | CGAUUGUUGACCAUCAAAAAU | SEQ ID NO: 1768 | UUUUGAUGGUCAACAAUCGGU | SEQ ID NO: 2710 | CGAUUGUUGACCAUCAAAA | 0.2433 | |
| MA0827 | SEQ ID NO: 827 | GAUUGUUGACCAUCAAAAAUG | SEQ ID NO: 1769 | UUUUUGAUGGUCAACAAUCGG | SEQ ID NO: 2711 | GAUUGUUGACCAUCAAAAA | 0.3497 | |
| MA0828 | SEQ ID NO: 828 | UGUUGACCAUCAAAAAUGUAC | SEQ ID NO: 1770 | ACAUUUUUGAUGGUCAACAAU | SEQ ID NO: 2712 | UGUUGACCAUCAAAAAUGU | 0.5364 | * |
| MA0829 | SEQ ID NO: 829 | GUUGACCAUCAAAAAUGUACU | SEQ ID NO: 1771 | UACAUUUUUGAUGGUCAACAA | SEQ ID NO: 2713 | GUUGACCAUCAAAAAUGUA | 0.4364 | |
| MA0830 | SEQ ID NO: 830 | UUGACCAUCAAAAAUGUACUG | SEQ ID NO: 1772 | GUACAUUUUUGAUGGUCAACA | SEQ ID NO: 2714 | UUGACCAUCAAAAAUGUAC | 0.5726 | * |
| MA0831 | SEQ ID NO: 831 | AAAAUGUACUGCUGCAUAUGA | SEQ ID NO: 1773 | AUAUGCAGCAGUACAUUUUUG | SEQ ID NO: 2715 | AAAAUGUACUGCUGCAUAU | 0.5474 | * |
| MA0832 | SEQ ID NO: 832 | UGUACUGCUGCAUAUGAAAAG | SEQ ID NO: 1774 | UUUCAUAUGCAGCAGUACAUU | SEQ ID NO: 2716 | UGUACUGCUGCAUAUGAAA | 0.4535 | |
| MA0833 | SEQ ID NO: 833 | CUGCUGCAUAUGAAAAGCCAC | SEQ ID NO: 1775 | GGCUUUUCAUAUGCAGCAGUA | SEQ ID NO: 2717 | CUGCUGCAUAUGAAAAGCC | 0.2403 | |
| MA0834 | SEQ ID NO: 834 | UGCUGCAUAUGAAAAGCCACC | SEQ ID NO: 1776 | UGGCUUUUCAUAUGCAGCAGU | SEQ ID NO: 2718 | UGCUGCAUAUGAAAAGCCA | 0.4553 | * |
| MA0835 | SEQ ID NO: 835 | GCUGCAUAUGAAAAGCCACCC | SEQ ID NO: 1777 | GUGGCUUUUCAUAUGCAGCAG | SEQ ID NO: 2719 | GCUGCAUAUGAAAAGCCAC | 0.5889 | |
| MA0836 | SEQ ID NO: 836 | CAUAUGAAAAGCCACCCUAUC | SEQ ID NO: 1778 | UAGGGUGGCUUUUCAUAUGCA | SEQ ID NO: 2720 | CAUAUGAAAAGCCACCCUA | 0.4989 | * |

**[Table 1-20]**

| double stranded nucleic acid number | SEQ ID NO: | sense strand sequence (5'--->3') | SEQ ID NO: | antisense strand sequence (5'---->3') | SEQ ID NO: | target MASP2 mRNA sequence | relative expression level | note |
|---|---|---|---|---|---|---|---|---|
| MA0837 | SEQ ID NO: 837 | CCACCCUAUCCAAGGGGAAGU | SEQ ID NO: 1779 | UUCCCCUUGGAUAGGGUGGCU | SEQ ID NO: 2721 | CCACCCUAUCCAAGGGGAA | 0.5249 | |
| MA0838 | SEQ ID NO: 838 | UAUCCAAGGGGAAGUGUAACU | SEQ ID NO: 1780 | UUACACUUCCCCUUGGAUAGG | SEQ ID NO: 2722 | UAUCCAAGGGGAAGUGUAA | 0.4134 | |
| MA0839 | SEQ ID NO: 839 | UCCAAGGGGAAGUGUAACUGC | SEQ ID NO: 1781 | AGUUACACUUCCCCUUGGAUA | SEQ ID NO: 2723 | UCCAAGGGGAAGUGUAACU | 0.4837 | * |
| MA0840 | SEQ ID NO: 840 | AAGUGUAACUGCUAACAUGCU | SEQ ID NO: 1782 | CAUGUUAGCAGUUACACUUCC | SEQ ID NO: 2724 | AAGUGUAACUGCUAACAUG | 0.4081 | |
| MA0841 | SEQ ID NO: 841 | GUGUAACUGCUAACAUGCUUU | SEQ ID NO: 1783 | AGCAUGUUAGCAGUUACACUU | SEQ ID NO: 2725 | GUGUAACUGCUAACAUGCU | 0.4491 | * |
| MA0842 | SEQ ID NO: 842 | CUGCUAACAUGCUUUGUGCUG | SEQ ID NO: 1784 | GCACAAAGCAUGUUAGCAGUU | SEQ ID NO: 2726 | CUGCUAACAUGCUUUGUGC | 0.2141 | |
| MA0843 | SEQ ID NO: 843 | UGCUAACAUGCUUUGUGCUGG | SEQ ID NO: 1785 | AGCACAAAGCAUGUUAGCAGU | SEQ ID NO: 2727 | UGCUAACAUGCUUUGUGCU | 0.4940 | |
| MA0844 | SEQ ID NO: 844 | CAUGCUUUGUGCUGGCUUAGA | SEQ ID NO: 1786 | UAAGCCAGCACAAAGCAUGUU | SEQ ID NO: 2728 | CAUGCUUUGUGCUGGCUUA | 0.5449 | * |
| MA0845 | SEQ ID NO: 845 | UGCUUUGUGCUGGCUUAGAAA | SEQ ID NO: 1787 | UCUAAGCCAGCACAAAGCAUG | SEQ ID NO: 2729 | UGCUUUGUGCUGGCUUAGA | 0.4866 | |
| MA0846 | SEQ ID NO: 846 | GCUUUGUGCUGGCUUAGAAAG | SEQ ID NO: 1788 | UUCUAAGCCAGCACAAAGCAU | SEQ ID NO: 2730 | GCUUUGUGCUGGCUUAGAA | 0.5630 | * |
| MA0847 | SEQ ID NO: 847 | AGGACAGCUGCAGAGGUGACA | SEQ ID NO: 1789 | UCACCUCUGCAGCUGUCCUUG | SEQ ID NO: 2731 | AGGACAGCUGCAGAGGUGA | 0.5885 | * |
| MA0848 | SEQ ID NO: 848 | UGCAGAGGUGACAGCGGAGGG | SEQ ID NO: 1790 | CUCCGCUGUCACCUCUGCAGC | SEQ ID NO: 2732 | UGCAGAGGUGACAGCGGAG | 0.4029 | |
| MA0849 | SEQ ID NO: 849 | GGGCACUGGUGUUUCUAGAUA | SEQ ID NO: 1791 | UCUAGAAACACCAGUGCCCCU | SEQ ID NO: 2733 | GGGCACUGGUGUUUCUAGA | 0.4905 | |
| MA0850 | SEQ ID NO: 850 | GCACUGGUGUUUCUAGAUAGU | SEQ ID NO: 1792 | UAUCUAGAAACACCAGUGCCC | SEQ ID NO: 2734 | GCACUGGUGUUUCUAGAUA | 0.5575 | * |
| MA0851 | SEQ ID NO: 851 | UGGUGUUUCUAGAUAGUGAAA | SEQ ID NO: 1793 | UCACUAUCUAGAAACACCAGU | SEQ ID NO: 2735 | UGGUGUUUCUAGAUAGUGA | 0.4764 | |
| MA0852 | SEQ ID NO: 852 | GUUUCUAGAUAGUGAAACAGA | SEQ ID NO: 1794 | UGUUUCACUAUCUAGAAACAC | SEQ ID NO: 2736 | GUUUCUAGAUAGUGAAACA | 0.5278 | * |
| MA0853 | SEQ ID NO: 853 | GUGAAACAGAGAGGUGGUUUG | SEQ ID NO: 1795 | AACCACCUCUCUGUUUCACUA | SEQ ID NO: 2737 | GUGAAACAGAGAGGUGGUU | 0.4690 | |
| MA0854 | SEQ ID NO: 854 | GAAACAGAGAGGUGGUUUGUG | SEQ ID NO: 1796 | CAAACCACCUCUCUGUUUCAC | SEQ ID NO: 2738 | GAAACAGAGAGGUGGUUUG | 0.5064 | |
| MA0855 | SEQ ID NO: 855 | GAGAGGUGGUUUGUGGGAGGA | SEQ ID NO: 1797 | CUCCCACAAACCACCUCUCUG | SEQ ID NO: 2739 | GAGAGGUGGUUUGUGGGAG | 0.4722 | |
| MA0856 | SEQ ID NO: 856 | GGUGGUUUGUGGGAGGAAUAG | SEQ ID NO: 1798 | AUUCCUCCCACAAACCACCUC | SEQ ID NO: 2740 | GGUGGUUUGUGGGAGGAAU | 0.5179 | * |
| MA0857 | SEQ ID NO: 857 | GAGGAAUAGUGUCCUGGGGUU | SEQ ID NO: 1799 | CCCCAGGACACUAUUCCUCCC | SEQ ID NO: 2741 | GAGGAAUAGUGUCCUGGGG | 0.5684 | * |
| MA0858 | SEQ ID NO: 858 | AGGAAUAGUGUCCUGGGGUUC | SEQ ID NO: 1800 | ACCCCAGGACACUAUUCCUCC | SEQ ID NO: 2742 | AGGAAUAGUGUCCUGGGGU | 0.5121 | * |
| MA0859 | SEQ ID NO: 859 | UAGUGUCCUGGGGUUCCAUGA | SEQ ID NO: 1801 | AUGGAACCCCAGGACACUAUU | SEQ ID NO: 2743 | UAGUGUCCUGGGGUUCCAU | 0.5732 | * |
| MA0860 | SEQ ID NO: 860 | GGGUUCCAUGAAUUGUGGGGA | SEQ ID NO: 1802 | CCCACAAUUCAUGGAACCCCA | SEQ ID NO: 2744 | GGGUUCCAUGAAUUGUGGG | 0.5680 | * |
| MA0861 | SEQ ID NO: 861 | CAUGAAUUGUGGGGAAGCAGG | SEQ ID NO: 1803 | UGCUUCCCCACAAUUCAUGGA | SEQ ID NO: 2745 | CAUGAAUUGUGGGGAAGCA | 0.4750 | * |
| MA0862 | SEQ ID NO: 862 | UGUGGGGAAGCAGGUCAGUAU | SEQ ID NO: 1804 | ACUGACCUGCUUCCCCACAAU | SEQ ID NO: 2746 | UGUGGGGAAGCAGGUCAGU | 0.5107 | |
| MA0863 | SEQ ID NO: 863 | GCAGGUCAGUAUGGAGUCUAC | SEQ ID NO: 1805 | AGACUCCAUACUGACCUGCUU | SEQ ID NO: 2747 | GCAGGUCAGUAUGGAGUCU | 0.5578 | * |
| MA0864 | SEQ ID NO: 864 | CAGGUCAGUAUGGAGUCUACA | SEQ ID NO: 1806 | UAGACUCCAUACUGACCUGCU | SEQ ID NO: 2748 | CAGGUCAGUAUGGAGUCUA | 0.5509 | * |
| MA0865 | SEQ ID NO: 865 | AGGUCAGUAUGGAGUCUACAC | SEQ ID NO: 1807 | GUAGACUCCAUACUGACCUGC | SEQ ID NO: 2749 | AGGUCAGUAUGGAGUCUAC | 0.1647 | |
| MA0866 | SEQ ID NO: 866 | GGUCAGUAUGGAGUCUACACA | SEQ ID NO: 1808 | UGUAGACUCCAUACUGACCUG | SEQ ID NO: 2750 | GGUCAGUAUGGAGUCUACA | 0.3371 | |
| MA0867 | SEQ ID NO: 867 | UCAGUAUGGAGUCUACACAAA | SEQ ID NO: 1809 | UGUGUAGACUCCAUACUGACC | SEQ ID NO: 2751 | UCAGUAUGGAGUCUACACA | 0.4485 | |
| MA0868 | SEQ ID NO: 868 | CAGUAUGGAGUCUACACAAAA | SEQ ID NO: 1810 | UUGUGUAGACUCCAUACUGAC | SEQ ID NO: 2752 | CAGUAUGGAGUCUACACAA | 0.2882 | |
| MA0869 | SEQ ID NO: 869 | AGUAUGGAGUCUACACAAAAG | SEQ ID NO: 1811 | UUUGUGUAGACUCCAUACUGA | SEQ ID NO: 2753 | AGUAUGGAGUCUACACAAA | 0.5147 | * |
| MA0870 | SEQ ID NO: 870 | UAUGGAGUCUACACAAAAGUU | SEQ ID NO: 1812 | CUUUUGUGUAGACUCCAUACU | SEQ ID NO: 2754 | UAUGGAGUCUACACAAAAG | 0.4701 | * |
| MA0871 | SEQ ID NO: 871 | GGAGUCUACACAAAAGUUAUU | SEQ ID NO: 1813 | UAACUUUUGUGUAGACUCCAU | SEQ ID NO: 2755 | GGAGUCUACACAAAAGUUA | 0.4153 | |
| MA0872 | SEQ ID NO: 872 | GAGUCUACACAAAAGUUAUUA | SEQ ID NO: 18 14 | AUAACUUUUGUGUAGACUCCA | SEQ ID NO: 2756 | GAGUCUACACAAAAGUUAU | 0.2917 | |
| MA0873 | SEQ ID NO: 873 | CUACACAAAAGUUAUUAACUA | SEQ ID NO: 1815 | GUUAAUAACUUUUGUGUAGAC | SEQ ID NO: 2757 | CUACACAAAAGUUAUUAAC | 0.4407 | |
| MA0874 | SEQ ID NO: 874 | UUAUUAACUAUAUUCCCUGGA | SEQ ID NO: 1816 | CAGGGAAUAUAGUUAAUAACU | SEQ ID NO: 2758 | UUAUUAACUAUAUUCCCUG | 0.5889 | * |
| MA0875 | SEQ ID NO: 875 | CUAUAUUCCCUGGAUCGAGAA | SEQ ID NO: 1817 | CUCGAUCCAGGGAAUAUAGUU | SEQ ID NO: 2759 | CUAUAUUCCCUGGAUCGAG | 0.5580 | * |
| MA0876 | SEQ ID NO: 876 | AUAUUCCCUGGAUCGAGAACA | SEQ ID NO: 1818 | UUCUCGAUCCAGGGAAUAUAG | SEQ ID NO: 2760 | AUAUUCCCUGGAUCGAGAA | 0.5589 | * |
| MA0877 | SEQ ID NO: 877 | CCCUGGAUCGAGAACAUAAUU | SEQ ID NO: 1819 | UUAUGUUCUCGAUCCAGGGAA | SEQ ID NO: 2761 | CCCUGGAUCGAGAACAUAA | 0.3726 | |
| MA0878 | SEQ ID NO: 878 | CUGGAUCGAGAACAUAAUUAG | SEQ ID NO: 1820 | AAUUAUGUUCUCGAUCCAGGG | SEQ ID NO: 2762 | CUGGAUCGAGAACAUAAUU | 0.5584 | * |
| MA0879 | SEQ ID NO: 879 | GAUCGAGAACAUAAUUAGUGA | SEQ ID NO: 1821 | ACUAAUUAUGUUCUCGAUCCA | SEQ ID NO: 2763 | GAUCGAGAACAUAAUUAGU | 0.5592 | * |
| MA0880 | SEQ ID NO: 880 | UCGAGAACAUAAUUAGUGAUU | SEQ ID NO: 1822 | UCACUAAUUAUGUUCUCGAUC | SEQ ID NO: 2764 | UCGAGAACAUAAUUAGUGA | 0.5565 | * |

**[Table 1-21]**

| double stranded nucleic acid number | SEQ ID NO: | sense strand sequence (5'--->3') | SEQ ID NO: | antisense strand sequence (5'--->3') | SEQ ID NO : | target MASP2 mRNA sequence | relative expression level | note |
|---|---|---|---|---|---|---|---|---|
| MA0881 | SEQ ID NO: 881 | CGAGAACAUAAUUAGUGAUUU | SEQ ID NO: 1823 | AUCACUAAUUAUGUUCUCGAU | SEQ ID NO: 2765 | CGAGAACAUAAUUAGUGAU | 0.4365 | |
| MA0882 | SEQ ID NO: 882 | ACAUAAUUAGUGAUUJUUAAC | SEQ ID NO: 1824 | UAAAAAUCACUAAUUAUGUUC | SEQ ID NO: 2766 | ACAUAAUUAGUGAUUUUA | 0.5751 | * |
| MA0883 | SEQ ID NO: 883 | CAUAAUUAGUGAUUUUUAACU | SEQ ID NO: 1825 | UUAAAAAUCACUAAUUAUGUU | SEQ ID NO: 2767 | CAUAAUUAGUGAUUUUUAA | 0.5234 | * |
| MA0884 | SEQ ID NO: 884 | AUAAUUAGUGAUUUUUAACUU | SEQ ID NO: 1826 | GUUAAAAAUCACUAAUUAUGU | SEQ ID NO: 2768 | AUAAUUAGUGAU UUUUAAC | 0.5516 | * |
| MA0885 | SEQ ID NO: 885 | CUUGCGUGUCUGCAGUCAAGG | SEQ ID NO: 1827 | UUGACUGCAGACACGCAAGUU | SEQ ID NO: 2769 | CUUGCGUGUCUGCAGUCAA | 0.5509 | * |
| MA0886 | SEQ ID NO: 886 | GCGUGUCUGCAGUCAAGGAUU | SEQ ID NO: 1828 | UCCUUGACUGCAGACACGCAA | SEQ ID NO: 2770 | GCGUGUCUGCAGUCAAGGA | 0.4770 | * |
| MA0887 | SEQ ID NO: 887 | CUGCAGUCAAGGAUUCUUCAU | SEQ ID NO: 1829 | GAAGAAUCCUUGACUGCAGAC | SEQ ID NO: 2771 | CUGCAGUCAAGGAUUCUUC | 0.5236 | * |
| MA0888 | SEQ ID NO: 888 | GCAGUCAAGGAUUCUUCAUUU | SEQ ID NO: 1830 | AUGAAGAAUCCUUGACUGCAG | SEQ ID NO: 2772 | GCAGUCAAGGAUUCUUCAU | 0.4528 | |
| MA0889 | SEQ ID NO: 889 | GUCAAGGAUUCUUCAUUUUUA | SEQ ID NO: 1831 | AAAAUGAAGAAUCCUUGACUG | SEQ ID NO: 2773 | GUCAAGGAUUCUUCAUUUU | 0.3764 | |
| MA0890 | SEQ ID NO: 890 | CAAGGAUUCUUCAUUUUUAGA | SEQ ID NO: 1832 | UAAAAAUGAAGAAUCCUUGAC | SEQ ID NO: 2774 | CAAGGAUUCUUCAU UUUUA | 0.5676 | * |
| MA0891 | SEQ ID NO: 891 | AUUCUUCAUUUUUAGAAAUGC | SEQ ID NO: 1833 | AUUUCUAAAAAUGAAGAAUCC | SEQ ID NO: 2775 | AUUCUUCAUUUUUAGAAAU | 0.3217 | |
| MA0892 | SEQ ID NO: 892 | GACCUUGGCAGCGACGUGGCU | SEQ ID NO: 1834 | CCACGUCGCUGCCAAGGUCUU | SEQ ID NO: 2776 | GACCUUGGCAGCGACGUGG | 0.5836 | |
| MA0893 | SEQ ID NO: 893 | AGCGACGUGGCUCGAGAAGCA | SEQ ID NO: 1835 | CUUCUCGAGCCACGUCGCUGC | SEQ ID NO: 2777 | AGCGACGUGGCUCGAGAAG | 0.4633 | |
| MA0894 | SEQ ID NO: 894 | GCUCGAGAAGCAUUCAUCAUU | SEQ ID NO: 1836 | UGAUGAAUGCUUCUCGAGCCA | SEQ ID NO: 2778 | GCUCGAGAAGCAUUCAUCA | 0.4291 | * |
| MA0895 | SEQ ID NO: 895 | GCAUUCAUCAUUACUGUGGAC | SEQ ID NO: 1837 | CCACAGUAAUGAUGAAUGCUU | SEQ ID NO: 2779 | GCAUUCAUCAUUACUGUGG | 0.5987 | * |
| MA0896 | SEQ ID NO: 896 | CAUUCAUCAUUACUGUGGACA | SEQ ID NO: 1838 | UCCACAGUAAUGAUGAAUGCU | SEQ ID NO: 2780 | CAUUCAUCAUUACUGUGGA | 0.5723 | * |
| MA0897 | SEQ ID NO: 897 | CAUCAUUACUGUGGACAUGGC | SEQ ID NO: 1839 | CAUGUCCACAGUAAUGAUGAA | SEQ ID NO: 2781 | CAUCAUUACUGUGGACAUG | 0.3083 | |
| MA0898 | SEQ ID NO: 898 | AUCAUUACUGUGGACAUGGCA | SEQ ID NO: 1840 | CCAUGUCCACAGUAAUGAUGA | SEQ ID NO: 2782 | AUCAUUACUGUGGACAUGG | 0.5209 | * |
| MA0899 | SEQ ID NO: 899 | CAUUACUGUGGACAUGGCAGU | SEQ ID NO: 1841 | UGCCAUGUCCACAGUAAUGAU | SEQ ID NO: 2783 | CAUUACUGUGGACAUGGCA | 0.5665 | |
| MA0900 | SEQ ID NO: 900 | AGUUGUUGCUCCACCCAAAAA | SEQ ID NO: 1842 | UUUGGGUGGAGCAACAACUGC | SEQ ID NO: 2784 | AGUUGUUGCUCCACCCAAA | 0.5279 | |
| MA0901 | SEQ ID NO: 901 | UCCAGGUGAGGCUGCUGUCAU | SEQ ID NO: 1843 | GACAGCAGCCUCACCUGGAGU | SEQ ID NO: 2785 | UCCAGGUGAGGCUGCUGUC | 0.5279 | * |
| MA0902 | SEQ ID NO: 902 | GUGAGGCUGCUGUCAUUUCUC | SEQ ID NO: 1844 | GAAAUGACAGCAGCCUCACCU | SEQ ID NO: 2786 | GUGAGGCUGCUGUCAUUUC | 0.5074 | * |
| MA0903 | SEQ ID NO: 903 | CUGCUGUCAUUUCUCCACUUG | SEQ ID NO: 1845 | AGUGGAGAAAUGACAGCAGCC | SEQ ID NO: 2787 | CUGCUGUCAUUUCUCCACU | 0.4151 | |
| MA0904 | SEQ ID NO: 904 | CUGUCAUUUCUCCACUUGCCA | SEQ ID NO: 1846 | GCAAGUGGAGAAAUGACAGCA | SEQ ID NO: 2788 | CUGUCAUUUCUCCACUUGC | 0.3187 | |
| MA0905 | SEQ ID NO: 905 | CAUUUCUCCACUUGCCAGUUU | SEQ ID NO: 1847 | ACUGGCAAGUGGAGAAAUGAC | SEQ ID NO: 2789 | CAUUUCUCCACUUGCCAGU | 0.4096 | |
| MA0906 | SEQ ID NO: 906 | UCUCCACUUGCCAGUUUAAUU | SEQ ID NO: 1848 | UUAAACUGGCAAGUGGAGAAA | SEQ ID NO: 2790 | UCUCCACUUGCCAGUUUAA | 0.2167 | |
| MA0907 | SEQ ID NO: 907 | CUCCACUUGCCAGUUUAAUUC | SEQ ID NO: 1849 | AUUAAACUGGCAAGUGGAGAA | SEQ ID NO: 2791 | CUCCACUUGCCAGUUUAAU | 0.4924 | |
| MA0908 | SEQ ID NO: 908 | GUUUAAUUCCAGCCUUACCCA | SEQ ID NO: 1850 | GGUAAGGCUGGAAUUAAACUG | SEQ ID NO: 2792 | GUUUAAUUCCAGCCUUACC | 0.5411 | |
| MA0909 | SEQ ID NO: 909 | UUCCAGCCUUACCCAUUGACU | SEQ ID NO: 1851 | UCAAUGGGUAAGGCUGGAAUU | SEQ ID NO: 2793 | UUCCAGCCUUACCCAUUGA | 0.5060 | * |
| MA0910 | SEQ ID NO: 910 | CAUUGACUCAAGGGGACAUAA | SEQ ID NO: | AUGUCCCCUUGAGUCAAUGGG | SEQ ID NO: 2794 | CAUUGACUCAAGGGGACAU | 0.5487 | * |
| MA0911 | SEQ ID NO: 911 | AUUGACUCAAGGGGACAUAAA | SEQ ID NO: 1853 | UAUGUCCCCUUGAGUCAAUGG | SEQ ID NO: 2795 | AUUGACUCAAGGGGACAUA | 0.3894 | |
| MA0912 | SEQ ID NO: 912 | AAGGGGACAUAAACCACGAGA | SEQ ID NO: 1854 | UCGUGGUUUAUGUCCCCUUGA | SEQ ID NO: 2796 | AAGGGGACAUAAACCACGA | 0.5170 | |
| MA0913 | SEQ ID NO: 913 | GGGGACAUAAACCACGAGAGU | SEQ ID NO: 1855 | UCUCGUGGUUUAUGUCCCCUU | SEQ ID NO: 2797 | GGGGACAUAAACCACGAGA | 0.5839 | * |
| MA0914 | SEQ ID NO: 914 | GGACAUAAACCACGAGAGUGA | SEQ ID NO: 1856 | ACUCUCGUGGUUUAUGUCCCC | SEQ ID NO: 2798 | GGACAUAAACCACGAGAGU | 0.4247 | |
| MA0915 | SEQ ID NO: 915 | GACAUAAACCACGAGAGUGAC | SEQ ID NO: 1857 | CACUCUCGUGGUUUAUGUCCC | SEQ ID NO: 2799 | GACAUAAACCACGAGAGUG | 0.5401 | * |
| MA0916 | SEQ ID NO: 916 | CAUAAACCACGAGAGUGACAG | SEQ ID NO: 1858 | GUCACUCUCGUGGUUUAUGUC | SEQ ID NO: 2800 | CAUAAACCACGAGAGUGAC | 0.5976 | * |
| MA0917 | SEQ ID NO: 917 | AUAAACCACGAGAGUGACAGU | SEQ ID NO: 1859 | UGUCACUCUCGUGGUUUAUGU | SEQ ID NO: 2801 | AUAAACCACGAGAGUGACA | 0.5629 | |
| MA0918 | SEQ ID NO: 918 | CCACGAGAGUGACAGUCAUCU | SEQ ID NO: 1860 | AUGACUGUCACUCUCGUGGUU | SEQ ID NO: 2802 | CCACGAGAGUGACAGUCAU | 0.5923 | * |
| MA0919 | SEQ ID NO: 919 | CGAGAGUGACAGUCAUCUUUG | SEQ ID NO: 1861 | AAGAUGACUGUCACUCUCGUG | SEQ ID NO: 2803 | CGAGAGUGACAGUCAUCUU | 0.5774 | * |
| MA0920 | SEQ ID NO: 920 | AUCUUUGCCCACCCAGUGUAA | SEQ ID NO: 1862 | ACACUGGGUGGGCAAAGAUGA | SEQ ID NO: 2804 | AUCUUUGCCCACCCAGUGU | 0.5828 | * |
| MA0921 | SEQ ID NO: 921 | CUUUGCCCACCCAGUGUAAUG | SEQ ID NO: 1863 | UUACACUGGGUGGGCAAAGAU | SEQ ID NO: 2805 | CUUUGCCCACCCAGUGUAA | 0.4104 | |
| MA0922 | SEQ ID NO: 922 | UGCCCACCCAGUGUAAUGUCA | SEQ ID NO: 1864 | ACAUUACACUGGGUGGGCAAA | SEQ ID NO: 2806 | UGCCCACCCAGUGUAAUGU | 0.5373 | |
| MA0923 | SEQ ID NO: 923 | GCCCACCCAGUGUAAUGUCAC | SEQ ID NO: 1865 | GACAUUACACUGGGUGGGCAA | SEQ ID NO: 2807 | GCCCACCCAGUGUAAUGUC | 0.4744 | |
| MA0924 | SEQ ID NO: 924 | CCACCCAGUGUAAUGUCACUG | SEQ ID NO: 1866 | GUGACAUUACACUGGGUGGGC | SEQ ID NO: 2808 | CCACCCAGUGUAAUGUCAC | 0.4833 | |

**[Table 1-22]**

| double stranded nucleic acid number | SEQ ID NO: | sense strand sequence (5'--- >3') | SEQ ID NO: | antisense strand sequence (5'--->3') | SEQ ID NO: | target MASP2 mRNA sequence | relative expression level | note |
|---|---|---|---|---|---|---|---|---|
| MA0925 | SEQ ID NO: 925 | ACCCAGUGUAAUGUCACUGCU | SEQ ID NO: 1867 | CAGUGACAUUACACUGGGUGG | SEQ ID NO: 2809 | ACCCAGUGUAAUGUCACUG | 0.4053 | |
| MA0926 | SEQ ID NO: 926 | CCCAGUGUAAUGUCACUGCUC | SEQ ID NO: 1868 | GCAGUGACAUUACACUGGGUG | SEQ ID NO: 2810 | CCCAGUGUAAUGUCACUGC | 0.4145 | |
| MA0927 | SEQ ID NO: 927 | CCAGUGUAAUGUCACUGCUCA | SEQ ID NO: 1869 | AGCAGUGACAUUACACUGGGU | SEQ ID NO: 2811 | CCAGUGUAAUGUCACUGCU | 0.5300 | * |
| MA0928 | SEQ ID NO: 928 | GCUCAAAUUACAUUUCAUUAC | SEQ ID NO: 1870 | AAUGAAAUGUAAUUUGAGCAG | SEQ ID NO: 2812 | GCUCAAAUUACAUUUCAUU | 0.3970 | |
| MA0929 | SEQ ID NO: 929 | AAUUACAUUUCAUUACCUUAA | SEQ ID NO: 1871 | AAGGUAAUGAAAUGUAAUUUG | SEQ ID NO: 2813 | AAUUACAUUUCAUUACCUU | 0.5199 | |
| MA0930 | SEQ ID NO: 930 | AUUACAUUUCAUUACCUUAAA | SEQ ID NO: 1872 | UAAGGUAAUGAAAUGUAAUUU | SEQ ID NO: 2814 | AUUACAUUUCAUUACCUUA | 0.4176 | |
| MA0931 | SEQ ID NO: 931 | ACAUUUCAUUACCUUAAAAAG | SEQ ID NO: 1873 | UUUUAAGGUAAUGAAAUGUAA | SEQ ID NO: 2815 | ACAUUUCAUUACCUUAAAA | 0.3769 | |
| MA0932 | SEQ ID NO: 932 | CAUUUCAUUACCUUAAAAAGC | SEQ ID NO: 1874 | UUUUUAAGGUAAUGAAAUGUA | SEQ ID NO: 2816 | CAUUUCAUUACCUUAAAAA | 0.5313 | * |
| MA0933 | SEQ ID NO: 933 | UCAUUACCUUAAAAAGCCAGU | SEQ ID NO: 1875 | UGGCUUUUUAAGGUAAUGAAA | SEQ ID NO: 2817 | UCAUUACCUUAAAAAGCCA | 0.4984 | * |
| MA0934 | SEQ ID NO: 934 | GCUGUUGGCAUUUCUGUAAAC | SEQ ID NO: 1876 | UUACAGAAAUGCCAACAGCCA | SEQ ID NO: 2818 | GCUGUUGGCAUUUCUGUAA | 0.4096 | |
| MA0935 | SEQ ID NO: 935 | CUGUUGGCAUUUCUGUAAACU | SEQ ID NO: 1877 | UUUACAGAAAUGCCAACAGCC | SEQ ID NO: 2819 | CUGUUGGCAUUUCUGUAAA | 0.5750 | * |
| MA0936 | SEQ ID NO: 936 | GUUGGCAUUUCUGUAAACUGC | SEQ ID NO: 1878 | AGUUUACAGAAAUGCCAACAG | SEQ ID NO: 2820 | GUUGGCAUUUCUGUAAACU | 0.2238 | |
| MA0937 | SEQ ID NO: 937 | UGUAAACUGCCUGUCCAUGCU | SEQ ID NO: 1879 | CAUGGACAGGCAGUUUACAGA | SEQ ID NO: 2821 | UGUAAACUGCCUGUCCAUG | 0.5911 | * |
| MA0938 | SEQ ID NO: 938 | GUAAACUGCCUGUCCAUGCUC | SEQ ID NO: 1880 | GCAUGGACAGGCAGUUUACAG | SEQ ID NO: 2822 | GUAAACUGCCUGUCCAUGC | 0.3848 | |
| MA0939 | SEQ ID NO: 939 | UAAACUGCCUGUCCAUGCUCU | SEQ ID NO: 1881 | AGCAUGGACAGGCAGUUUACA | SEQ ID NO: 2823 | UAAACUGCCUGUCCAUGCU | 0.5680 | * |
| MA0940 | SEQ ID NO: 940 | UGUCCAUGCUCUUUGUUUUUA | SEQ ID NO: 1882 | AAAACAAAGAGCAUGGACAGG | SEQ ID NO: 2824 | UGUCCAUGCUCUUUGUUUU | 0.4754 | * |
| MA0941 | SEQ ID NO: 941 | CAUGCUCUUUGUUUUUAAACU | SEQ ID NO: 1883 | UUUAAAAACAAAGAGCAUGGA | SEQ ID NO: 2825 | CAUGCUCUUUGUUUUUAAA | 0.5897 | * |
| MA0942 | SEQ ID NO: 942 | UCUUUGUUUUUAAACUUGUUC | SEQ ID NO: 1884 | ACAAGUUUAAAAACAAAGAGC | SEQ ID NO: 2826 | UCUUUGUUUUUAAACUUGU | 0.5967 | * |

### [Example 2]

### Measurement of knockdown activity of MASP2 mRNA in human cell with modified siRNA

HepG2/C3A cells (obtained from ATCC, ATCC number: CRL-10741), which is a cell line derived from human liver cancer, were seeded to a 96-well culture plate at 10,000 cells/80 µL/well. As the medium, MEM medium (manufactured by Nacalai Tesque, catalog No.:21442-25) containing 10% fetal bovine serum (FBS), 1 mM sodium pyruvate (manufactured by Nacalai Tesque, catalog No.: 06977-34), 1% MEM Non-Essential Amino Acids (manufactured by Thermo Fisher Scientific, catalog No.: 11140-050) was used. As the double stranded nucleic acids, those described in Table 2 and synthesized by GeneDesign, Inc. were used. Specifically, they are constituted of double-stranded nucleic acids obtained by annealing sense strands consisting of the ribonucleotide shown in SEQ ID NO: 2828 - 2899 and antisense strands consisting of the ribonucleotide shown in SEQ ID NO: 2900 - 2971 (sense strand shown in SEQ ID NO: n (n=2828 - 2899) and antisense strand shown in SEQ ID NO: [n+72] form a pair). A double-stranded nucleic acid described in Table 2 and an RNAiMax transfection reagent (manufactured by Thermo Fisher Scientific, catalog No.: 13778-150) were diluted with Opti-MEM medium (manufactured by Thermo Fisher Scientific, catalog No. 31985-070), and 20 µL of each siRNA/RNAiMax mixture was added to 96-well culture plate to the final concentration of double-stranded nucleic acid of 10 nM, and the mixture was cultured under the conditions 37°C, 5% CO₂ for 24 hrs. Thereafter, the cells were washed with PBS (phosphate buffered saline), and cDNA was synthesized from each plate by using SuperPrep (registered trade mark) Cell Lysis & RT Kit for qPCR kit (manufactured by Toyobo, catalog No.: SCQ-101) and according to the method described in the manual attached to the product. The cDNA (4 µL) was added to MicroAmp Optical 384-well plate (manufactured by Applied Biosystems, catalog No. 4309849), and 10 µL of TaqMan (registered trade mark) Gene Expression Master Mix (manufactured by Applied Biosystems, catalog No. 4369016), 4 µL of UltraPure Distilled Water (manufactured by Thermo Fisher Scientific, catalog No.: 10977-015), 1 µL of human MASP2 probe (manufactured by Applied Biosystems, Hs00198244_m1), and 1 µL of human GAPDH probe (manufactured by Applied Biosystems, Hs02786624_g1) were further added. The real-time PCR of human MASP2 gene and human GAPDH (D-glyceraldehyde-3-phosphate dehydrogenase) gene was performed by using the QuantStudio (registered trade mark) 12K Flex real-time PCR system. GAPDH is a constitutively expressed gene and was measured as the internal control, and the MASP2 expression level was normalized. The MASP2 mRNA relative expression level when each double stranded nucleic acid was introduced was calculated relative to the MASP2 mRNA amount when HepG2/C3A cells were treated with a transfection reagent alone without addition of siRNA as 1.0. This experiment was performed 2 or 4 times and the mean of the MASP2 mRNA relative expression level is shown in Table 2. In the column of note in Table 2, * shows mean of four experiments and other shows mean of two experiments.

**[Table 2]**

| double stranded nucleic acid number | SEQ ID NO: | sense strand sequence (5'--->3') | SEQ ID NO: | antisense strand sequence (5'--->3') | relative expression level | note |
|---|---|---|---|---|---|---|
| MB0001 | SEQ ID NO: 2828 | U(F)G(M)G(F)G(M)C(F)C(M)C(F)G(M)A(F)A(M)G (F)U(F)G(M)G(F)C(M)C(F)U(M)G(F)A(M)A(F)C( M) | SEQ ID NO: 2900 | G(F)U(M)U(F)C(M)A(F)G(M)G(F)C(M)C(F) A(M)C(M)U(F)U(M)C(F)G(M)G(F)G(M)C(F) C(M)C(F)A(M)A(F)G(M) | 0.598 | |
| MB0002 | SEQ ID NO: 2829 | C(F)C(M)C(F)G(M)A(F)A(M)G(F)U(M)G(F)G(M)C (F)C(F)U(M)G(F)A(M)A(F)C(M)C(F)U(M)G(F)U( M) | SEQ ID NO: 2901 | A(F)C(M)A(F)G(M)G(F)U(M)U(F)C(M)A(F) G(M)G(M)C(F)C(M)A(F)C(M)U(F)U(M)C(F) G(M)G(F)G(M)C(F)C(M) | 0.464 | |
| MB0003 | SEQ ID NO: 2830 | A(F)G(M)G(F)A(M)G(F)C(M)G(F)G(M)C(F)G(M)C (F)U(F)G(M)G(F)A(M)C(F)C(M)C(F)U(M)G(F)A( M) | SEQ ID NO: 2902 | U(F)C(M)A(F)G(M)G(F)G(M)U(F)C(M)C(F) A(M)G(M)C(F)G(M)C(F)C(M)G(F)C(M)U(F) C(M)C(F)U(M)G(F)G(M) | 0.492 | |
| MB0004 | SEQ ID NO: 2831 | A(F)C(M)C(F)G(M)C(F)C(M)U(F)G(M)C(F)G(M)C (F)C(F)U(M)C(F)U(M)A(F)C(M)U(F)U(M)C(F)A( M) | SEQ ID NO: 2903 | U(F) G(M) A(F) A(M) G(F) U(M) A(F) G(M) A(F) G(M) G(M) C(F) G(M) C(F) A(M) G(F) G(M) C(F) G(M) G(F) U(M) A(F) G(M) | 0.400 | |
| MB0005 | SEQ ID NO: 2832 | C(F)C(M)G(F)C(M)C(F)U(M)G(F)C(M)G(F)C(M)C (F)U(F)C(M)U(F)A(M)C(F)U(M)U(F)C(M)A(F)C( M) | SEQ ID NO: 2904 | G(F) U(M) G(F) A(M) A(F) G(M) U(F) A(M) G(F) A(M) G(M) G(F) C(M) G(F) C(M) A(F) G(M) G(F) C(M) G(F) G(M) U(F) A(M) | 0.397 | |
| MB0006 | SEQ ID NO: 2833 | C(F)G(M)C(F)C(M)U(F)G(M)C(F)G(M)C(F)C(M)U (F)C(F)U(M)A(F)C(M)U(F)U(M)C(F)A(M)C(F)C( M) | SEQ ID NO: 2905 | G(F)G(M)U(F)G(M)A(F)A(M)G(F)U(M)A(F) G(M)A(M)G(F)G(M)C(F)G(M)C(F)A(M)G(F) G(M)C(F)G(M)G(F)U(M) | 0.327 | |
| MB0007 | SEQ ID NO: 2834 | C(F)C(M)U(F)C(M)U(F)A(M)C(F)U(M)U(F)C(M)A (F)C(F)C(M)C(F)A(M)C(F)U(M)U(F)C(M)G(F)A( M) | SEQ ID NO: 2906 | U(F)C(M)G(F)A(M)A(F)G(M)U(F)G(M)G(F) G(M)U(M)G(F)A(M)A(F)G(M)U(F)A(M)G(F) A(M)G(F)G(M)C(F)G(M) | 0.437 | |
| MB0008 | SEQ ID NO: 2835 | C(F)U(M)C(F)U(M)A(F)C(M)U(F)U(M)C(F)A(M)C (F)C(F)C(M)A(F)C(M)U(F)U(M)C(F)G(M)A(F)C( M) | SEQ ID NO: 2907 | G(F)U(M)C(F)G(M)A(F)A(M)G(F)U(M)G(F) G(M)G(M)U(F)G(M)A(F)A(M)G(F)U(M)A(F) G(M)A(F)G(M)G(F)C(M) | 0.576 | |
| MB0009 | SEQ ID NO: 2836 | C(F)U(M)G(F)C(M)G(F)A(M)G(F)U(M)A(F)C(M)G (F)A(F)C(M)U(F)U(M)C(F)G(M)U(F)C(M)A(F)A( M) | SEQ ID NO: 2908 | U(F)U(M)G(F)A(M)C(F)G(M)A(F)A(M)G(F) U(M)C(M)G(F)U(M)A(F)C(M)U(F)C(M)G(F) C(M)A(F)G(M)A(F)G(M) | 0.342 | |
| MB0010 | SEQ ID NO: 2837 | A(F)C(M)A(F)C(M)U(F)U(M)U(F)C(M)U(F)A(M)C (F)U(F)C(M)G(F)C(M)U(F)G(M)G(F)G(M)C(F)U( M) | SEQ ID NO: 2909 | A(F)G(M)C(F)C(M)C(F)A(M)G(F)C(M)G(F) A(M)G(M)U(F)A(M)G(F)A(M)A(F)A(M)G(F) U(M)G(F)U(M)C(F)C(M) | 0.539 | |
| MB0011 | SEQ ID NO: 2838 | G(F)G(M)G(F)C(M)U(F)C(M)C(F)A(M)G(F)C(M)C (F)U(F)G(M)G(F)A(M)C(F)A(M)U(F)U(M)A(F)C( M) | SEQ ID NO: 2910 | G(F)U(M)A(F)A(M)U(F)G(M)U(F)C(M)C(F) A(M)G(M)G(F)C(M)U(F)G(M)G(F)A(M)G(F) C(M)C(F)C(M)A(F)G(M) | 0.501 | |
| MB0012 | SEQ ID NO: 2839 | G(F)C(M)C(F)U(M)G(F)G(M)A(F)C(M)A(F)U(M)U (F)A(F)C(M)C(F)U(M)U(F)C(M)C(F)G(M)C(F)U( M) | SEQ ID NO: 2911 | A(F)G(M)C(F)G(M)G(F)A(M)A(F)G(M)G(F) U(M)A(M)A(F)U(M)G(F)U(M)C(F)C(M)A(F) G(M)G(F)C(M)U(F)G(M) | 0.578 | |
| MB0013 | SEQ ID NO: 2840 | G(F)G(M)G(F)U(M)U(F)C(M)G(F)A(M)G(F)G(M)C (F)C(F)U(M)U(F)C(M)U(F)A(M)U(F)G(M)C(F)A( M) | SEQ ID NO: 2912 | U(F)G(M)C(F)A(M)U(F)A(M)G(F)A(M)A(F) G(M)G(M)C(F)C(M)U(F)C(M)G(F)A(M)A(F) C(M)C(F)C(M)C(F)G(M) | 0.426 | |
| MB0014 | SEQ ID NO: 2841 | G(F)C(M)A(F)G(M)C(F)C(M)G(F)A(M)G(F)G(M)A (F)C(F)A(M)U(F)U(M)G(F)A(M)C(F)G(M)A(F)G( M) | SEQ ID NO: 2913 | C(F)U(M)C(F)G(M)U(F)C(M)A(F)A(M)U(F) G(M)U(M)C(F)C(M)U(F)C(M)G(F)G(M)C(F) U(M)G(F)C(M)A(F)U(M) | 0.393 | |
| MB0015 | SEQ ID NO: 2842 | C(F)C(M)U(F)G(M)U(F)G(M)U(F)C(M)C(F)C(M)U (F)A(F)C(M)G(F)A(M)C(F)U(M)U(F)U(M)C(F)U( M) | SEQ ID NO: 2914 | A(F)G(M)A(F)A(M)A(F)G(M)U(F)C(M)G(F) U(M)A(M)G(F)G(M)G(F)A(M)C(F)A(M)C(F) A(M)G(F)G(M)G(F)U(M) | 0.432 | |
| MB0016 | SEQ ID NO: 2843 | C(F)U(M)A(F)C(M)G(F)A(M)C(F)U(M)U(F)U(M)C (F)U(F)C(M)A(F)A(M)G(F)A(M)U(F)U(M)C(F)A( M) | SEQ ID NO: 2915 | U(F)G(M)A(F)A(M)U(F)C(M)U(F)U(M)G(F) A(M)G(M)A(F)A(M)A(F)G(M)U(F)C(M)G(F) U(M)A(F)G(M)G(F)G(M) | 0.529 | |
| MB0017 | SEQ ID NO: 2844 | U(F)U(M)C(F)U(M)C(F)A(M)A(F)G(M)A(F)U(M)U (F)C(F)A(M)A(F)A(M)C(F)A(M)G(F)A(M)C(F)A( M) | SEQ ID NO: 2916 | U(F)G(M)U(F)C(M)U(F)G(M)U(F)U(M)U(F) G(M)A(M)A(F)U(M)C(F)U(M)U(F)G(M)A(F) G(M)A(F)A(M)A(F)G(M) | 0.344 | |
| MB0018 | SEQ ID NO: 2845 | U(F)C(M)U(F)C(M)A(F)A(M)G(F)A(M)U(F)U(M)C (F)A(F)A(M)A(F)C(M)A(F)G(M)A(F)C(M)A(F)G( M) | SEQ ID NO: 2917 | C(F)U(M)G(F)U(M)C(F)U(M)G(F)U(M)U(F) U(M)G(M)A(F)A(M)U(F)C(M)U(F)U(M)G(F) A(M)G(F)A(M)A(F)A(M) | 0.213 | |
| MB0019 | SEQ ID NO: 2846 | U(F)C(M)A(F)C(M)A(F)G(M)A(F)U(M)G(F)A(M)A (F)U(F)C(M)A(F)G(M)G(F)A(M)G(F)A(M)C(F)C( M) | SEQ ID NO: 2918 | G(F)G(M)U(F)C(M)U(F)C(M)C(F)U(M)G(F) A(M)U(M)U(F)C(M)A(F)U(M)C(F)U(M)G(F) U(M)G(F)A(M)C(F)A(M) | 0.245 | |
| MB0020 | SEQ ID NO: 2847 | C(F)C(M)A(F)A(M)A(F)U(M)A(F)C(M)A(F)U(M)C (F)C(F)U(M)G(F)A(M)A(F)A(M)G(F)A(M)C(F)A( M) | SEQ ID NO: 2919 | U(F)G(M)U(F)C(M)U(F)U(M)U(F)C(M)A(F) G(M)G(M)A(F)U(M)G(F)U(M)A(F)U(M)U(F) U(M)G(F)G(M)C(F)U(M) | 0.505 | |
| MB0021 | SEQ ID NO: 2848 | C(F)C(M)U(F)G(M)A(F)A(M)A(F)G(M)A(F)C(M)A (F)G(F)C(M)U(F)U(M)C(F)U(M)C(F)C(M)A(F)U( M) | SEQ ID NO: 2920 | A(F)U(M)G(F)G(M)A(F)G(M)A(F)A(M)G(F) C(M)U(M)G(F)U(M)C(F)U(M)U(F)U(M)C(F) A(M)G(F)G(M)A(F)U(M) | 0.534 | |
| MB0022 | SEQ ID NO: 2849 | G(F)A(M)C(F)U(M)G(F)G(M)C(F)U(M)A(F)U(M)G (F)A(F)G(M)C(F)U(M)U(F)C(M)U(F)G(M)C(F)A( M) | SEQ ID NO: 2921 | U(F)G(M)C(F)A(M)G(F)A(M)A(F)G(M)C(F) U(M)C(M)A(F)U(M)A(F)G(M)C(F)C(M)A(F) G(M)U(F)C(M)U(F)C(M) | 0.431 | |
| MB0023 | SEQ ID NO: 2850 | C(F)U(M)A(F)C(M)A(F)A(M)A(F)G(M)C(F)U(M)G (F)U(F)G(M)A(F)U(M)U(F)C(M)A(F)G(M)U(F)A( M) | SEQ ID NO: 2922 | U(F)A(M)C(F)U(M)G(F)A(M)A(F)U(M)C(F) A(M)C(M)A(F)G(M)C(F)U(M)U(F)U(M)G(F) U(M)A(F)G(M)G(F)U(M) | 0.211 | |
| MB0024 | SEQ ID NO: 2851 | U(F)A(M)C(F)A(M)A(F)A(M)G(F)C(M)U(F)G(M)U (F)G(F)A(M)U(F)U(M)C(F)A(M)G(F)U(M)A(F)C( M) | SEQ ID NO: 2923 | G(F)U(M)A(F)C(M)U(F)G(M)A(F)A(M)U(F) C(M)A(M)C(F)A(M)G(F)C(M)U(F)U(M)U(F) G(M)U(F)A(M)G(F)G(M) | 0.296 | |
| MB0025 | SEQ ID NO: 2852 | A(F)C(M)A(F)A(M)A(F)G(M)C(F)U(M)G(F)U(M)G (F)A(F)U(M)U(F)C(M)A(F)G(M)U(F)A(M)C(F)A( M) | SEQ ID NO: 2924 | U(F)G(M)U(F)A(M)C(F)U(M)G(F)A(M)A(F) U(M)C(M)A(F)C(M)A(F)G(M)C(F)U(M)U(F) U(M)G(F)U(M)A(F)G(M) | 0.188 | |
| MB0026 | SEQ ID NO: 2853 | G(F)U(M)G(F)A(M)U(F)U(M)C(F)A(M)G(F)U(M)A (F)C(F)A(M)G(F)C(M)U(F)G(M)U(F)G(M)A(F)A( M) | SEQ ID NO: 2925 | U(F)U(M)C(F)A(M)C(F)A(M)G(F)C(M)U(F) G(M)U(M)A(F)C(M)U(F)G(M)A(F)A(M)U(F) C(M)A(F)C(M)A(F)G(M) | 0.326 | |
| MB0027 | SEQ ID NO: 2854 | U(F)U(M)C(F)A(M)G(F)U(M)A(F)C(M)A(F)G(M)C (F)U(F)G(M)U(F)G(M)A(F)A(M)G(F)A(M)G(F)A( M) | SEQ ID NO: 2926 | U(F)C(M)U(F)C(M)U(F)U(M)C(F)A(M)C(F) A(M)G(M)C(F)U(M)G(F)U(M)A(F)C(M)U(F) G(M)A(F)A(M)U(F)C(M) | 0.480 | |
| MB0028 | SEQ ID NO: 2855 | C(F)A(M)G(F)U(M)A(F)C(M)A(F)G(M)C(F)U(M)G (F)U(F)G(M)A(F)A(M)G(F)A(M)G(F)A(M)C(F)C( M) | SEQ ID NO: 2927 | G(F)G(M)U(F)C(M)U(F)C(M)U(F)U(M)C(F) A(M)C(M)A(F)G(M)C(F)U(M)G(F)U(M)A(F) C(M)U(F)G(M)A(F)A(M) | 0.347 | |
| MB0029 | SEQ ID NO: 2856 | U(F)A(M)C(F)A(M)G(F)C(M)U(F)G(M)U(F)G(M)A (F)A(F)G(M)A(F)G(M)A(F)C(M)C(F)U(M)U(F)C( M) | SEQ ID NO: 2928 | G(F)A(M)A(F)G(M)G(F)U(M)C(F)U(M)C(F) U(M)U(M)C(F)A(M)C(F)A(M)G(F)C(M)U(F) G(M)U(F)A(M)C(F)U(M) | 0.327 | |
| MB0030 | SEQ ID NO: 2857 | G(F)C(M)U(F)G(M)U(F)G(M)A(F)A(M)G(F)A(M)G (F)A(F)C(M)C(F)U(M)U(F)C(M)U(F)A(M)C(F)A( M) | SEQ ID NO: 2929 | U(F)G(M)U(F)A(M)G(F)A(M)A(F)G(M)G(F) U(M)C(M)U(F)C(M)U(F)U(M)C(F)A(M)C(F) A(M)G(F)C(M)U(F)G(M) | 0.235 | |
| MB0031 | SEQ ID NO: 2858 | C(F)U(M)G(F)U(M)G(F)A(M)A(F)G(M)A(F)G(M)A (F)C(F)C(M)U(F)U(M)C(F)U(M)A(F)C(M)A(F)C( M) | SEQ ID NO: 2930 | G(F)U(M)G(F)U(M)A(F)G(M)A(F)A(M)G(F) G(M)U(M)C(F)U(M)C(F)U(M)U(F)C(M)A(F) C(M)A(F)G(M)C(F)U(M) | 0.577 | |
| MB0032 | SEQ ID NO: 2859 | G(F)U(M)G(F)A(M)A(F)G(M)A(F)G(M)A(F)C(M)C (F)U(F)U(M)C(F)U(M)A(F)C(M)A(F)C(M)A(F)A( M) | SEQ ID NO: 2931 | U(F)U(M)G(F)U(M)G(F)U(M)A(F)G(M)A(F) A(M)G(M)G(F)U(M)C(F)U(M)C(F)U(M)U(F) C(M)A(F)C(M)A(F)G(M) | 0.336 | |
| MB0033 | SEQ ID NO: 2860 | A(F)A(M)U(F)G(M)A(F)A(M)A(F)G(M)U(F)G(M)A (F)A(F)U(M)G(F)A(M)U(F)G(M)G(F)U(M)A(F)A( M) | SEQ ID NO: 2932 | U(F)U(M)A(F)C(M)C(F)A(M)U(F)C(M)A(F) U(M)U(M)C(F)A(M)C(F)U(M)U(F)U(M)C(F) A(M)U(F)U(M)G(F)U(M) | 0.200 | |
| MB0034 | SEQ ID NO: 2861 | A(F)G(M)U(F)G(M)A(F)A(M)U(F)G(M)A(F)U(M)G (F)G(F)U(M)A(F)A(M)A(F)U(M)A(F)U(M)G(F)U( M) | SEQ ID NO: 2933 | A(F)C(M)A(F)U(M)A(F)U(M)U(F)U(M)A(F) C(M)C(M)A(F)U(M)C(F)A(M)U(F)U(M)C(F) A(M)C(F)U(M)U(F)U(M) | 0.362 | |
| MB0035 | SEQ ID NO: 2862 | A(F)A(M)A(F)U(M)A(F)U(M)G(F)U(M)G(F)U(M)G (F)U(F)G(M)A(F)G(M)G(F)C(M)U(F)G(M)A(F)U( M) | SEQ ID NO: 2934 | A(F)U(M)C(F)A(M)G(F)C(M)C(F)U(M)C(F) A(M)C(M)A(F)C(M)A(F)C(M)A(F)U(M)A(F) U(M)U(F)U(M)A(F)C(M) | 0.252 | |
| MB0036 | SEQ ID NO: 2863 | A(F)U(M)G(F)U(M)G(F)U(M)G(F)U(M)G(F)A(M)G (F)G(F)C(M)U(F)G(M)A(F)U(M)G(F)G(M)A(F)U( M) | SEQ ID NO: 2935 | A(F)U(M)C(F)C(M)A(F)U(M)C(F)A(M)G(F) C(M)C(M)U(F)C(M)A(F)C(M)A(F)C(M)A(F) C(M)A(F)U(M)A(F)U(M) | 0.567 | |
| MB0037 | SEQ ID NO: 2864 | U(F)G(M)U(F)G(M)U(F)G(M)U(F)G(M)A(F)G(M)G (F)C(F)U(M)G(F)A(M)U(F)G(M)G(F)A(M)U(F)U( M) | SEQ ID NO: 2936 | A(F)A(M)U(F)C(M)C(F)A(M)U(F)C(M)A(F) G(M)C(M)C(F)U(M)C(F)A(M)C(F)A(M)C(F) A(M)C(F)A(M)U(F)A(M) | 0.277 | |
| MB0038 | SEQ ID NO: 2865 | G(F)U(M)G(F)U(M)G(F)U(M)G(F)A(M)G(F)G(M)C (F)U(F)G(M)A(F)U(M)G(F)G(M)A(F)U(M)U(F)C( M) | SEQ ID NO: 2937 | G(F)A(M)A(F)U(M)C(F)C(M)A(F)U(M)C(F) A(M)G(M)C(F)C(M)U(F)C(M)A(F)C(M)A(F) C(M)A(F)C(M)A(F)U(M) | 0.325 | * |
| MB0039 | SEQ ID NO: 2866 | U(F)G(M)U(F)G(M)U(F)G(M)A(F)G(M)G(F)C(M)U (F)G(F)A(M)U(F)G(M)G(F)A(M)U(F)U(M)C(F)U( M) | SEQ ID NO: 2938 | A(F)G(M)A(F)A(M)U(F)C(M)C(F)A(M)U(F) C(M)A(M)G(F)C(M)C(F)U(M)C(F)A(M)C(F) A(M)C(F)A(M)C(F)A(M) | 0.382 | |
| MB0040 | SEQ ID NO: 2867 | G(F)U(M)G(F)A(M)G(F)G(M)C(F)U(M)G(F)A(M)U (F)G(F)G(M)A(F)U(M)U(F)C(M)U(F)G(M)G(F)A( M) | SEQ ID NO: 2939 | U(F)C(M)C(F)A(M)G(F)A(M)A(F)U(M)C(F) C(M)A(M)U(F)C(M)A(F)G(M)C(F)C(M)U(F) C(M)A(F)C(M)A(F)C(M) | 0.457 | |
| MB0041 | SEQ ID NO: 2868 | A (F) G (M) G (F) C (M) U (F) G (M) A (F) U (M) G (F) G (M) A (F) U (F) U (M) C (F) U (M) G (F) G (M) A (F) C (M) G (F) A ( M) | SEQ ID NO: 2940 | U(F)C(M)G(F)U(M)C(F)C(M)A(F)G(M)A(F) A(M)U(M)C(F)C(M)A(F)U(M)C(F)A(M)G(F) C(M)C(F)U(M)C(F)A(M) | 0.362 | |
| MB0042 | SEQ ID NO: 2869 | C(F)U(M)G(F)A(M)U(F)G(M)G(F)A(M)U(F)U(M)C (F)U(F)G(M)G(F)A(M)C(F)G(M)A(F)G(M)C(F)U( M) | SEQ ID NO: 2941 | A(F)G(M)C(F)U(M)C(F)G(M)U(F)C(M)C(F) A(M)G(M)A(F)A(M)U(F)C(M)C(F)A(M)U(F) C(M)A(F)G(M)C(F)C(M) | 0.477 | |
| MB0043 | SEQ ID NO: 2870 | C(F)U(M)G(F)G(M)A(F)C(M)G(F)A(M)G(F)C(M)U (F)C(F)C(M)A(F)A(M)A(F)G(M)G(F)A(M)G(F)A( M) | SEQ ID NO: 2942 | U(F)C(M)U(F)C(M)C(F)U(M)U(F)U(M)G(F) G(M)A(M)G(F)C(M)U(F)C(M)G(F)U(M)C(F) C(M)A(F)G(M)A(F)A(M) | 0.470 | |
| MB0044 | SEQ ID NO: 2871 | A(F)G(M)C(F)U(M)C(F)C(M)A(F)A(M)A(F)G(M)G (F)A(F)G(M)A(F)A(M)A(F)A(M)A(F)U(M)C(F)A( M) | SEQ ID NO: 2943 | U(F)G(M)A(F)U(M)U(F)U(M)U(F)U(M)C(F) U(M)C(M)C(F)U(M)U(F)U(M)G(F)G(M)A(F) G(M)C(F)U(M)C(F)G(M) | 0.413 | |
| MB0045 | SEQ ID NO: 2872 | A(F)G(M)G(F)G(M)C(F)A(M)A(F)A(M)A(F)G(M)G (F)C(F)A(M)A(F)A(M)A(F)C(M)C(F)U(M)G(F)G( M) | SEQ ID NO: 2944 | C(F)C(M)A(F)G(M)G(F)U(M)U(F)U(M)U(F) G(M)C(M)C(F)U(M)U(F)U(M)U(F)G(M)C(F) C(M)C(F)U(M)C(F)C(M) | 0.429 | |
| MB0046 | SEQ ID NO: 2873 | A(F)A(M)A(F)A(M)G(F)G(M)C(F)A(M)A(F)A(M)A (F)C(F)C(M)U(F)G(M)G(F)U(M)G(F)A(M)U(F)U( M) | SEQ ID NO: 2945 | A(F)A(M)U(F)C(M)A(F)C(M)C(F)A(M)G(F) G(M)U(M)U(F)U(M)U(F)G(M)C(F)C(M)U(F) U(M)U(F)U(M)G(F)C(M) | 0.397 | |
| MB0047 | SEQ ID NO: 2874 | A(F)A(M)A(F)G(M)G(F)C(M)A(F)A(M)A(F)A(M)C (F)C(F)U(M)G(F)G(M)U(F)G(M)A(F)U(M)U(F)U( M) | SEQ ID NO: 2946 | A(F)A(M)A(F)U(M)C(F)A(M)C(F)C(M)A(F) G(M)G(M)U(F)U(M)U(F)U(M)G(F)C(M)C(F) U(M)U(F)U(M)U(F)G(M) | 0.454 | |
| MB0048 | SEQ ID NO: 2875 | A(F)A(M)G(F)G(M)C(F)A(M)A(F)A(M)A(F)C(M)C (F)U(F)G(M)G(F)U(M)G(F)A(M)U(F)U(M)U(F)U( M) | SEQ ID NO: 2947 | A(F)A(M)A(F)A(M)U(F)C(M)A(F)C(M)C(F) A(M)G(M)G(F)U(M)U(F)U(M)U(F)G(M)C(F) C(M)U(F)U(M)U(F)U(M) | 0.184 | |
| MB0049 | SEQ ID NO: 2876 | A(F)C(M)C(F)U(M)G(F)G(M)U(F)G(M)A(F)U(M)U (F)U(F)U(M)C(F)C(M)U(F)U(M)G(F)G(M)C(F)A( M) | SEQ ID NO: 2948 | U(F)G(M)C(F)C(M)A(F)A(M)G(F)G(M)A(F) A(M)A(M)A(F)U(M)C(F)A(M)C(F)C(M)A(F) G(M)G(F)U(M)U(F)U(M) | 0.199 | |
| MB0050 | SEQ ID NO: 2877 | C (F) C (M) U (F) G (M) G (F) U (M) G (F) A (M) U (F) U (M) U (F) U (F) C (M) C (F) U (M) U (F) G (M) G (F) C (M) A (F) A ( M) | SEQ ID NO: 2949 | U(F)U(M)G(F)C(M)C(F)A(M)A(F)G(M)G(F) A(M)A(M)A(F)A(M)U(F)C(M)A(F)C(M)C(F) A(M)G(F)G(M)U(F)U(M) | 0.256 | |
| MB0051 | SEQ ID NO: 2878 | U (F) G (M) G (F) U (M) G (F) A (M) U (F) U (M) U (F) U (M) C (F) C (F) U (M) U (F) G (M) G (F) C (M) A (F) A (M) G (F) U ( M) | SEQ ID NO: 2950 | A(F)C(M)U(F)U(M)G(F)C(M)C(F)A(M)A(F) G(M)G(M)A(F)A(M)A(F)A(M)U(F)C(M)A(F) C(M)C(F)A(M)G(F)G(M) | 0.366 | |
| MB0052 | SEQ ID NO: 2879 | U(F)U(M)C(F)C(M)U(F)U(M)G(F)G(M)C(F)A(M)A (F)G(F)U(M)C(F)C(M)U(F)G(M)A(F)U(M)A(F)U( M) | SEQ ID NO: 2951 | A(F)U(M)A(F)U(M)C(F)A(M)G(F)G(M)A(F) C(M)U(M)U(F)G(M)C(F)C(M)A(F)A(M)G(F) G(M)A(F)A(M)A(F)A(M) | 0.347 | |
| MB0053 | SEQ ID NO: 2880 | U(F)U(M)G(F)G(M)C(F)A(M)A(F)G(M)U(F)C(M)C (F)U(F)G(M)A(F)U(M)A(F)U(M)U(F)A(M)G(F)G( M) | SEQ ID NO: 2952 | C(F)C(M)U(F)A(M)A(F)U(M)A(F)U(M)C(F) A(M)G(M)G(F)A(M)C(F)U(M)U(F)G(M)C(F) C(M)A(F)A(M)G(F)G(M) | 0.287 | |
| MB0054 | SEQ ID NO: 2881 | A(F)G(M)C(F)A(M)G(F)G(M)U(F)G(M)C(F)A(M)C (F)U(F)U(M)U(F)U(M)A(F)U(M)A(F)U(M)G(F)A( M) | SEQ ID NO: 2953 | U(F)C(M)A(F)U(M)A(F)U(M)A(F)A(M)A(F) A(M)G(M)U(F)G(M)C(F)A(M)C(F)C(M)U(F) G(M)C(F)U(M)G(F)C(M) | 0.357 | |
| MB0055 | SEQ ID NO: 2882 | C(F)A(M)G(F)G(M)U(F)G(M)C(F)A(M)C(F)U(M)U (F)U(F)U(M)A(F)U(M)A(F)U(M)G(F)A(M)C(F)A( M) | SEQ ID NO: 2954 | U (F) G (M) U (F) C (M) A (F) U (M) A (F) U (M) A (F) A (M) A (M) A (F) G (M) U (F) G (M) C (F) A (M) C (F) C (M) U (F) G (M) C (F) U (M) | 0.246 | |
| MB0056 | SEQ ID NO: 2883 | U(F)A(M)A(F)C(M)A(F)G(M)C(F)U(M)G(F)C(M)U (F)C(F)A(M)U(F)G(M)C(F)C(M)G(F)U(M)C(F)U( M) | SEQ ID NO: 2955 | A(F)G(M)A(F)C(M)G(F)G(M)C(F)A(M)U(F) G(M)A(M)G(F)C(M)A(F)G(M)C(F)U(M)G(F) U(M)U(F)A(M)G(F)G(M) | 0.497 | |
| MB0057 | SEQ ID NO: 2884 | U(F)U(M)C(F)G(M)A(F)A(M)U(F)G(M)G(F)G(M)C (F)A(F)C(M)C(F)C(M)U(F)G(M)A(F)A(M)A(F)A( M) | SEQ ID NO: 2956 | U(F)U(M)U(F)U(M)C(F)A(M)G(F)G(M)G(F) U(M)G(M)C(F)C(M)C(F)A(M)U(F)U(M)C(F) G(M)A(F)A(M)U(F)G(M) | 0.524 | |
| MB0058 | SEQ ID NO: 2885 | A(F)C(M)C(F)C(M)U(F)G(M)A(F)A(M)A(F)A(M)G (F)A(F)C(M)U(F)A(M)U(F)C(M)A(F)C(M)C(F)U( M) | SEQ ID NO: 2957 | A(F) G(M) G(F) U(M) G(F) A(M) U(F) A(M) G(F) U(M) C(M) U(F) U(M) U(F) U(M) C(F) A(M) G(F) G(M) G(F) U(M) G(F) C(M) | 0.557 | |
| MB0059 | SEQ ID NO: 2886 | U(F)A(M)U(F)A(M)C(F)U(M)C(F)A(M)U(F)G(M)A (F)U(F)G(M)C(F)U(M)G(F)G(M)C(F)U(M)U(F)U( M) | SEQ ID NO: 2958 | A(F)A(M)A(F)G(M)C(F)C(M)A(F)G(M)C(F) A(M)U(M)C(F)A(M)U(F)G(M)A(F)G(M)U(F) A(M)U(F)A(M)A(F)C(M) | 0.241 | |
| MB0060 | SEQ ID NO: 2887 | U(F)A(M)C(F)U(M)C(F)A(M)U(F)G(M)A(F)U(M)G (F)C(F)U(M)G(F)G(M)C(F)U(M)U(F)U(M)G(F)A( M) | SEQ ID NO: 2959 | U(F)C(M)A(F)A(M)A(F)G(M)C(F)C(M)A(F) G(M)C(M)A(F)U(M)C(F)A(M)U(F)G(M)A(F) G(M)U(F)A(M)U(F)A(M) | 0.453 | |
| MB0061 | SEQ ID NO: 2888 | U(F)C(M)A(F)U(M)G(F)A(M)U(F)G(M)C(F)U(M)G (F)G(F)C(M)U(F)U(M)U(F)G(M)A(F)C(M)A(F)A( M) | SEQ ID NO: 2960 | U(F)U(M)G(F)U(M)C(F)A(M)A(F)A(M)G(F) C(M)C(M)A(F)G(M)C(F)A(M)U(F)C(M)A(F) U(M)G(F)A(M)G(F)U(M) | 0.493 | |
| MB0062 | SEQ ID NO: 2889 | A(F)U(M)G(F)A(M)U(F)G(M)C(F)U(M)G(F)G(M)C (F)U(F)U(M)U(F)G(M)A(F)C(M)A(F)A(M)U(F)G( M) | SEQ ID NO: 2961 | C(F)A(M)U(F)U(M)G(F)U(M)C(F)A(M)A(F) A(M)G(M)C(F)C(M)A(F)G(M)C(F)A(M)U(F) C(M)A(F)U(M)G(F)A(M) | 0.240 | |
| MB0063 | SEQ ID NO: 2890 | U(F)G(M)A(F)U(M)G(F)C(M)U(F)G(M)G(F)C(M)U (F)U(F)U(M)G(F)A(M)C(F)A(M)A(F)U(M)G(F)A( M) | SEQ ID NO: 2962 | U(F)C(M)A(F)U(M)U(F)G(M)U(F)C(M)A(F) A(M)A(M)G(F)C(M)C(F)A(M)G(F)C(M)A(F) U(M)C(F)A(M)U(F)G(M) | 0.211 | |
| MB0064 | SEQ ID NO: 2891 | A(F)U(M)G(F)C(M)U(F)G(M)G(F)C(M)U(F)U(M)U (F)G(F)A(M)C(F)A(M)A(F)U(M)G(F)A(M)C(F)A( M) | SEQ ID NO: 2963 | U(F)G(M)U(F)C(M)A(F)U(M)U(F)G(M)U(F) C(M)A(M)A(F)A(M)G(F)C(M)C(F)A(M)G(F) C(M)A(F)U(M)C(F)A(M) | 0.440 | |
| MB0065 | SEQ ID NO: 2892 | U(F)G(M)C(F)U(M)G(F)G(M)C(F)U(M)U(F)U(M)G (F)A(F)C(M)A(F)A(M)U(F)G(M)A(F)C(M)A(F)U( M) | SEQ ID NO: 2964 | A(F)U(M)G(F)U(M)C(F)A(M)U(F)U(M)G(F) U(M)C(M)A(F)A(M)A(F)G(M)C(F)C(M)A(F) G(M)C(F)A(M)U(F)C(M) | 0.253 | |
| MB0066 | SEQ ID NO: 2893 | G(F)C(M)U(F)G(M)G(F)C(M)U(F)U(M)U(F)G(M)A (F)C(F)A(M)A(F)U(M)G(F)A(M)C(F)A(M)U(F)A( M) | SEQ ID NO: 2965 | U(F)A(M)U(F)G(M)U(F)C(M)A(F)U(M)U(F) G(M)U(M)C(F)A(M)A(F)A(M)G(F)C(M)C(F) A(M)G(F)C(M)A(F)U(M) | 0.189 | |
| MB0067 | SEQ ID NO: 2894 | G(F)G(M)C(F)U(M)U(F)U(M)G(F)A(M)C(F)A(M)A (F)U(F)G(M)A(F)C(M)A(F)U(M)A(F)G(M)C(F)A( M) | SEQ ID NO: 2966 | U(F)G(M)C(F)U(M)A(F)U(M)G(F)U(M)C(F) A(M)U(M)U(F)G(M)U(F)C(M)A(F)A(M)A(F) G(M)C(F)C(M)A(F)G(M) | 0.253 | |
| MB0068 | SEQ ID NO: 2895 | U(F)U(M)G(F)A(M)C(F)A(M)A(F)U(M)G(F)A(M)C (F)A(F)U(M)A(F)G(M)C(F)A(M)C(F)U(M)G(F)A( M) | SEQ ID NO: 2967 | U(F)C(M)A(F)G(M)U(F)G(M)C(F)U(M)A(F) U(M)G(M)U(F)C(M)A(F)U(M)U(F)G(M)U(F) C(M)A(F)A(M)A(F)G(M) | 0.392 | |
| MB0069 | SEQ ID NO: 2896 | A(F)U(M)G(F)A(M)C(F)A(M)U(F)A(M)G(F)C(M)A (F)C(F)U(M)G(F)A(M)U(F)U(M)A(F)A(M)A(F)U( M) | SEQ ID NO: 2968 | A(F)U(M)U(F)U(M)A(F)A(M)U(F)C(M)A(F) G(M)U(M)G(F)C(M)U(F)A(M)U(F)G(M)U(F) C(M)A(F)U(M)U(F)G(M) | 0.316 | |
| MB0070 | SEQ ID NO: 2897 | A(F)U(M)A(F)G(M)C(F)A(M)C(F)U(M)G(F)A(M)U (F)U(F)A(M)A(F)A(M)U(F)U(M)G(F)A(M)A(F)U( M) | SEQ ID NO: 2969 | A(F)U(M)U(F)C(M)A(F)A(M)U(F)U(M)U(F) A(M)A(M)U(F)C(M)A(F)G(M)U(F)G(M)C(F) U(M)A(F)U(M)G(F)U(M) | 0.269 | |
| MB0071 | SEQ ID NO: 2898 | A(F)A(M)U(F)A(M)A(F)C(M)A(F)A(M)A(F)G(M)U (F)U(F)G(M)U(F)A(M)A(F)U(M)C(F)A(M)A(F)U( M) | SEQ ID NO: 2970 | A(F)U(M)U(F)G(M)A(F)U(M)U(F)A(M)C(F) A(M)A(M)C(F)U(M)U(F)U(M)G(F)U(M)U(F) A(M)U(F)U(M)C(F)A(M) | 0.359 | |
| MB0072 | SEQ ID NO: 2899 | A(F)A(M)G(F)U(M)U(F)G(M)U(F)A(M)A(F)U(M)C (F)A(F)A(M)U(F)A(M)G(F)C(M)A(F)A(M)C(F)A( M) | SEQ ID NO: 2971 | U (F) G (M) U (F) U (M) G (F) C (M) U (F) A (M) U (F) U (M) G (M) A (F) U (M) U (F) A (M) C (F) A (M) A (F) C (M) U (F) U (M) U (F) G (M) | 0.561 | |

This application is based on a patent application No. 2017-045226 filed in Japan (filing date: March 9, 2017), the contents of which are incorporated in full herein.

### [INDUSTRIAL APPLICABILITY]

The present invention provides a nucleic acid having activity to suppress expression of MASP2, a pharmaceutical composition comprising the nucleic acid as an active ingredient, and the like. The nucleic acid and pharmaceutical composition of the present invention suppress expression of MASP2, and are useful for the treatment or prophylaxis of autoimmune diseases such as APS, SLE and the like and thrombosis in hemodialysis.

## Claims

1. A double-stranded nucleic acid that decreases expression of MASP2 gene, which consists of a sense strand and an antisense strand, and comprises a double-stranded region of at least 11 base pairs, wherein an oligonucleotide strand having a strand length of at least 17 nucleotides and 30 nucleotides at most in the antisense strand is complementary to a target MASP2 mRNA sequence selected from the group described in Table 1-1 to Table 1-22.

2. The double-stranded nucleic acid according to claim 1, wherein the double-stranded region is composed of 11 - 27 base pairs, and the 2nd nucleotide from the 5'-terminus of the antisense strand complementary to the target MASP2 mRNA sequence selected from the group described in Tables 1-1 to Table 1-22 is complement to the 2nd ribonucleotide from the 3'-terminus of the target MASP2 mRNA sequence.

3. The double-stranded nucleic acid according to claim 1 or 2, wherein the 3'-terminus of the sense strand and the 5'-terminus of the antisense strand form a blunt end.

4. The double-stranded nucleic acid according to claim 3, wherein the sense strand is 21 nucleotides in length and the antisense strand is 23 nucleotides in length.

5. The double-stranded nucleic acid according to claim 1 or 2, wherein the sense strand is 21 nucleotides in length and the antisense strand is 21 nucleotides in length.

6. The double-stranded nucleic acid according to claim 5, wherein the 3'-terminal of the sense strand and the 3'-terminal of the antisense strand have an overhang.

7. The double-stranded nucleic acid according to any one of claims 1 to 6, wherein the antisense strand comprises a sequence selected from the groups described in "antisense strand" in Table 1-1 to Table 1-22 and Table 2.

8. The double-stranded nucleic acid according to any one of claims 1 to 6, wherein the sense strand comprises a sequence selected from the groups described in "sense strand" in Table 1-1 to Table 1-22 and Table 2.

9. The double-stranded nucleic acid according to any one of claims 1 to 6, comprising a sequence of 1 pair of sense strand/antisense strand selected from the group consisting of the sense strands/antisense strands described in Table 1-1 to Table 1-22 and Table 2.

10. The double-stranded nucleic acid according to any one of claims 1 to 9, comprising a 2'-modified nucleotide.

11. The double-stranded nucleic acid according to claim 10, wherein 50 - 100% of the nucleotides in the double strand region are 2'-modified nucleotides.

12. The double-stranded nucleic acid according to any one of claims 1 to 11, comprising a ligand.

13. A single strand nucleic acid consisting only of an antisense strand in the double-stranded nucleic acid described in any one of claims 1 to 12.

14. A pharmaceutical composition comprising the nucleic acid described in any one of claims 1 to 13.

15. A method of treating a disorder mediated by abnormal complement lectin pathway, comprising a step of administering a therapeutically effective amount of the nucleic acid described in any one of claims 1 to 13 or the pharmaceutical composition according to claim 14 to a human in need of such treatment.

16. The method according to claim 15, wherein the disorder is IgA nephropathy, membranous nephropathy, lupus nephropathy, C3 nephropathy, thrombotic thrombocytopenic purpura, thrombotic microangiopathy, atypical hemolytic uremic syndrome (aHUS), kidney disorder during ischemia or tissue injury during cerebral infarction.
